(19) Europäisches Patentamt
European Patent Office
Office européen des brevets

(11) EP 0 647 219 B1

(12) **EUROPÄISCHE PATENTSCHRIFT**

(45) Veröffentlichungstag und Bekanntmachung des Hinweises auf die Patenterteilung: **31.07.1996 Patentblatt 1996/31**

(21) Anmeldenummer: **93914720.3**

(22) Anmeldetag: **24.06.1993**

(51) Int. Cl.$^6$: **C07C 401/00**, A61K 31/59, C07F 7/18, C07C 69/013, C07C 35/32

(86) Internationale Anmeldenummer: **PCT/EP93/01620**

(87) Internationale Veröffentlichungsnummer: **WO 94/00428 (06.01.1994 Gazette 1994/02)**

(54) **DERIVATE IN DER VITAMIN D-REIHE MIT MODIFIKATIONEN IN DER 20 POSITION, VERFAHREN ZU IHRER HERSTELLUNG, ZWISCHENPRODUKTE FÜR DIESES VERFAHREN, DIESE DERIVATE ENTHALTENDE PHARMAZEUTISCHE PRÄPARATE SOWIE DEREN VERWENDUNG ZUR HERSTELLUNG VON ARZNEIMITTELN**

DERIVATIVES IN THE VITAMIN D SERIES MODIFIED AT THE 20 POSITION, A METHOD OF PREPARING SUCH DERIVATIVES, INTERMEDIATES USED IN THIS METHOD, PHARMACEUTICAL PREPARATIONS CONTAINING THE DERIVATIVES AND THEIR USE IN THE PREPARATION OF DRUGS

DERIVES DE LA SERIE DE LA VITAMINE D MODIFIES A LA POSITION 20, LEUR PROCEDE DE PREPARATION, PRODUITS INTERMEDIAIRES POUR CE PROCEDE, PREPARATIONS PHARMACEUTIQUES CONTENANT CES DERIVES ET LEUR UTILISATION POUR PREPARER DES MEDICAMENTS

(84) Benannte Vertragsstaaten: **AT BE CH DE DK ES FR GB GR IE IT LI LU MC NL PT SE**

(30) Priorität: **24.06.1992 DE 4220757**

(43) Veröffentlichungstag der Anmeldung: **12.04.1995 Patentblatt 1995/15**

(73) Patentinhaber: **SCHERING AKTIENGESELLSCHAFT D-13353 Berlin (DE)**

(72) Erfinder:
- **STEINMEYER, Andreas, Dr. D-14052 Berlin (DE)**
- **NEEF, Günter, Dr. D-10711 Berlin (DE)**
- **KIRSCH, Gerald, Dr. D-14199 Berlin (DE)**
- **SCHWARZ, Katica D-10585 Berlin (DE)**
- **THIEROFF-EKERDT, Ruth, Dr. D-13469 Berlin (DE)**
- **WIESINGER, Herbert, Dr. D-10781 Berlin (DE)**
- **HABEREY, Martin, Dr. D-12247 Berlin (DE)**

(56) Entgegenhaltungen:
**EP-A- 0 184 112**
**EP-A- 0 387 077**
**EP-A- 0 521 550**
**WO-A-90/06121**
**DE-A- 4 141 746**

- **CHEMICAL AND PHARMACEUTICAL BULLETIN. Bd. 40, Nr. 3, M rz 1992, TOKYO JP Seiten 648 - 651 N. KUBODERA ET AL 'Synthetic Studies of Vitamin D Analogs. X. Synthesis and Biological Activities of 1-alpha,25-dihydroxy-21-nor-Vitamin D3'**
- **JOURNAL OF ORGANIC CHEMISTRY. Bd. 57, Nr. 11, 22. Mai 1992, EASTON US Seiten 3173 - 3178 B. FERNANDEZ ET AL 'Synthesis of Hydrindan Derivatives Related to Vitamin D'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.13.0/3.4

## Beschreibung

Die vorliegende Erfindung betrifft Vitamin D-Derivate der allgemeinen Formel I

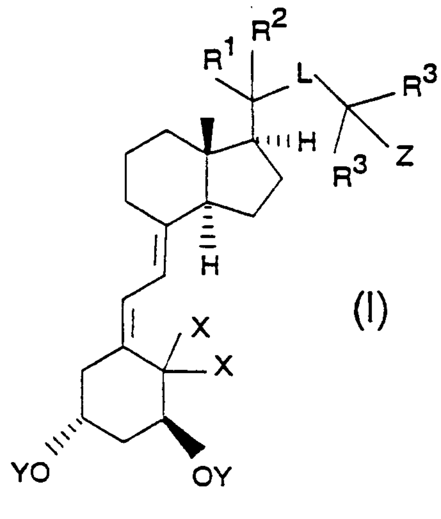

(I)

worin Y je ein Wasserstoffatom oder je eine Alkanoylgruppe mit 1 bis 9 Kohlenstoffatomen, oder eine Aroylgruppe,
Z ein Wasserstoffatom, eine Hydroxylgruppe oder eine Alkanoyl- gruppe mit 1 bis 9 Kohlenstoffatomen,
X je ein Wasserstoffatom oder beide X gemeinsam eine exocyclische Methylengruppe,
$R^1$ und $R^2$ unabhängig voneinander je eine Akylgruppe mit 1 bis 4 Kohlenstoffatomen, gemeinsam eine Methylengruppe oder gemeinsam mit dem quartären Kohlenstoffatom 20 eine Cyclopropyleinheit, wobei wenn beide X eine Methylengruppe bedeuten, $R^1$ und $R^2$ nicht Methyl sind,
$R^3$ je ein Wasserstoffatom oder je eine lineare oder verzweigte Alkylgruppe mit 1 bis 5 Kohlenstoffatomen, je eine Trifluormethylgruppe oder einen gemeinsam mit dem tertiären Kohlenstoffatom 25 gebildeten gesättigten oder ungesättigten, carbocyclischen oder heterocyclischen 3-, 4-, 5- oder 6-gliedrigen Ring,
L entweder die Gruppierung

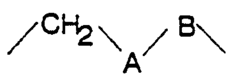

,

wobei
A eine Methylengruppe oder ein Sauerstoff-, Schwefel-, oder ein hydrid- oder $C_1$-$C_4$-alkylsubstituiertes Stickstoffatom und
B eine Alkylengruppe $-(CH_2)_n-$, worin n = 1, 2, 3, 4, 5 oder 6 ist und eine beliebige Methylengruppe durch ein Sauerstoffatom ersetzt sein kann, darstellen, oder
L die Gruppierung

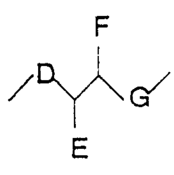

,

wobei
D eine direkte Bindung, eine Methylenbrücke oder eine (E) 1,2-Ethendiylbrücke zwischen den Kohlenstoffatomen 20 und 22,
E und F jeweils ein Wasserstoffatom, oder gemeinsam eine E-Doppelbindung, und
G eine direkte Bindung oder eine Alkylengruppe $-(CH_2)_n-$, worin n = 1, 2, 3, 4, 5 oder 6 ist und eine beliebige Methylengruppe durch ein Sauerstoffatom ersetzt sowie jede Methylengruppe durch eine Hydroxylgruppe oder ein Fluor-, Chlor- oder Bromatom substituiert sein kann, darstellen, bedeuten,

sowie ein Verfahren zu ihrer Herstellung, Zwischenprodukte für dieses Verfahren, pharmazeutische Präparate, die diese Verbindungen enthalten sowie deren Verwendung zur Herstellung von Arzneimitteln.

Die als Reste Y sowie Z möglichen Acylgruppen oder Acyloxygruppen sind insbesondere von gesättigten Carbonsäuren mit 1 bis 9 Kohlenstoffatomen oder auch von der Benzoesäure abgeleitet.

Als Alkylgruppen für $R^3$ kommen in erster Linie die Methyl-, Ethyl- oder Propylgruppe sowie ein zusammen mit dem tertiären Kohlenstoffatom gebildeter Cyclopropyl- oder Cyclopentylring infrage.

In den Vitamin D-Derivaten der allgemeinen Formel bedeuten vorzugsweise $R^1$ und $R^2$ je eine Alkylgruppe und beide X je ein Wasserstoffatom, $R^1$ und $R^2$ gemeinsam eine Methylengruppe und beide X je ein Wasserstoffatom, $R^1$ und $R^2$ gemeinsam eine Methylengruppe und beide X gemeinsam eine Methylengruppe, $R^1$ und $R^2$ zusammen mit dem Kohlenstoffatom 20 einen Cyclopropylring und beide X gemeinsam eine Methylengruppe oder $R^1$ und $R^2$ zusammen mit dem Kohlenstoffatom 20 einen Cyclopropylring und beide X je ein Wasserstoffatom.

Bevorzugt sind weiterhin die Derivate mit folgenden Seitenketten:

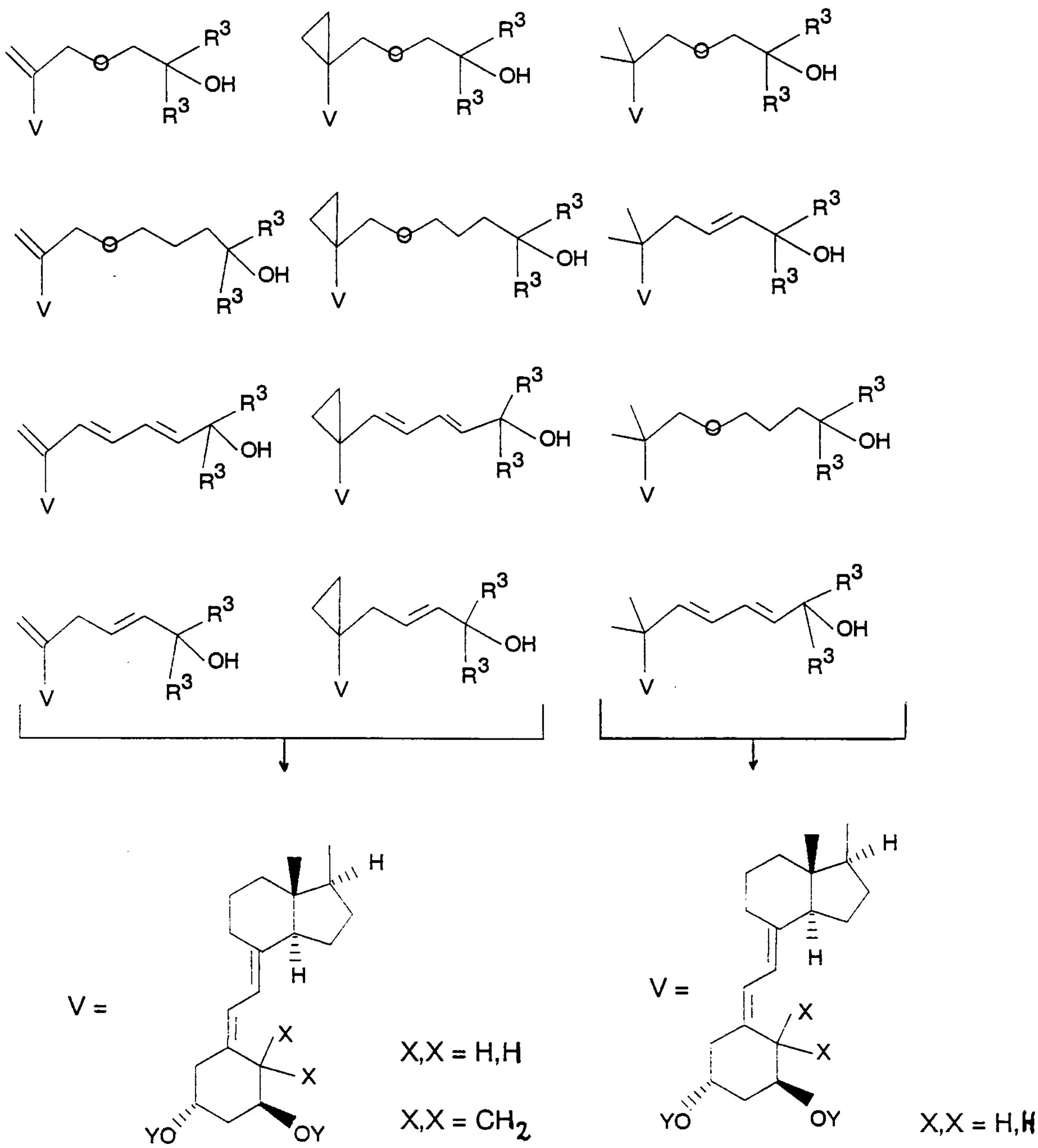

V =

X,X = H,H

X,X = $CH_2$

V =

X,X = H,H

Besonders bevorzugt sind die Verbindungen:

(5Z,7E)-(1S,3R)-26,27-Dimethyl-23-oxa-9,10-secocholesta-5,7,10(19),20-tetraen-1,3,25-triol,

(5Z,7E)-(1S,3R)-26,27-Diethyl-23-oxa-9,10-secocholesta-5,7,10(19),20-tetraen-1,3,25-triol,
7E)-(1R,3R)-20,26,27-Trimethyl-23-oxa-19-nor 9,10-secocholesta-5,7-dien-1,3,25-triol,
(5Z,7E)-(1S,3R)-26,27-Dimethyl-20,21-methylen-23-oxa-9,10-secocholesta-5,7,10(19)-trien-1,3,25-triol,
(5Z,7E)-(1S,3R)-23-Oxa-9,10-secocholesta-5,7,10(19),20-tetraen-1,3,25-triol,
(5Z,7E)-(1S,3R)-24-(3-Hydroxy-3-methylbutyl)-23-oxa-9,10-secochola-5,7,10(19),20-tetraen-1,3-diol,
(5Z,7E)-(1S,3R)-24-(3-Ethyl-3-hydroxypentyl)-23-oxa-9,10-secochola-5,7,10(19),20-tetraen-1,3-diol,
(5Z,7E)-(1S,3R)-26,27-Diethyl-20,21-methylen-23-oxa-9,10-secocholesta-5,7,10(19)-trien-1,3,25-triol,
(7E)-(1R,3R)-23-Oxa-19-nor-9,10-secocholesta-5,7,20-trien-1,3,25-triol,
(5Z,7E)-(1S,3R)-20,21-Methylen-23-oxa-9,10-secocholesta-5,7,10(19)-trien-1,3,25-triol,
(5Z,7E)-(1S,3R)-24-(3-Hydroxy-3-methylbutyl)-20,21-methylen-23-oxa-9,10-secochola-5,7,10(19)-trien-1,3-diol,
(5Z,7E)-(1S,3R)-24-(3-Ethyl-3-hydroxypentyl)-20,21-methylen-23-oxa-9,10-secochola-5,7,10(19)-trien-1,3-diol,
(7E)-(1R,3R)-20-Methyl-19-nor-23-oxa-9,10-secocholesta-5,7-dien-1,3,25-triol,
(7E)-(1R,3R)-26,27-Diethyl-20-methyl-19-nor-23-oxa-9,10-secocholesta-5,7-dien-1,3,25-triol,
(7E)-(1R,3R)-24-(3-Hydroxy-3-methylbutyl)-20-methyl-19-nor-23-oxa-9,10-secochola-5,7-dien-1,3-diol,
(7E)-(1R,3R)-24-(3-Ethyl-3-hydroxypentyl)-20-methyl-19-nor-23-oxa-9,10-secochola-5,7-dien-1,3-diol,
(7E)-(1R,3R)-26,27-Dimethyl-19-nor-23-oxa-9,10-secocholesta-5,7,20-trien-1,3,25-triol,
(7E)-(1R,3R)-26,27-Diethyl-19-nor-23-oxa-9,10-secocholesta-5,7,20-trien-1,3,25-triol,
(7E)-(1R,3R)-24-(3-Hydroxy-3-methylbutyl)-19-nor-23-oxa-9,10-secochola-5,7,20-trien-1,3-diol,
(7E)-(1R,3R)-24-(3-Ethyl-3-hydroxypentyl)-19-nor-23-oxa-9,10-secochola-5,7,20-trien-1,3-diol,
(5Z,7E,22E)-(1S,3R)-24-(2-Hydroxy-2-methylpropoxy)-9,10-secochola-5,7,10(19),20,22-pentaen-1,3-diol,
(5Z,7E,22E)-(1S,3R)-24-(2-Ethyl-2-hydroxybutoxy)-9,10-secochola-5,7,10(19),20,22-pentaen-1,3-diol,
(7E,22E)-(1R,3R)-24-(2-Hydroxy-2-methylpropoxy)19-nor-9,10-secochola-5,7,20,22-tetraen-1,3-diol,
(7E,22E)-(1R,3R)-24-(2-Ethyl-2-hydroxybutoxy)-19-nor-9,10-secochola-5,7,20,22-tetraen-1,3-diol.

Die natürlichen Vitamine $D_2$ und $D_3$ (vgl. allgemeine Formel Vit.D) sind an sich biologisch inaktiv und werden erst nach Hydroxylierung in 25-Position in der Leber bzw. in 1-Position in der Niere in deren biologisch aktive Metaboliten umgewandelt. Die Wirkung der Vitamine $D_2$und $D_3$ besteht in der Stabilisierung des Plasma-$Ca^{++}$- und Plasma-Phosphat-Spiegels; sie wirken einem Absinken des Plasma-$Ca^{++}$-Spiegels entgegen.

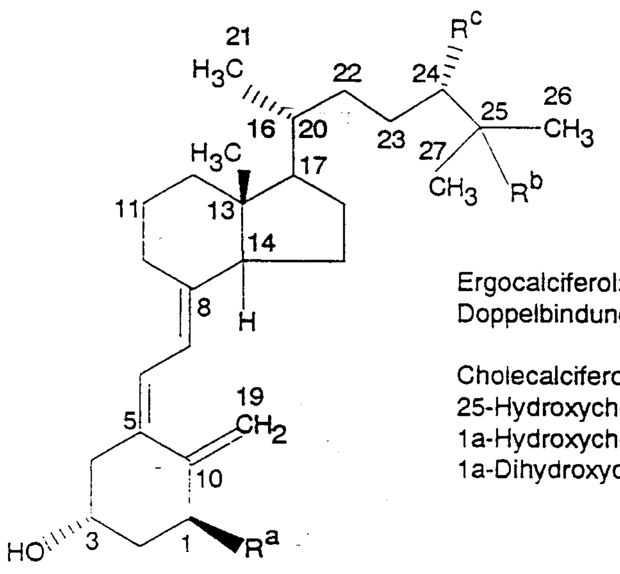

Ergocalciferol: Ra=Rb=H, Rc=CH3 Doppelbindung C-22/23 — Vitamin $D_2$

Cholecalciferol: Ra=Rb=Rc=H — Vitamin $D_3$
25-Hydroxycholecalciferol: Ra=Rc=H,Rb=OH
1a-Hydroxycholecalciferol: Ra=OH,Rb=Rc=H
1a-Dihydroxycholecalciferol: Ra=Rb=OH,Rc=H — Calcitriol

Neben ihrer ausgeprägten Wirkung auf den Calcium- und Phosphatstoffwechsel besitzen Vitamin $D_2$ und $D_3$ und seine synthetischen Abkömmlinge auch proliferationshemmende und zelldifferenzierende Wirkungen (H.F. De Luca, The Metabolism and Function of Vitamin D in Biochemistry of Steroid Hormones, Hrsg. H.L.J. Makin, 2nd Edition, Blackwell Scientific Publications 1984, S. 71-116).
Bei Vitamin D-Anwendung kann es aber zu Überdosierungserscheinungen kommen (Hypercalcämie).

In 24-Stellung hydroxylierte 1α-Cholecalciferole gehen bereits aus der DE-A-25 26 981 hervor; sie besitzen eine geringere Toxizität als das entsprechende nicht-hydroxylierte 1α-Cholecalciferol. Die hydroxylierten Verbindungen zeigen eine selektive Aktivierung der intestinalen Calciumabsorption und eine schwächere Knochenabsorptionswirkung als 1α-Cholecalciferol.
Die in der internationalen Patentanmeldung WO 87/00834 beschriebenen 24-Hydroxy-Vitamin D-Analoga können für

die Behandlung von durch abnormer Zellproliferation und/oder Zelldifferentiation hervorgerufenen Störungen beim Menschen und Tier dienen.

Desweiteren beschreibt die EP-A-184 112 hauptsächlich Verbindungen, die sich ebenfalls durch eine Planarisierung an C-20 auszeichnen. Diese Derivate besitzen an C-20 ausschließlich eine Sauerstoffunktion, wie beispielsweise Hydroxyl-, Keto-, Oxim- oder Oximetherfunktionen. Die erfindungsgemäßen Verbindungen besitzen ebenfalls an C-20 ein planares Zentrum, im Gegensatz zu den Verbindungen der EP-A-184 112 ist durch die fehlende Sauerstofffunktion die elektronische Situation gravierend verändert worden und somit unterscheiden sich diese Verbindungen grundlegend von den erfindungsgemäßen Verbindungen.

WO-A-93/12081 beschreibt 20-Methyl-C19-Vitamin D Derivate, die von der vorliegenden Erfindung, (Formel (I)) nicht umfaßt sind Für verschiedene 1,25-Dihydroxy-Homo-Vitamin D-Derivate ist eine Dissoziation bezüglich der Eigenschaften Knochenabsorptionswirkung und HL-60 Zelldifferentiation schon kürzlich von DeLuca erwähnt worden. Die Knochenabsorptionswirkung in vitro ist dabei ein direktes Maß für die Calciummobilisierung in vivo.

Die Vitamin D-Aktivität der erfindungsgemäßen Verbindungen wird mittels des Calcitriol-Rezeptortests bestimmt. Er wird unter Verwendung eines spezifischen Rezeptorproteins aus dem Darm von jungen Schweinen durchgeführt (M.C. Dame, E.A. Pierce, H.F. DeLuca, Proc. Natl. Acad. Sci. USA 82, 7825 (1985)).

Rezeptorhaltiges Bindungsprotein wird mit $^3$H-Calcitriol ($5x10^{-10}$mol/l) in einem Reaktionsvolumen von 0,270 ml in Abwesenheit und in Anwesenheit der Prüfsubstanzen für zwei Stunden bei 4° C in einem Teströhrchen inkubiert. Zur Trennung von freiem und rezeptorgebundenem Calcitriol wird eine Charcoal-Dextran-Absorption durchgeführt. Dazu werden 250 µl einer Charcoal-Dextran-Suspension jedem Teströhrchen zugeführt und bei 4° C für 20 Minuten inkubiert. Anschließend werden die Proben bei 10 000 x g 5 Minuten bei 4° C zentrifugiert. Der Überstand wird dekantiert und nach 1stündiger Äquilibrierung in Picofluor 15 TM in einem β-Zähler gemessen.
Die mit verschiedenen Konzentrationen der Prüfsubstanz sowie der Referenzsubstanz (unmarkiertes Calcitriol) bei konstanter Konzentration der Bezugssubstanz ($^3$H-Calcitriol) erhaltenen Kompetitionskurven werden in Beziehung zueinander gesetzt und ein Kompetitionsfaktor ($K_F$) ermittelt.
Er ist definiert als Quotient aus den Konzentrationen der jeweiligen Prüfsubstanz und der Referenzsubstanz, die für 50%ige Kompetition erforderlich sind:

$$K_F = \frac{\text{Konzentration Prüfsubstanz bei 50\% Kompetition}}{\text{Konzentration Referenzsubstanz bei 50\% Kompetition}}$$

Zur Bestimmung der akuten hypercalcämischen Wirkung verschiedener Calcitriolderivate wird nachfolgend beschriebener Test durchgeführt:
Die Wirkung von Kontrolle (Lösungsmittelgrundlage), Referenzsubstanz ($1,25(OH)_2$-$D_3$= Calcitriol) und Testsubstanz wird jeweils nach einmaliger subcutaner Applikation in Gruppen von 10 nativen männlichen Ratten (140-170 g) getestet. Die Ratten werden während der Versuchszeit in speziellen Käfigen zur Bestimmung der Exkretion von Wasser und Mineralstoffen gehalten. Der Harn wird in 2 Fraktionen (0-16 h und 16-22h) gesammelt. Eine orale Calciumlast (0.1 mM Calcium in 6.5% Alphahydroxypropylcellulose, 5 ml/ Tier) ersetzt zum Zeitpunkt 16 h die durch Futterentzug fehlende Calciumaufnahme. Zu Versuchsende werden die Tiere durch Dekapitieren getötet und für die Bestimmung der Serum-Calciumwerte entblutet. Für die primäre Screen-Untersuchung in vivo wird eine einzelne Standarddosis (200 µg/kg) getestet. Für ausgewählte Substanzen wird das Ergebnis durch Erstellung einer Dosis-Wirkungs-Beziehung abgesichert.

Eine hypercalcämische Wirkung zeigt sich in im Vergleich Zur Kontrolle erhöhten Serum-Calciumspiegel-Werten.

Die Signifikanz auftretender Unterschiede zwischen Substanzgruppen und Kontrollen sowie zwischen Testsubstanz und Referenzsubstanz werden mit geeigneten statistischen Verfahren abgesichert. Das Ergebnis wird als Dosisrelation DR (DR = Faktor Testsubstanzdosis/Referenzsubstanzdosis für vergleichbare Wirkungen) angegeben.

Die differenzierungsstimulierende Wirkung von Calcitriolanaloga wird ebenfalls quantitativ erfaßt.
Es ist literaturbekannt (Mangelsdorf, D. J. et al., J. Cell. Biol. 98: 391-398 (1984)), daß die Behandlung humaner Leukämiezellen (Promyelozytenzellinie HL 60) in vitro mit Calcitriol die Differenzierung der Zellen Zu Makrophagen induziert.

HL 60-Zellen werden in Gewebekulturmedium (RPMI -10% fetales Kälberserum) bei 37° C in einer Atmosphäre 5% $CO_2$ in Luft kultiviert.

Zur Substanztestung werden die Zellen abzentrifugiert und 2,0 x $10^5$ Zellen/ml in phenolrotfreiem Gewebekulturmedium aufgenommen. Die Testsubstanzen werden in Ethanol gelöst und mit Gewebekulturmedium ohne Phenolrot auf die gewünschte Konzentration verdünnt. Die Verdünnungsstufen werden mit der Zellsuspension in einem Verhältnis von 1:10 gemischt und je 100 µl dieser mit Substanz versetzten Zellsuspension in eine Vertiefung einer 96-Loch-Platte pipettiert. Zur Kontrolle wird eine Zellsuspension analog mit dem Lösungsmittel versetzt.

Nach Inkubation über 96 Stunden bei 37° C in 5 % $CO_2$ in Luft wird in jede Vertiefung der 96-Loch-Platte zu der Zellsuspension 100 µl einer NBT-TPA-Lösung (Nitroblautetrazolium (NBT), Endkonzentration im Ansatz 1 mg/ml, Tetradecanoylphorbolmyristat-13-acetat (TPA), Endkonzentration im Ansatz 2 x $10^{-7}$ mol/l) pipettiert.

Durch Inkubation über 2 Stunden bei 37° C und 5% $CO_2$ in Luft wird infolge der intrazellulären Sauerstoffradikalfreisetzung, stimuliert durch TPA, in den zu Makrophagen differenzierten Zellen NBT zu unlöslichem Formazan reduziert.

Zur Beendigung der Reaktion werden die Vertiefungen der 96-Loch-Platte abgesaugt und die anhaftenden Zellen durch Zugabe von Methanol fixiert und nach Fixation getrocknet. Zur Lösung der gebildeten intrazellulären Formazankristalle werden in jede Vertiefung 100 µl Kaliumhydroxid (2 val/l) und 100 µl Dimethylsulfoxid pipettiert und 1 Minute ultrabeschallt. Die Konzentration von Formazan wird spektralphotometrisch bei 650 nm gemessen.
Als Maß für die Differenzierungsinduktion der HL 60-Zellen zu Makrophagen gilt die Konzentration an gebildetem Formazan. Das Ergebnis wird ebenfalls als Dosisrelation (DR = Faktor Testsubstanzdosis/Referenzsubstanzdosis für vergleichbare Wirkungen) angegeben.

Die Ergebnisse des Calcitriol-Rezeptortests sowie der Bestimmung der Dosisrelation der Differenzierungsinduktion von HL 60-Zellen und der Dosisrelation für Hypercalcämie sind nachfolgend zusammengefaßt:
(5Z,7E)-(1S,3R)-24-(3-Ethyl-3-hydroxypentyl)-23-oxa-9,10-secochola-5,7,10(19),20-tetraen-1,3-diol **16**
(5Z,7E)-(1S,3R)-26,27-Diethyl-20,21-methylen-23-oxa-9,10-secocholesta-5,7,10(19)-trien-1,3,25-triol **21**
(7E)-(1R,3R)-20,26,27-Trimethyl-19-nor-23-oxa-9,10-secocholesta-5,7-dien-1,3,25-triol **40**
(7E)-(1R,3R)-24-(3-Ethyl-3-hydroxypentyl)-19-nor-23-oxa-9,10-secochola-5,7,20 trien-1,3-diol **87**
(5Z,7E,22E)-(1S,3R)-24-(2-Hydroxy-2-methylpropoxy)-9,10-secochola-5,7,10(19),20,22-pentaen-1,3-diol **92**
(5Z,7E,22E)-(1S,3R)-24-(2-Ethyl-2-hydroxybutoxy)-9,10-secochola-5,7,10(19),20,22-pentaen-1,3-diol **93**
Vergleichsverbindung: Calcitriol

Biologische Daten der ausgewählten Verbindungen:

| Verbindung | $K_F$ (Rezeptor) | DR (HL 60) | DR (Hypercalcämie) |
|---|---|---|---|
| Calcitriol | 1 | 1 | 1 |
| **16** | 1,6 | 0,3 | 30 |
| **21** | 2,2 | 10 | 100 |
| **40** | 1,5 | 0,2 | 5 |
| **87** | 6,3 | 1 | 100 |
| **92** | 8,3 | 1,5 | 100 |
| **93** | 2,2 | 1 | 100 |

Durch das verminderte Hypercalciämie-Risiko eignen sich die erfindungsgemäßen Substanzen in besonderer Weise zur Herstellung von Arzneimitteln für die Behandlung von Erkrankungen, die durch eine Hyperproliferation von Zellen gekennzeichnet sind, z.B. hyperproliferative Erkrankungen der Haut (Psoriasis) und maligne Tumoren (Leukämie, Coloncarcinom, Mammacarcinom) und Akne (J. Invest. Dermatol., Vol. 92 No. 3, 1989). Auch Zur Behandlung und Prophylaxe von Störungen, die durch eine Störung des Gleichgewichts des Immunsystems gekennzeichnet sind, beispielsweise Autoimmunkrankheiten, einschließlich Diabetes mellitus und der Abstoßungsreaktionen bei Transplantationen (WO-A-91/00855), können die erfindungsgemäßen Verbindungen verwendet werden. In einer besonders bevorzugten Ausführungsform der Erfindung werden vor der Behandlung im Zielorgan Calcitriolrezeptoren nachgewiesen.
Weiterhin wurde überraschenderweise gefunden, daß durch topische Applikation der erfindungsgemäßen Verbindungen auf die Haut von Mäusen, Ratten und Meerschweinchen eine vermehrte Hautrötung und Zunahme der Epidermisdicke induziert werden kann. Die Zunahme der Hautrötung wird anhand der Erhöhung des mit einem Farbmeßgerät quantifizierbaren Rotwertes der Hautoberfläche ermittelt. Der Rotwert ist nach dreimaliger Subsstanzapplikation (Dosis 0,003%) im Abstand von 24 Stunden typischerweise um das 1,5-fache erhöht. Die Zunahme der Epidermisdicke wird im histologischen Präparat quantifiziert. Die Anzahl der proliferierenden Epidermiszellen (Zellen in der S-Phase des Zellcyclus) wird durchflußcytometrisch ermittelt und ist typischerweise um den Faktor 6 erhöht.
Diese Eigenschaften der erfindungsgemäßen Verbindungen lassen sie zum therapeutischen Einsatz bei atrophischer Haut, wie sie bei natürlicher Hautalterung, vorzeitiger Hautalterung infolge erhöhter Lichtexposition oder medikamentös induzierter Hautatrophie durch Behandlung mit Glucocorticoiden auftritt, geeignet erscheinen. Außerdem kann die Wundheilung durch topische Applikation mit den neuen Verbindungen beschleunigt werden.

Die vorliegende Erfindung bezieht sich somit auch auf pharmazeutische Präparate, die mindestens eine Verbindung gemäß der allgemeinen Formel I zusammen mit einem pharmazeutisch verträglichen Träger enthalten.

Die Verbindungen können formuliert werden als Lösungen in pharmazeutisch verträglichen Solventien oder als Emulsionen, Suspensionen oder Dispersionen in geeigneten pharmazeutischen Solventien oder Trägern oder als Pillen, Tabletten oder Kapseln, die in an sich bekannter Weise feste Trägerstoffe enthalten. Für eine topische Anwendung werden die Verbindungen vorteilhafterweise als Cremes oder Salben oder in einer ähnlichen, zur topischen Anwendung geeigneten Arzneimittelform formuliert. Jede derartige Formulierung kann auch andere pharmazeutisch verträgliche und nichttoxische Hilfsstoffe enthalten, wie z.B. Stabilisatoren, Antioxidantien, Bindemittel, Farbstoffe, Emulgatoren oder Geschmackskorrigentien. Die Verbindungen werden vorteilhafterweise durch Injektion oder intravenöse Infusion geeigneter steriler Lösungen oder als orale Dosierung über den Ernährungstrakt oder topisch in der Form von Cremes, Salben, Lotions oder geeigneter transdermaler Pflaster appliziert, wie in der EP-A 0 387 077 beschrieben ist.

Die tägliche Dosis liegt bei

0,1 µg/Patient/Tag - 1000 µg (1 mg)/Patient/Tag,

vorzugsweise

1,0 µg/Patient/Tag - 500 µg/Patient/Tag.

Die erfindungsgemäßen Verbindungen werden im allgemeinen verabreicht analog zur Verabreichung des bekannten Mittels "Calcipotriol" zur Behandlung der Psoriasis.

Außerdem betrifft die Erfindung die Verwendung der Verbindungen gemäß Formel I zur Herstellung von Arzneimitteln.

Die Verbindungen der allgemeinen Formel I und insbesondere die zu deren Herstellung benötigten Ausgangsverbindungen werden nach neuen Verfahren hergestellt. Die Erfindung betrifft daher auch Verfahren zur Herstellung dieser Verbindungen.

Die nachstehenden Verbindungen der allgemeinen Formel I'

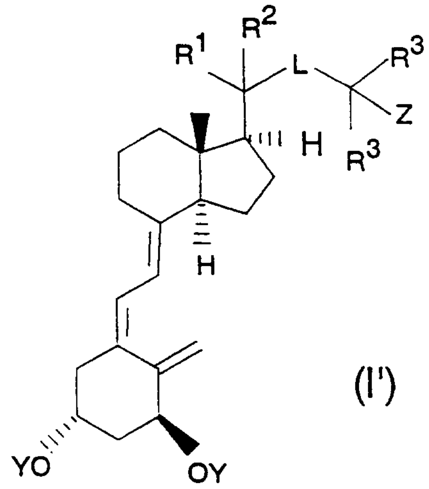

(I')

leiten sich aus I ab, wobei die beiden in der allgemeinen Formel I mit X bezeichneten Substituenten eine exocyclische Methylengruppe bilden.

Startmaterial zu deren Herstellung sind die literaturbekannten Verbindungen der allgemeinen Formel VII (siehe WO 90/09991, Leo Pharmaceutical Products); zur Herstellung der Verbindung 1 (Beispiel 1.) wird tert.-Butyldiphenylsilylchlorid analog anstelle von tert.-Butyldimethylsilylchlorid verwendet.

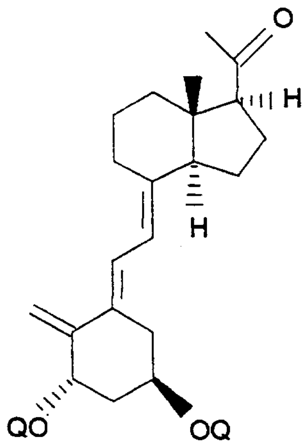

(VII)

worin Q alkyl- oder aryl- oder alkyl- und arylsubstituierte (gemischt-substituierte) Silylgruppen bedeuten. Als Beispiele seien genannt: Tert.-Butyl-dimethylsilyl-, Trimethylsilyl-, tert.-Butyl-diphenylsilyl-, Triphenylsilyl-.

Durch Reaktion mit Schwefelyliden, die aus Reagenzien des Typs $Me_3S^+I^-$ oder $Me_3S^+(O)I^-$ durch Deprotonierung mit einer Base wie Kalium-tert.-butanolat (KOtBu), NaH oder KH erzeugt werden, erhält man die Verbindung VIII, wobei die Stereochemie an C-20 nicht einheitlich sein muß. Die Reaktion wird in einem polaren, aprotischen Solvens, z. B. in Dimethylformamid, Dimethylsulfoxid oder Tetrahydrofuran durchgeführt.

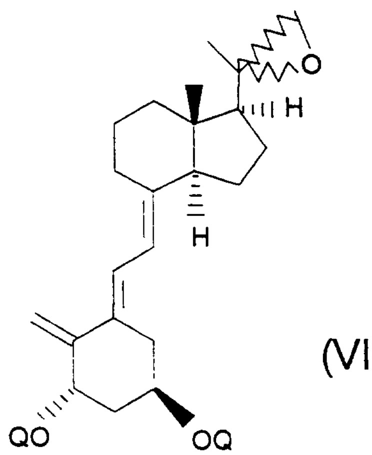

(VIII)

Durch Umlagerung der Epoxide VIII mit Basen wie z. B. Lithiumdiisopropylamid (LDA), Lithiumdiethylamid ($LiNEt_2$), Lithium-bis(trimethylsilylamid ($LiN(TMS)_2$), Aluminiumisopropylat ($Al(OiPr)_3$) erhält man die Allylalkohole IX, die flexibel zu den Verbindungen der allgemeinen Formel I umgesetzt werden können. Derartige Reaktionen an Steroiden sind beispielsweise in Liebigs Ann. 2119 (1982) von P. Welzel, H. Stein und T. Milkowa beschrieben.

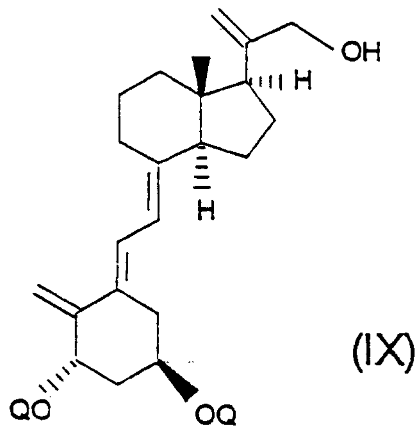

(IX)

Zur Synthese der Verbindungen der allgemeinen Formel I', in denen $R^1$ und $R^2$ zusammen eine Methylengruppe bilden, L für die Gruppierung

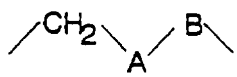

steht und A ein Sauerstoffatom ist, wird IX mit einer Verbindung der allgemeinen Formel X

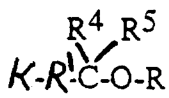

(X),

worin
K für eine Abgangsgruppe wie Br, I, $CH_3C_6H_4SO_2O$,
$R^1$ für einen Alkylenrest $-(CH_2)_n-$ mit n=1, 2 oder 3, sowie
R für einen geradkettigen oder verzweigten Alkylrest mit 1 bis 8 Kohlenstoffatomen sowie
$R^4$ und $R^5$ ebenfalls je für einen Rest OR oder $R^4$ und $R^5$ gemeinsam für ein Sauerstoffatom stehen,
unter Erhalt einer Verbindung der allgemeinen Formel XI

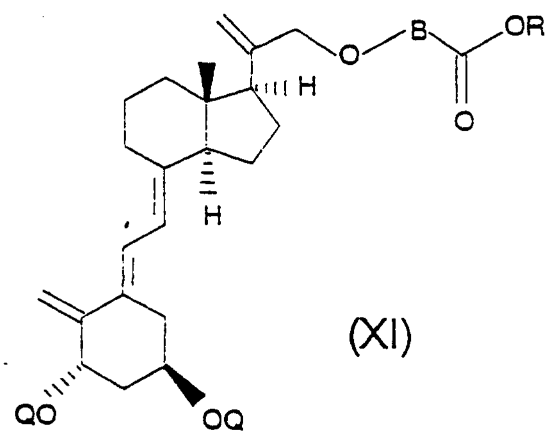

(XI)

verethert (DE-A-41 01 953 und WO-A-92/12 963).
An deren Carbonylgruppe wird ein nucleophiles Reagenz der allgemeinen Formel XII

$R^3$-M (XII),

worin $R^3$ eine lineare oder verzweigte Alkylgruppe mit 1 bis 5 Kohlenstoffatomen und M MgHal (Hal = Cl, Br, I) oder ein Alkaliatom (Li, Na, K) bedeutet, unter Bildung einer Verbindung der allgemeinen Formel XIII

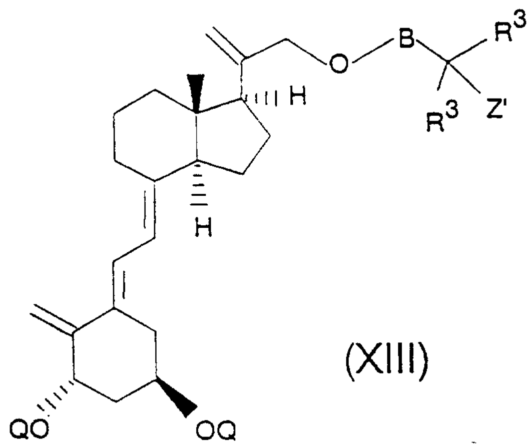

(XIII)

addiert, worin Z' eine Hydroxylgruppe bedeutet.

Die Verbindung XIII wird durch photochemische Isomerisierung des Triensystems in Gegenwart eines Triplettsensibilisators in eine Verbindung der allgemeinen Formel XIV überführt.

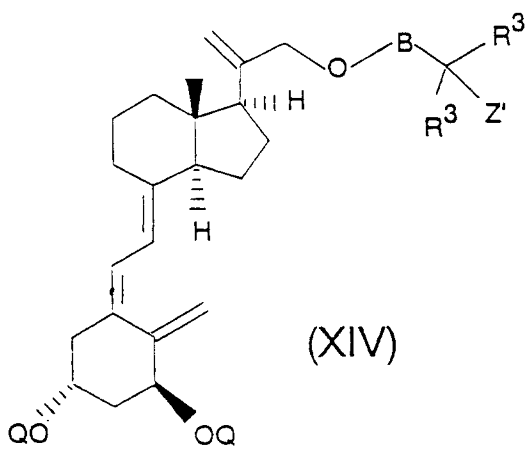

(XIV)

Die Silylgruppen werden abgespalten und anschließend werden gegebenenfalls die freien Hydroxylgruppen teilweise oder vollständig mit einem Alkancarbonsäurechlorid, -bromid oder -anhydrid, welche im Alkanoylrest 1 bis 9 Kohlenstoffatome tragen, oder mit Benzoylchlorid, verestert.

Die Umwandlung einer Verbindung der allgemeinen Formel XIII in eine Verbindung der allgemeinen Formel XIV erfolgt z.B. durch Bestrahlung mit ultraviolettem Licht in Gegenwart eines sogenannten "Triplettsensibilisators". Im Rahmen der vorliegenden Erfindung wird hierfür Anthracen verwendet. Durch Spaltung der pi-Bindung der 5,6-Doppelbindung, Rotation des A-Ringes um 180° um die 5,6-Einfachbindung und Reetablierung der 5,6-Doppelbindung wird die Stereoisomerie an der 5,6-Doppelbindung umgekehrt.

Anschließend werden vorhandene Hydroxyschutzgruppen abgespalten, vorzugweise unter Verwendung von Tetra-n-butyl-ammoniumfluorid sowie gewünschtenfalls die freien Hydroxygruppen nach gängigen Verfahren partiell oder vollständig mit dem entsprechenden Carbonsäurehalogenid (Halogenid = Chlorid, Bromid) oder Carbonsäureanhydrid verestert.

Zur Synthese der Verbindungen der allgemeinen Formel I', worin $R^1$ und $R^2$ zusammen mit dem quartären Kohlenstoffatom 20 einen Cyclopropylring bilden und L für die Gruppierung

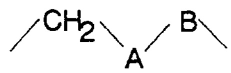

steht, wird der Allylalkohol IX zunächst analog zur Umsetzung XIIIXIV photochemisch zu XV isomerisiert und anschließend mit einem metallorganischen Reagenz vom Typ I-$CH_2$-Zn-I, das aus Zn/Cu, Zn/Ag oder $Et_2Zn$ (Diethylzink) mit $CH_2I_2$ gebildet wird, zur Verbindung der Formel XVI umgesetzt (Simmons-Smith-Reaktion; u. a. J. M. Denis, C. Girard, J. M. Conia, Synthesis 549 (1972)).

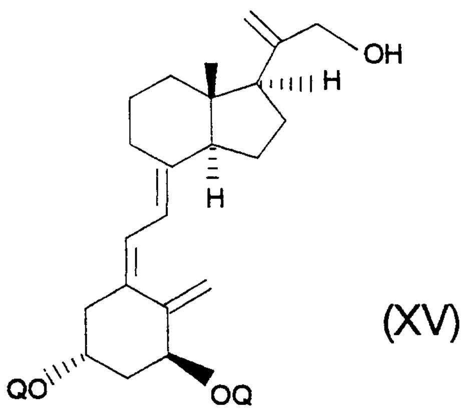

(XV)

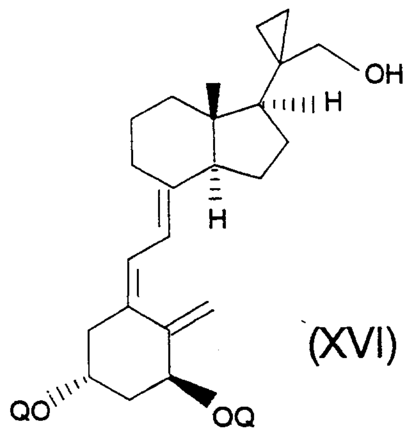

(XVI)

Analog zu den vorstehend beschriebenen Reaktionen wird XVI über die Zwischenstufen der allgemeinen Formeln XVII und XVIII in eine Verbindung der allgemeinen Formel IXX umgewandelt, wobei für B, Q und Z' die bereits beschriebenen Definitionen und Umwandlungsmöglichkeiten gelten.

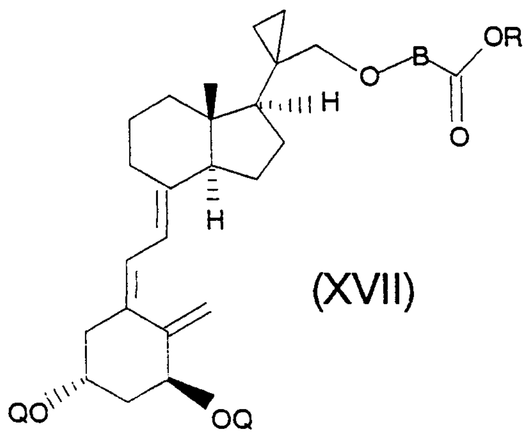
(XVII)

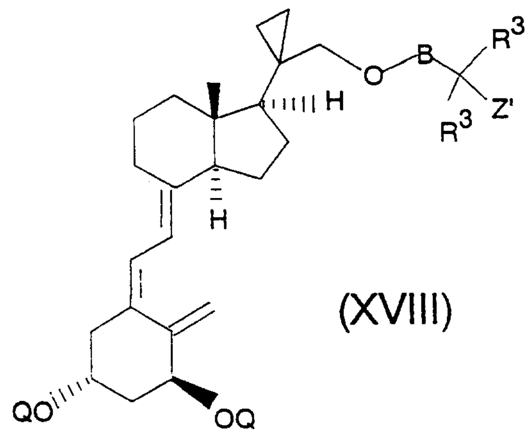
(XVIII)

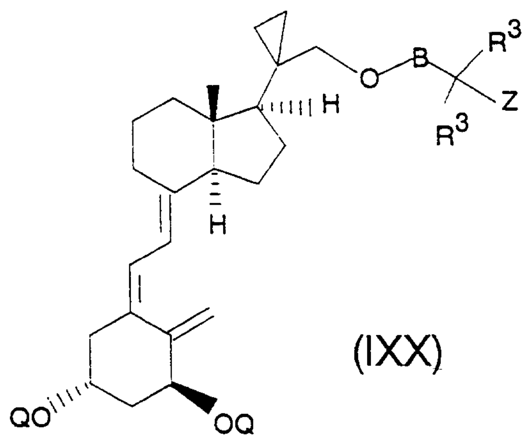
(IXX)

Zur Synthese der Verbindungen der allgemeinen Formel I",

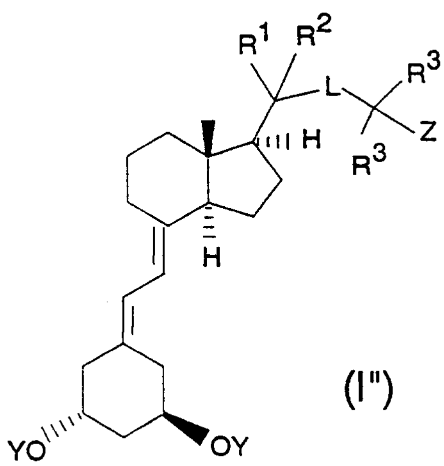
(I")

in denen die beiden Reste X der allgemeinen Formel I Wasserstoffatome sind, und L für eine Gruppierung

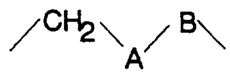

oder

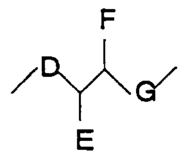

steht,
benutzt man einen konvergenten Syntheseweg (CD-Teil und A-Teil werden separat aufgebaut).

Als Ausgangsmaterial wird der literaturbekannte Aldehyd der allgemeinen Formel XX verwendet (H.H. Inhoffen et al. Chem. Ber. 91, 780 (1958), Chem. Ber. 92, 1772 (1959)),

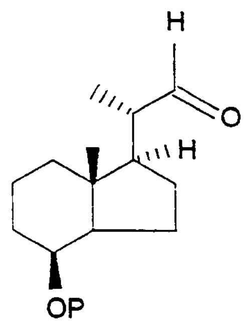

(XX)

worin P ein Wasserstoffatom, eine Alkanoylgruppe mit 1 bis 9 Kohlenstoffatomen, eine Tetrahydropyranyl- oder Tetrahydrofuranylgruppe, eine alkyl- oder arylsubstituierte oder eine alkyl- und arylsubstituierte (gemischt-substituierte) Silylgruppe bedeutet. Die Alkanoylgruppe stammt vorzugsweise von gerad- oder verzweigtkettigen, gesättigten Carbonsäuren ab; als bevorzugte Vertreter seien beispielhaft die Acetyl- sowie die Pivaloylgruppe genannt. Als Silylgruppen kommen insbesondere folgende Gruppen in Betracht: Tert.-Butyl-dimethylsilyl-, Trimethylsilyl-, tert.-Butyl-diphenylsilyl-, Triphenylsilyl-.

Zur Synthese von Verbindungen der allgemeinen Formel I'', worin $R^1$ und $R^2$ je eine Methylgruppe bedeutet, wird XX mit einer Base wie NaH, KH, Lithiumdiisopropylamid (LDA), Kalium-tert.-butanolat (KOtBu) deprotoniert und mit einem elektrophilen Reagenz $CH_3X$ (X=Cl, Br, I, $CH_3C_6H_4SO_2O$) zur Verbindung XXI umgesetzt (DE-A-41 41 746 und PCT/EP92/02887).

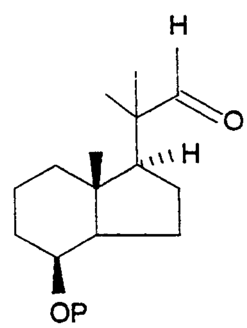

(XXI)

Durch Reduktion der Carbonylgruppe in XXI mit einem Reduktionsmittel wie $NaBH_4$, $NaBH_4/CeCl_3$, $LiAlH_4$ oder Diisobutylaluminiumhydrid erhält man die Verbindung XXII,

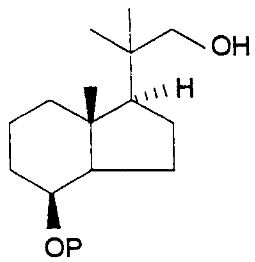

(XXII)

die mit der schon vorher beschriebenen Verbindung der allgemeinen Formel X unter Erhalt einer Verbindung der allgemeinen Formel XXIII verethert wird.

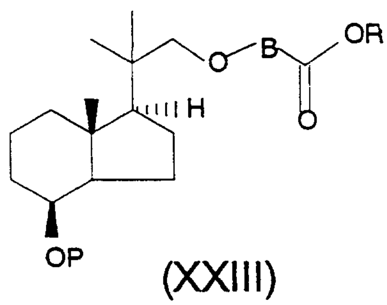

(XXIII)

An die Carbonylgruppe in XXIII wird ein nukleophiles Reagenz der allgemeinen Formel XII addiert, wodurch man eine Verbindung der allgemeinen Formel XXIV

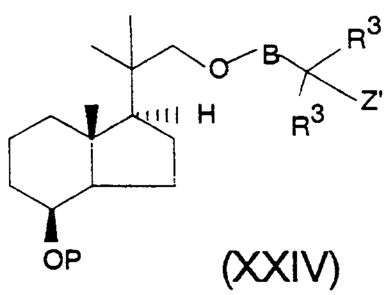

(XXIV)

erhält,
worin Z' eine Hydroxylgruppe bedeutet.

Eine in XXIV gegebenenfalls vorhandene Schutzgruppe P wird abgespalten; im Falle einer Acylgruppe unter basischen Bedingungen ($K_2CO_3$/Methanol, KOH oder NaOH/Methanol), im Falle einer Silylschutzgruppe mit Fluorid-Reagenzien (Tetrabutylammoniumfluorid, HF, HF/Pyridin) und im Falle der Tetrahydropyranyl- oder Tetrahydrofuranyl-Ether-Schutzgruppe unter Säurekatalyse (p-Toluolsulfonsäure, PPTS, Ionentauscher) unter Erhalt einer Verbindung der allgemeinen Formel XXV,

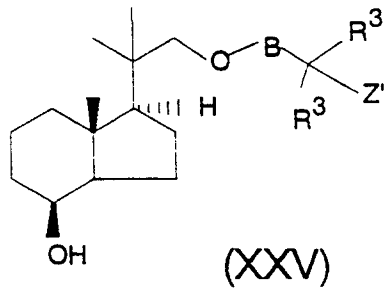

(XXV)

deren sekundäre Hydroxylgruppe nach Standardverfahren mit einem Oxidationsmittel (Pyridiniumchlorochromat [PCC], Pyridiniumdichromat [PDC], Collins-Reagenz, BaMnO4) oxidiert und deren tertiäre Hydroxylgruppe Z' z.B. als Silylether, vorzugsweise als Trimethylsilylether, geschützt wird, wobei eine Verbindung der allgemeinen Formel XXVI entsteht,

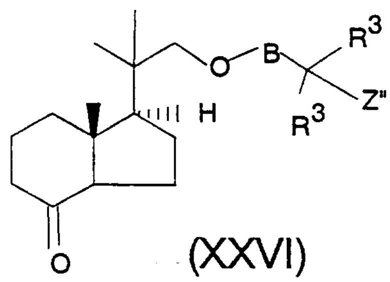

(XXVI)

worin Z" eine Silyloxy-, vorzugsweise die Trimethylsilyloxygruppe, eine Tetrahydropyranyl- oder Tetrahydrofuranylgruppe bedeutet.

Durch Reaktion mit dem durch eine Base wie n-Butyllithium (BuLi) oder Lithiumdiisopropylamid (LDA) erzeugten Anion des literaturbekannten Phosphinoxids XXVII (H.F. DeLuca, Tetrahedron Lett. 32, 7663 (1991)),

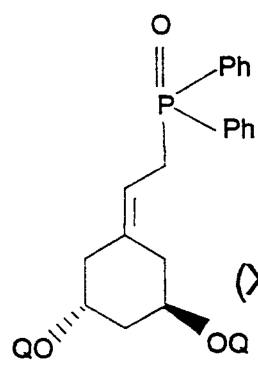

(XXVII)

worin Q alkyl- oder arylsubstituierte Silylgruppen bedeuten, erhält man eine Verbindung der allgemeinen Formel XXVIII,

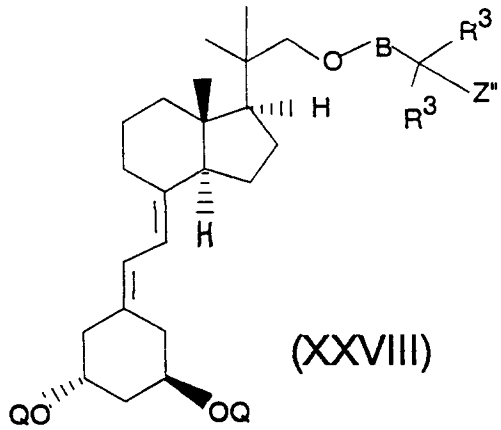

(XXVIII)

deren Schutzgruppen Q und Z" wie vorstehend beschrieben gespalten und die freien Hydroxylgruppen gegebenenfalls acyliert werden.

Zur Synthese von Verbindungen der allgemeinen Formel I", worin $R^1$ und $R^2$ zusammen eine Methylengruppe oder gemeinsam mit dem quartären Kohlenstoffatom 20 eine Cyclopropyleinheit bilden, wird der literaturbekannte Aldehyd XX analog der Darstellung von VII zum Keton V abgebaut,

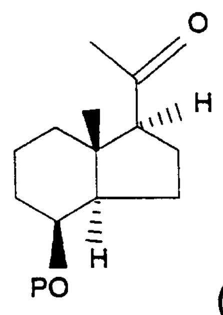

(V)

worin P die schon beschriebene Bedeutung hat.

Analog der Sequenz VII→VIII→IX wird V über die Zwischenstufe der allgemeinen Formel XXIX in den Allylalkohol der allgemeinen Formel VI überführt.

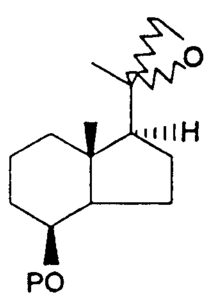

(XXIX)

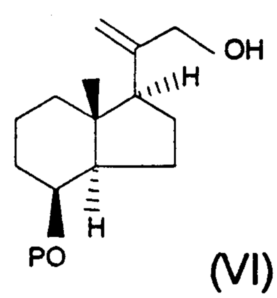

(VI)

In einer Simmons-Smith-Reaktion (Bedingungen analog XV→XVI) wird aus VI die Verbindung der allgemeinen Formel XXX erhalten.

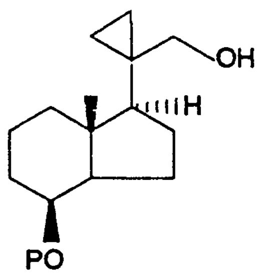

(XXX)

Zur Synthese von Verbindungen der allgemeinen Formel I", worin L für

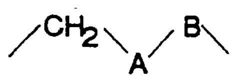

steht und A ein Sauerstoffatom ist und $R^1$ und $R^2$ zusammen eine Methylengruppe oder gemeinsam mit dem quartären Kohlenstoffatom 20 eine Cyclopropyleinheit bilden, werden VI bzw. XXX mit einer Verbindung der allgemeinen Formel X

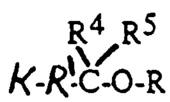

(X),

unter Erhalt einer Verbindung der allgemeinen Formel XXXI umgesetzt,

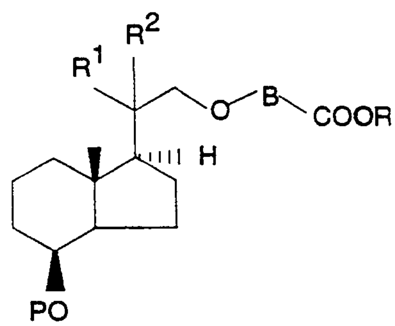

(XXXI)

worin $R^1$ und $R^2$ sowie P die bereits angegebenen Bedeutungen haben.

Die Umwandlung einer Verbindung der allgemeinen Formel XXXI in das Keton der allgemeinen Formel XXXIV erfolgt wie beschrieben über die Zwischenprodukte der allgemeinen Formeln XXXII und XXXIII.

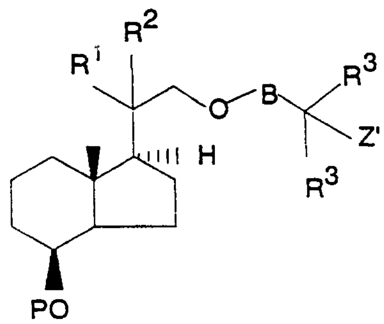

(XXXII)

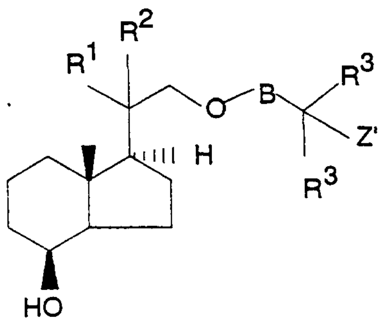

(XXXIII)

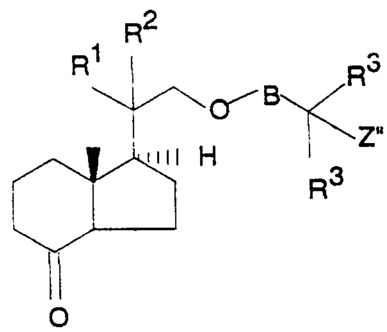

(XXXIV)

Wie beschrieben wird nun das Keton der allgemeinen Formel XXXIV mit dem literaturbekannten Phosphinoxid XXVII gekuppelt, wobei man eine Verbindung der allgemeinen Formel XXXV erhält,

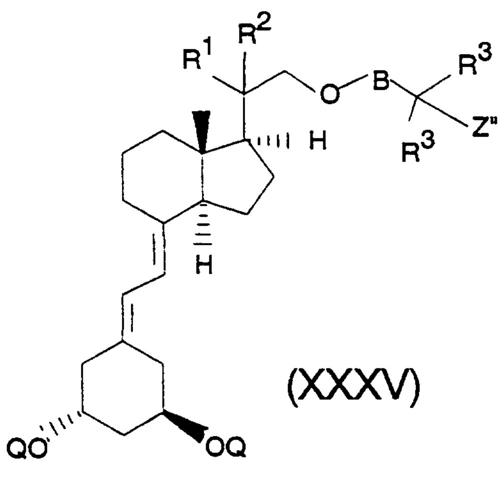

(XXXV)

deren Schutzgruppen wie vorstehend beschrieben gespalten und deren freie Hydroxygruppen gegebenenfalls acyliert werden.

Zur Synthese von Verbindungen der allgemeinen Formel I', in denen L für

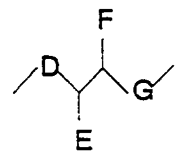

steht und D eine direkte Bindung zwischen den Kohlenstoffatomen 20 und 22, E und F eine E-Doppelbindung und G eine $CH_2$-O-$CH_2$-Einheit bedeuten, wird z. B. ein Alkohol der allgemeinen Formel XV mit einem Oxidationsmittel (Mangandioxid, Pyridiniumchlorochromat, Pyridiniumdichromat, Bariummanganat) zum Aldehyd der allgemeinen Formel XXXVI oxidiert.

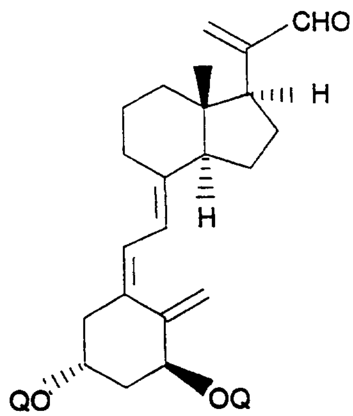

(XXXVI)

Durch Wadsworth-Emmons-Reaktion (Org. React. 25, 73 (1977)) mit einem durch Deprotonierung mit einer Base (NaH, KH, Lithiumdiisopropylamid, Kalium-tert.-butanolat) erzeugten Anion eines Phosphonates der allgemeinen Formel XXXVII

$(RO)_2P(O)$-$CH_2$-COOR' (XXXVII),

worin R und R' unabhängig voneinander geradkettige oder verzweigte Alkylgruppen mit bis zu 9 Kohlenstoffatomen oder Phenylgruppen bedeuten, erzeugt man eine Verbindung der allgemeinen Formel XXXVIII,

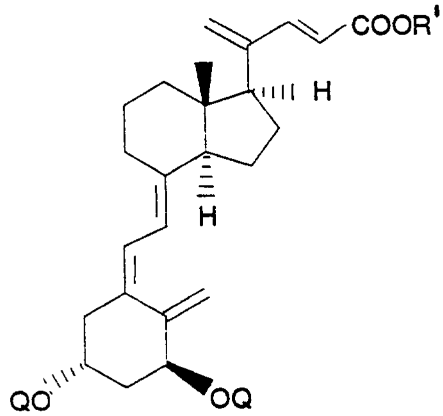

(XXXVIII)

deren Estergruppe mit einem Reduktionsmittel ($LiAlH_4$, Diisobutylaluminiumhydrid [DIBAH]) zu einem Alkohol der allgemeinen Formel XXXIX reduziert wird.

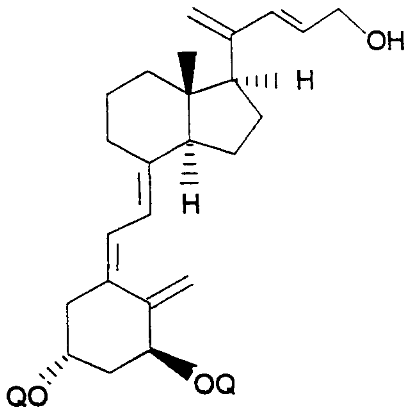

(XXXIX)

Durch Veretherung mit einer der schon vorher beschriebenen Verbindungen der allgemeinen Formel X wird eine Verbindung der allgemeinen Formel XL erhalten.

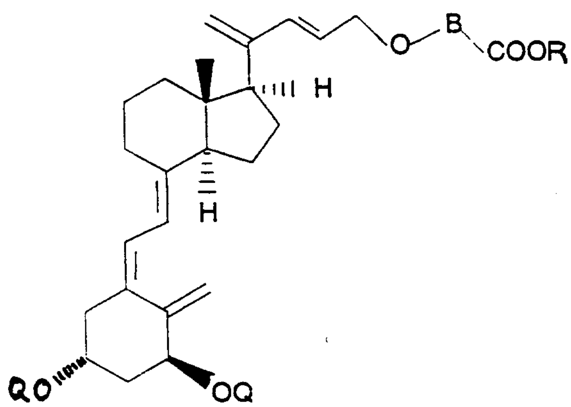

(XL)

An deren Carbonylgruppe wird ein nukleophiles Reagenz der schon beschriebenen allgemeinen Formel $R_3$-M (XII) addiert, wobei eine Verbindung der allgemeinen Formel XLI entsteht,

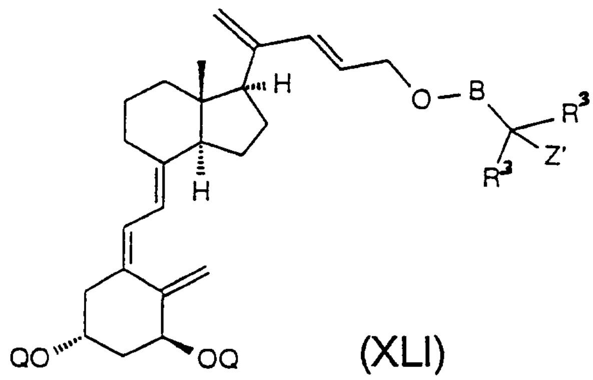

(XLI)

wobei für B, Q und Z' die bereits beschriebenen Definitionen und Umwandlungsmöglichkeiten gelten.

Zur Synthese der Verbindungen der allgemeinen Formel I'', worin L für

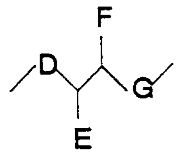

steht und die Reste X der allgemeinen Formel I Wasserstoffatome sind und in denen D, E und F sowie G die vorstehend beschriebenen Bedeutungen haben, wird ein Allylalkohol der allgemeinen Formel VI, analog wie für die Synthese der Verbindung der allgemeinen Formel XLI beschrieben, zu einer Verbindung der allgemeinen Formel XLII oxidiert.

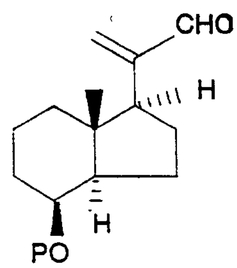

(XLII)

Der Aufbau der Seitenketten erfolgt analog wie schon für die Herstellung der Verbindungen der allgemeinen Formel XLI beschrieben, über die Zwischenstufen der allgemeinen Formeln XLIII, XLIV, und XLV zu XLVI,

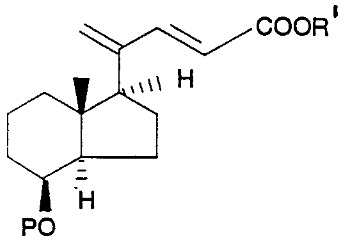

(XLIII)

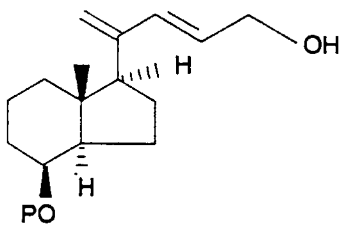

(XLIV)

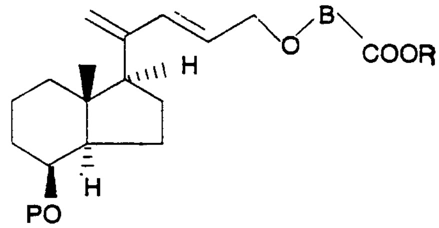

(XLV)

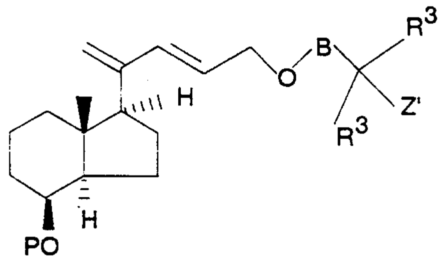

(XLVI)

wobei für B, Q und Z' wiederum die bereits angegebenen Definitionen und Umwandlungsmöglichkeiten gelten.

Durch Verwendung schon beschriebener Methoden erfolgt aus einer Verbindung der allgemeinen Formel XLVI der Aufbau des Ketons der allgemeinen Formel XLVIII über das Zwischenprodukt der allgemeinen Formel XLVII.

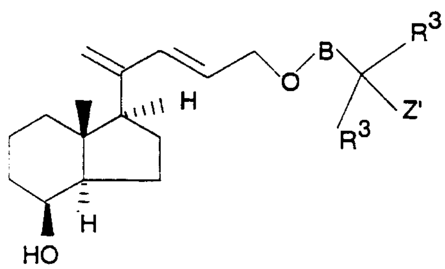

(XLVII)

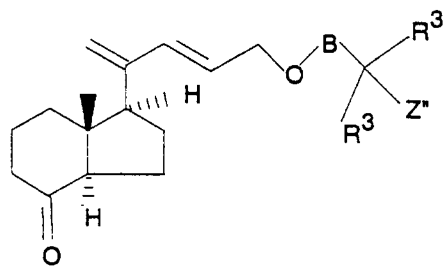

(XLVIII)

Ein Keton der allgemeinen Formel XLVIII wird analog wie die Verbindung der allgemeinen Formel XXVI mit dem literaturbekannten Phosphinoxid XXVII gekuppelt, wobei man eine Verbindung der allgemeinen Formel IL erhält,

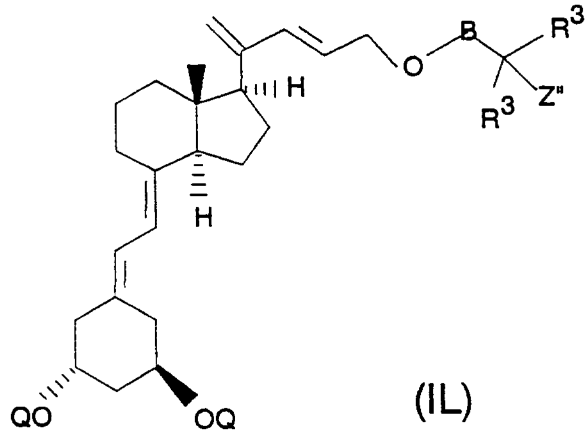

(IL)

deren Schutzgruppen wie vorstehend gespalten und deren freie Hydroxylgruppen gegebenenfalls acyliert werden.

Die vorliegende Erfindung betrifft auch die neuen Zwischenprodukte der allgemeinen Formeln IX und XV,

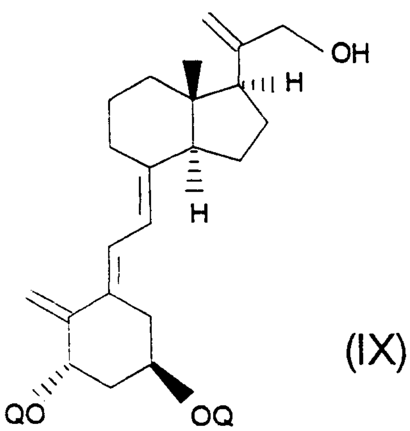

(IX)

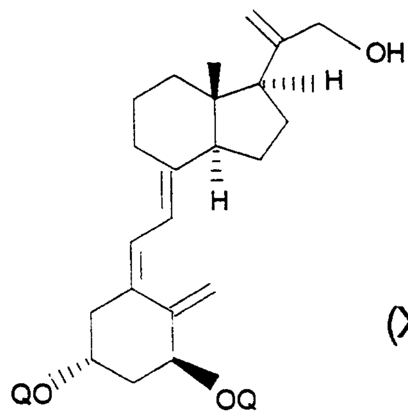

(XV)

worin Q die bereits angegebene Bedeutung hat.

Insbesondere betrifft sie folgende Zwischenverbindungen:

(5E,7E)-(1S,3R)-1,3-Bis[[(1,1-dimethylethyl)diphenylsilyl]oxy]-20-methylen-9,10-secopregna-5,7,10(19)-trien-21-ol,

(5Z,7E)-(1S,3R)-1,3-Bis[[(1,1-dimethylethyl)diphenylsilyl]oxy]-20-methylen-9,10-secopregna-5,7,10(19)-trien-21-ol,

Die nachfolgenden Beispiele dienen der näheren Erläuterung der Erfindung:

**Beispiele:**

**1. (5*E*,7*E*)-(*1S*,3*R*,20*R*)-1,3-Bis[[(1,1-dimethylethyl)diphenylsilyl]oxy]-20,21-epoxy-20-methyl-9,10-secopregna-5,7,10(19)-trien 2**

3,1 g (3,84 mmol) (5*E*,7*E*)-(1*S*,3*R*)-Bis[[(1,1-dimethylethyl)diphenylsilyl]oxy]-9,10-secopregna-5,7,10(19)-trien-20-on 1 (Bis-TBDMS-Ether siehe WO 90/09991, Leo Pharmaceutical Products) werden in 70 ml Dimethylformamid unter Argon gelöst und mit 1,06 g (5,2 mmol) Trimethylsulfoniumiodid versetzt. Man kühlt auf 0°C und gibt portionsweise 0,51 g (5,2 mmol) Kalium-tert.-butylat zu. Nach 15 Min. bei 0°C wird gesättigte Natriumchlorid-Lösung zugegeben, mit Essigester extrahiert und die organische Phase mehrmals mit Natriumchlorid-Lösung gewaschen. Nach Trocknen über Natriumsulfat entfernt man das Solvens und reinigt den Rückstand an Kieselgel mit Hexan/Essigester, wobei 2,2 g der Titelverbindung als farbloser Schaum anfallen.

$^1$H-NMR ($CDCl_3$): δ= 0,58 ppm (s, 3H, H-18); 0,89 u. 0,94 (2x s, je 9H, Si-t-Butyl); 1,32 (s, 3H, H-21); 2,31 u. 2,50 (2x d, J=5 Hz, je 1H, H-22 u. H-22'); 4,19 (m, 1H, H-3); 4,59 (t, J=5,5 Hz, 1H, H-1); 4,70 u. 4,82 (2x s, je 1H, H-19 u. H-19'); 5,57 u. 6,31 (2x d, J=11 Hz, je 1H, H-6 u. H-7); 7,12-7,68 (m, 20H, Si-Phenyl) [Es wird durchgängig die Steroid-Nummerierung verwendet]

**2. (5*E*,7*E*)-(1*S*,3*R*)-1,3-Bis[[(1,1-dimethylethyl)diphenylsilyl]oxy]-20-methylen-9,10-secopregna-5,7,10(19)-trien-21-ol 3**

Man löst 0,28 g (3,8 mmol) Diethylamin unter Argon in 35 ml Diethylether und gibt bei 0°C 2,4 ml (3,8 mmol) n-Butyllithium-Lösung (1,6 M in Hexan) zu. Nach 30 Min. bei dieser Temperatur tropft man 0,72 g (0,88 mmol) 2 in 5 ml Diethylether zu und rührt 1 Std. bei 0°C und 1 Std. bei Raumtemperatur nach. Anschließend wird mit Natriumchlorid-Lösung versetzt, mit Essigester extrahiert und die organische Phase mit Natriumchlorid-Lösung gewaschen. Nach Trocknen über Natriumsulfat engt man ein und chromatographiert den Rückstand an Kieselgel mit Hexan/Essigester, wobei man 360 mg der Titelverbindung als farblosen Schaum neben 280 mg des Ausgangsproduktes erhält.
$^1$H-NMR ($CDCl_3$): δ= 0,45 ppm (s, 3H, H-18); 0,99 u. 1,00 (2x s, je 9H, Si-t-Butyl); 4,08 u. 4,17 (2x d, J=14,5 Hz, je 1H, H-22 u. H-22'); 4,29 (m, 1H, H-3); 4,65 (m, 1H, H-1); 4,75 u. 4,90 (2x s, je 1H, H-19 u. H-19'); 5,03 u. 5,23 (2x s, je 1H, H-21 u. H-21'); 5,67 u. 6,39 (2x d, J=11 Hz, je 1H, H-6 u. H-7); 7,20-7,62 (m, 20H, Si-Phenyl)

**3. (5*E*,7*E*)-(1*S*,3*R*)-1,3-Bis[[(1,1-dimethylethyl)diphenylsilyl]oxy]-23-oxa-9,10-secochola-5,7,10(19),20-tetraen-24-carbonsäure-1,1-dimethylethylester 4**

Man legt 800 mg (0,97 mmol) 3 in 3 ml Toluol vor und gibt 4,6 ml wässrige Natriumhydroxid-Lösung (25%), 1,45 g (7,4 mmol) Bromessigsäure-tert.-butylester und 22 mg Tetrabutylammoniumhydrogensulfat unter Argon zu. Es wird über Nacht bei Raumtemperatur gerührt und anschließend auf Natriumchlorid-Lösung gegossen. Nach Extraktion mit Essigester, Waschen der organischen Phase mit Natriumchlorid-Lösung, Trocken über Natriumsulfat und Entfernen des Lösungsmittels reinigt man das Rohprodukt an Kieselgel mit Hexan/Essigester, wobei 640 mg der Titelverbindung als farbloser Schaum anfallen.
$^1$H-NMR ($CDCl_3$): δ= 0,43 ppm (s, 3H, H-18); 0,96 u. 0,98 (2x s, je 9H, Si-t-Butyl); 1,50 (s, 9H, t-Butylester); 3,98 (s, 2H, H-24); 4,02 (sbr, 2H, H-22); 4,29 (m, 1H, H-3); 4,63 (m, 1H, H-1); 4,72 u. 4,89 (2x s, je 1H, H-19 u. H-19'), 5,07 u. 5,23 (2x s, je 1H, H-21 u. H-21'); 5,65 u. 6,39 (2x d, J=11 Hz, je 1H, H-6 u. H-7); 7,24-7,65 (m, 20H, Si-Phenyl)
IR (KBr): ν= 1750 $cm^{-1}$

**4. (5*E*,7*E*)-(1*S*,3*R*)-1,3-Bis[[(1,1-dimethylethyl)diphenylsilyl]oxy]-23-oxa-9,10-secocholesta-5,7,10(19),20-tetraen-25-ol 5**

Aus 90 mg (3,7 mmol) Magnesiumspänen und 522 mg (3,7 mmol) Jodmethan wird in 5 ml Diethylether das Grignard-Reagenz bereitet. Bei 0°C gibt man nun 350 mg (0,37 mmol) 4 in 2 ml Tetrahydrofuran zu und rührt 1 Std. bei Raumtemperatur nach. Es wird mit Ammoniumchlorid-Lösung hydrolysiert, die wässrige Phase mit Essigester extrahiert, die organische Phase mit Natriumchlorid-Lösung gewaschen und über Natriumsulfat getrocknet. Nach Entfernen des Lösungsmittels chromatographiert man den Rückstand an Kieselgel mit Hexan/Essigester und erhält 125 mg der Titelverbindung als farblosen Schaum.
$^1$H-NMR ($CDCl_3$); δ= 0,43 ppm (s, 3H, H-18); 0,98 (s, 18H, Si-t-Butyl); 1,20 (s, 6H, H-26 u. H-27); 3,21 (d, J=9,5 Hz, 1H, H-24); 3,28 (d, J=9,5 Hz, 1H, H-24'); 3,94 (d, J=12,5 Hz, 1H, H-22); 4,01 (d, J=12,5 Hz, 1H, H-22'); 4,30 (m, 1H, H-3); 4,53 (m, 1H, H-1); 4,69 u. 4,89 (2x s, je 1H, H-19 u. H-19'); 5,02 u. 5,20 (2x s, je 1H, H-21 u. H-21'); 5,67 u. 6,41 (2x d, J=11 Hz, je 1H, H-6 u. H-7); 7,25-7,65 (m, 20 H, Si-Phenyl)

**5. (5*E*,7*E*)-(1*S*,3*R*)-1,3-Bis[[(1,1-dimethylethyl)diphenylsilyl]oxy]-26,27-dimethyl-23-oxa-9,10-secocholesta-5,7,10(19),20-tetraen-25-ol 6**

Aus 350 mg (3,2 mmol) Bromethan und 78 mg (3,2 mmol) Magnesiumspänen bereitet man in 5 ml Tetrahydrofuran unter Argon das Grignard-Reagenz und setzt analog **4.** mit 310 mg (0,33 mmol) 4 um. Man erhält 270 mg der Titelverbindung als farblosen Schaum.
$^1$H-NMR ($CDCl_3$): δ= 0,46 ppm (s, 3H, H-18); 0,90 (t, J=7 Hz, 6H, H-28 u. H-29); 0,96 u. 0,98 (2x s, je 9H, Si-t-Butyl); 1,56 (q, J=7 Hz, 4H, H-26 u. H-27); 3,27 u. 3,32 (2x d, J=10 Hz, je 1H, H-24 u. H-24'); 3,92 u. 4,01 (2x d, J=13 Hz, je 1H, H-22 u. H-22'); 4,29 (m, 1H, H-3); 4,64 (m, 1H, H-1); 4,73 u. 4,90 (2x s, je 1H, H-19 u. H-19'); 5,03 u. 5,20 (2x s, je 1H, H-21 u. H-21'); 5,65 u. 6,39 (2x d, J=11 Hz, je 1H, H-6 u. H-7); 7,24-7,64 (m, 20H, Si-Phenyl)

**6. (5*E*,7*E*)-(1*S*,3*R*)-1,3-Bis[[(1,1-dimethylethyl)diphenylsilyl]oxy]-26,27-diethyl-23-oxa-9,10-secocholesta-5,7,10(19),20-tetraen-25-ol 7**

Aus 390 mg (3,2 mmol) 1-Brompropan und 78 mg (3,2 mmol) Magnesiumspänen bereitet man in 5 ml Tetrahydrofuran unter Argon das Grignard-Reagenz und setzt analog **4.** mit 310 mg (0,33 mmol) 4 um. Es werden 265 mg der Titelverbindung als farbloser Schaum isoliert.
$^1$H-NMR ($CDCl_3$): δ= 0,44 ppm (s, 3H, H-18); 0,92 (t, J=7 Hz, 6H, H-30 u. H-31); 0,96 u. 0,98 (2x s, je 9H, Si-t-Butyl);

3,24 u. 3,30 (2x d, J=11 Hz, je 1H, H-24 u. H-24'); 3,91 u. 4,00 (2x d, J=13 Hz, je 1H, H-22 u. H-22'); 4,29 (m, 1H, H-3); 4,63 (m, 1H, H-1); 4,73 u. 4,90 (2x s, je 1H, H-19 u. H-19'); 5,02 u. 5,20 (2x s, je 1H, H-21 u. H-21'); 5,65 u. 6,39 (2x d, J=11 Hz, je 1H, H-6 u. H-7); 7,23-7,63 (m, 20H, Si-Phenyl)

**7. (5*Z*,7*E*)-(1*S*,3*R*)-23-Oxa-9,10-secocholesta-5,7,10(19),20-tetraen-1,3,25-triol 8**

Man löst 125 mg (0,14 mmol) 5, 25 mg Anthracen und 5 µl Triethylamin in 80 ml Toluol in einem Pyrex-Tauchreaktor und bestrahlt 15 Min. mittels einer Quechsilberhochdrucklampe (Philips HPK 125) unter Stickstoffatmosphäre. Danach engt man ein, löst den Rückstand in 20 ml Tetrahydrofuran, versetzt mit 2,1 ml Tetrabutylammoniumfluorid-Lösung (1 M in Tetrahydrofuran) und rührt 1 Std. unter Argon bei 60°C. Anschließend wird das Reaktionsgemisch in gesättigte Natriumhydrogencarbonat-Lösung eingerührt, mit Essigester extrahiert, über Natriumsulfat getrocknet und das Solvens entfernt. Man reinigt das Produkt durch mehrmalige Chromatographie an Kieselgel mit Hexan/Essigester und erhält 25 mg der Titelverbindung als farblosen Schaum.
$^1$H-NMR ($CD_2Cl_2$): δ= 0,40 ppm (s, 3H, H-18); 1,12 (s, 6H, H-26 u. H-27); 3,12 (d, J=9,5 Hz, 1H, H-24); 3,18 (d, J=9,5 Hz, 1H, H-24'); 3,83 (d, J=14 Hz, 1H, H-22); 3,92 (d, J=14 Hz, 1H, H-22'); 4,08 (m, 1H, H-3); 4,31 (m, 1H, H-1); 4,89 (s, 2H, H-19 u. H-21); 5,10 (s, 1H, H-21'); 5,25 (s, 1H, H-19'); 5,98 u. 6,29 (2x d, J=11 Hz, je 1H, H-6 u. H-7)

**8. (5*Z*,7*E*)-(1*S*,3*R*)-26,27-Dimethyl-23-oxa-9,10-secocholesta-5,7,10(19),20-tetraen-1,3,25-triol 9**

Man setzt 270 mg (0,29 mmol) 6 analog **7.** um und erhält nach der entsprechenden Reinigung 41 mg der Titelverbindung als farblosen Schaum.
$^1$H-NMR ($CD_2Cl_2$): δ= 0,49 ppm (s, 3H, H-18); 0,88 u. 0,90 (2x t, J=7 Hz, je 3H, H-28 u. H-29); 1,51 (q, J=7 Hz, 4H, H-26 u. H-27); 3,22 u. 3,30 (2x d, J= 9,5 Hz, je 1H, H-24 u. H-24'); 3,90 u. 3,98 (2x d, J=14 Hz, je 1H, H-22 u. H-22'); 4,18 (m, 1H, H-3); 4,39 (m, 1H, H-1); 4,98 (s, 2H, H-19 u. H-21); 5,18 (s, 1H, H-21'); 5,30 (s, 1H, H-19'); 6,05 u. 6,38 (2x d, J=11 Hz, je 1H, H-6 u. H-7)

**9. (5*Z*,7*E*)-(1*S*,3*R*)-26,27-Diethyl-23-oxa-9,10-secocholesta-5,7,10(19),20-tetraen-1,3,25-triol 10**

265 mg (0,24 mmol) 7 werden analog **7.** umgesetzt. Nach der entsprechenden Reinigung fallen 38 mg der Titelverbindung als farbloser Schaum an.
$^1$H-NMR ($CD_2Cl_2$): δ= 0,48 ppm (s, 3H, H-18); 0,90 (t, J=7 Hz, 6H, H-30 u. H-31); 3,22 u. 3,28 (2x d, J=9,5 Hz, H-24 u. H-24'); 3,89 u. 3,98 (2x d, J=14 Hz, H-22 u. H-22'); 4,18 (m, 1H, H-3); 4,39 (m 1H, H-1); 4,98 (s, 2H, H-19 u. H-21); 5,17 (s, 1H, H-21'); 5,30 (s, 1H, H-19'); 6,04 u. 6,38 (2x d, J=11 Hz, je 1H, H-6 u. H-7)

**10. (5*Z*,7*E*)-(1*S*,3*R*)-1,3-Bis[[(1,1-dimethylethyl)diphenylsilyl]oxy]-20-methylen-9,10-seco-pregna-5,7,10(19)-trien-21-ol 11**

Man löst 500 mg (0,61 mmol) 3 in 80 ml Toluol, versetzt mit 80 mg (0,44 mmol) Anthracen und 15 µl Triethylamin und bestrahlt 18 Min. in der unter **7.** beschriebenen Apparatur. Nach Aufarbeitung und Reinigung erhält man 450 mg der Titelverbindung als farblosen Schaum.
$^1$H-NMR ($CDCl_3$): δ= 0,43 ppm (s, 3H, H-18); 0,95 u. 1,00 (2x s, je 9H, Si-t-Butyl); 4,05 u. 4,15 (2x d, J=14,5 Hz, je 1H, H-22 u. H-22'); 4,25 (m, 1H, H-3); 4,55 (m, 1H, H-1); 4,83 (s, 1H, H-19); 5,00 (s, 1H, H-21); 5,08 (s, 1H, H-19'); 5,21 (s, 1H, H-21'); 6,02 u. 6,10 (2x d, J=11 Hz, je 1H, H-6 u. H-7); 7,15-7,68 (m, 20H, Si-Phenyl)

**11. 3-[(5*Z*,7*E*)-(1*S*,3*R*)-1,3-Bis[[(1,1-dimethylethyl)diphenylsilyl]oxy]-23-oxa-9,10-secochola-5,7,10(19),20-tetraen-24-yl]propansäuremethylester 12**

Man löst 500 mg (0,61 mmol) 11 in 1 ml Toluol und versetzt mit 2,8 ml wässriger Natronlauge (25%), 12 mg Tetrabutylammomiumhydrogensulfat und 681 mg (1,83 mmol) 4-Brombuttersäureorthotrimethylester und rührt über Nacht bei Raumtemperatur. Anschließend gießt man auf Natriumchlorid-Lösung, extrahiert mit Essigester, wäscht die organische Phase mit Natriumchlorid-Lösung, trocknet über Natriumsulfat und entfernt das Solvens. Nach chromatographischer Reinigung erhält man 180 mg der Titelverbindung als farbloser Schaum neben 130 mg unumgesetztem Edukt.
$^1$H-NMR ($CDCl_3$): δ= 0.42 ppm (s, 3H, H-18); 0,92 u. 1,00 (2x s, je 9H, Si-t-Butyl); 2,47 (t, J=7 Hz, 2H, H-26); 3,70 (s, 3H, COOMe); 3,46 (m, 2H, H-24); 3,91 (s, 2H, H-22); 4,24 (m, 1H, H-3); 4,55 (m, 1H, H-1); 4,83 (s, 1H, H-19); 4,98 (s, 1H, H-21); 5,08 (s, 1H, H-19'); 5,17 (s, 1H, H-21'); 6,03 u. 6,10 (2x d, J=11 Hz, je 1H, H-6 u. H-7); 7,22-7,70 (m, 20H, Si-Phenyl)

**12. (5*Z*,7*E*)-(1*S*,3*R*)-1,3-Bis[[(1,1-dimethylethyl)diphenylsilyl]oxy]-24-(3-hydroxy-3-methylbutyl-23-oxa-9,10-secochola-5,7,10(19),20-tetraen 13**

Man bereitet das Grignard-Reagenz aus 185 mg (1,3 mmol) Jodmethan und 31 mg (1,3 mmol) Magnesiumspänen in 5 ml Diethylether und setzt analog **4.** mit 120 mg (0,13 mmol) **12** um, wobei man 60 mg der Titelverbindung als farblosen Schaum erhält.
$^1$H-NMR ($CDCl_3$): δ= 0,42 ppm (s, 3H, H-18); 0,91 u. 1,00 (2x s, je 9H, Si-t-Butyl); 1,23 (s, 6H, H-28 u. H-29); 3,48 (m, 2H, H-24); 3,92 (s, 2H, H-22); 4,24 (m, 1H, H-3); 4,54 (m, 1H, H-3); 4,83 (s, 1H, H-19); 5,00 (s, 1H, H-21); 5,09 (s, 1H, H-19'); 5,19 (s, 1H, H-21'); 6,01 u. 6,09 (2x d, J=11 Hz, H-6 u. H-7); 7,22-7,68 (m, 20H, Si-Phenyl)

**13. (5*Z*,7*E*)-(1*S*,3*R*)-1,3-Bis[[(1,1-dimethylethyl)diphenylsilyl]oxy]-24-(3-ethyl-3-hydroxypentyl)-23-oxa-9,10-secochola-5,7,10(19),20-tetraen 14**

Aus 142 mg (1,3 mmol) Brommethan und 31 mg (1,3 mmol) Magnesiumspänen bereitet man in 5 ml Tetrahydrofuran das Grignard-Reagenz und setzt analog **4.** mit 120 mg (0,13 mmol) **12** um. Man erhält 70 mg der Titelverbindung als farblosen Schaum.
$^1$H-NMR ($CDCl_3$): δ= 0,42 ppm (s, 3H, H-18); 0,88 (t, J=7 Hz, 6H, H-30 u. H-31); 0,92 u. 1,00 (2x s, je 9H, Si-t-Butyl); 1,49 (q, J=7 Hz, H-28 u. H-29); 3,46 (m, 2H, H-24); 3,91 (s, 2H, H-22); 4,24 (m, 1H, H-3); 4,54 (m, 1H, H-1); 4,82 (s, 1H, H-19); 4,98 (s, 1H, H-21); 5,08 (s, 1H, H-19'); 5,18 (s, 1H, H-21'); 6,01 u. 6,09 (2x d, J=11 Hz, H-6 u. H-7); 7,23-7,69 (m, 20H, Si-Phenyl)

**14. (5*Z*,7*E*)-(1*S*,3*R*)-24-(3-Hydroxy-3-methylbutyl)-23-oxa-9,10-secochola-5,7,10(19),20-tetraen-1,3-diol 15**

Man löst 57 mg (0,062 mmol) **13** in 5 ml Tetrahydrofuran, versetzt mit 0,67 ml Tetrabutylammoniumfluorid-Lösung (1M in Tetrahydrofuran) und rührt 1 Std. bei 60°C. Nach Zugabe von Natriumchlorid-Lösung wird mit Essigester extrahiert, die organische Phase mit Natriumchlorid-Lösung gewaschen, über Natriumsulfat getrocknet und das Lösungsmittel entfernt. Mehrmalige Chromatographie an Kieselgel mit Hexan/Essigester liefert 9 mg der Titelverbindung als farblosen Schaum.
$^1$H-NMR ($CD_2Cl_2$): δ= 0,47 ppm (s, 3H, H-18); 1,19 (s, 6H, H-28 u. H-29); 3,42 (m, 2H, H-24); 3,89 (s, 2H, H-22); 4,18 (m, 1H, H-3); 4,39 (m, 1H, H-1); 4,97 (s, 2H, H-19 u. H-21); 5,17 (s, 1H, H-19'); 5,30 (s, 1H, H-21'); 6,05 u. 6,29 (2x d, J= 11 Hz, je 1H, H-6 u. H-7)

**15. (5*Z*,7*E*)-(1*S*,3*R*)-24-(3-Ethyl-3-hydroxypentyl)-23-oxa-9,10-secochola-5,7,10(19),20-tetraen-1,3-diol 16**

Man setzt 67 mg (0,07 mmol) **14** analog **14.** mit 0,76 ml Tetrabutylammoniumfluorid-Lösung in 5 ml Tetrahydrofuran um und erhält nach Aufreinigung 11 mg der Titelverbindung als farblosen Schaum.
$^1$H-NMR ($CD_2Cl_2$): δ= 0,47 ppm (s, 3H, H-18); 0,84 (t, J=7 Hz, 6H, H-30 u. H-31); 1,47 (q, J=7 Hz, H-28 u. H.29); 3,40 (m, 2H, H-24); 3,89 (m, 2H, H-22); 4,18 (m, 1H, H-3); 4,39 (m, 1H, H-1); 4,97 (s, 2H, H-19 u. H-21); 5,16 (s, 1H, H-19'); 5,30 (s, 1H, H-21'); 6,04 u. 6,38 (2x d, J=11 Hz, H-6 u. H-7)

**16. (5*Z*,7*E*)-(1*S*,3*R*)-1,3-Bis[[(1,1-dimethylethyl)diphenylsilyl]oxy]-20,21-methylen-9,10-secopregna-5,7,10(19)-trien-20-methanol 17**

Aus Zinkpulver und Silberacetat wird analog J.M. Conia et al. (Synthesis 549 (1972)) das Zink/Silber-Reagenz bereitet. Man legt nun 98 mg (1,5 mmol) des Reagenzes in 5 ml Diethylether unter Argon vor und tropft langsam 268 mg (1 mmol) Dijodmethan zu, wobei ein leichtes Sieden der Reaktionslösung einsetzt. Man rührt 30 Min. bei Raumtemperatur und gibt dann 200 mg (0,24 mmol) **11** in 5 ml Diethylether zu. Es wird 1 Std. bei Raumtemperatur gerührt und anschließend werden 0,2 ml Pyridin zugesetzt. Der entstehende Niederschlag wird abfiltriert und das Filtrat mit Essigester verdünnt, die organische Phase mit Natriumhydrogencarbonat- und Natriumchlorid-Lösung gewaschen, über Natriumsulfat getrocknet und das Solvens entfernt. Den Rückstand reinigt man durch Chromatographie an Kieselgel mit Hexan/Essigester, wobei 65 mg der Titelverbindung als farbloser Schaum anfallen.
$^1$H-NMR ($CDCl_3$): δ= 0,20 ppm (m, 1H, Cyclopropyl); 0,34 (m, 2H, Cyclopropyl); 0,55 (s, 3H, H-18); 0,66 (m, 1H, Cyclopropyl); 0,92 u. 0,99 (2x s, je 9H, Si-t-Butyl); 3,04 u. 3,92 (2x dbr, J=10,5 Hz, je 1H, H-22 u. H-22'); 4,23 (m, 1H, H-3); 4,54 (m, 1H, H-1); 4,82 u. 5,09 (2x s, je 1H, H-19 u. H-19'); 5,98 u. 6,10 (2x d, J=11 Hz, je 1H, H-6 u. H-7); 7,22-7,68 (m, 20H, Si-Phenyl)

**17. (5*Z*,7*E*)-(1*S*,3*R*)-1,3-Bis[[(1,1-dimethylethyl)diphenylsilyl]oxy]-20,21-methylen-23-oxa-9,10-secochola-5,7,10(19)-trien-24-carbonsäure-1,1-dimethylester 18**

Man setzt 130 mg 17 in 1 ml Toluol mit 0,16 g (0,81 mmol) Bromessigsäure-tert.-butylester, 0,7 ml wässriger Natriumhydroxid-Lösung und 3 mg Tetrabutylammoniumhydrogensulfat analog **3.** um. Nach Aufreinigung erhält man 80 mg der Titelverbindung als farblosen Schaum.

$^1$H-NMR ($CD_2Cl_2$): δ= 0,28-0,42 ppm (m, 3H, Cyclopropyl); 0,53 (s, 3H, H-18); 0,62 (m, 1H, Cyclopropyl); 0,90 u. 0,98 (2x s, je 9H, Si-t-Butyl); 1,50 (s, 9H, t-Butylester); 2,99 (dbr, J=10,5 Hz, 1H, H-22); 3,72 (d, J=10 Hz, 1H, H-24); 3,90 (dbr, J=10,5 Hz, 1H, H-22'); 3,90 (d, J=10 Hz, 1H, H-24'); 4,25 (m, 1H, H-3); 4,55 (m, 1H, H-1); 4,82 u. 5,08 (2x s, je 1H, H-19 u. H-19'); 5,99 u. 6,12 (2x d, J=11 Hz, je 1H, H-6 u. H-7); 7,28-7,68 (m, 20H, Si-Phenyl)

**18.**
**(5*Z*,7*E*)-(1*S*,3*R*)-20,21-Methylen-23-oxa-9,10-secocholesta-5,7,10(19)-trien-1,3,25-triol 19**

Aus 170 mg (1,2 mmol) Jodmethan und 30 mg (1,2 mmol) Magnesiumspänen wird in 5 ml Diethylether das Grignard-Reagenz bereitet und analog **4.** mit 120 mg (0,13 mmol) 18 umgesetzt. Das hier anfallende Rohprodukt wird direkt mit 1,1 ml Tetrabutylammoniumfluorid-Lösung in Tetrahydrofuran analog **14.** umgesetzt, wobei man nach chromatographischer Reinigung 14 mg der Titelverbindung als farblosen Schaum erhält.

$^1$H-NMR ($CD_2Cl_2$): δ= 0,14-0,30 ppm (m, 3H, Cyclopropyl); 0,54 (s, 3H, H-18); 1,02 (m, 1H, Cyclopropyl); 1,18 (s, 6H, H-26 u. H-27); 2,82 (d, J=10 Hz, 1H, H-22); 3,05 (d, J=9,5 Hz, 1H, H-24); 3,17 (d, J=9,5 Hz, 1H, H-24'); 3,68 (d, J=10 Hz, 1H, H-22'); 4,09 (m, 1H, H-3); 4,30 (m, 1H, H-1); 4,88 u. 5,21 (2x s, je 1H, H-19 u. H-19'); 5,91 u. 6,28 (2x d, J=11 Hz, je 1H, H-6 u. H-7)

**19. (5*Z*,7*E*)-(1*S*,3*R*)-26,27-Dimethyl-20,21-methylen-23-oxa-9,10-secocholesta-5,7,10(19)-trien-1,3,25-triol 20**

Aus 19,4 mg (0,8 mmol) Magnesiumspänen und 88 mg (0,8 mmol) Bromethan bereitet man das Grignard-Reagenz, das man mit 80 mg (0,1 mmol) 18 analog **4.** umsetzt. Das Rohprodukt wird anschließend in 5 ml Tetrahydrofuran gelöst und mit 0,85 ml Tetrabutylammoniumfluorid-Lösung in Tetrahydrofuran analog **14.** umgesetzt. Nach mehrmaliger Chromatographie an Kieselgel mit Hexan/Essigester erhält man 22 mg der Titelverbindung als farblosen Schaum.

$^1$H-NMR ($CD_2Cl_2$): δ= 0,21-0,40 ppm (m, 3H, Cyclopropyl); 0,52 (s, 3H, H-18); 0,85 (t, J=7 Hz, 6H, H-28 u. H-29); 0,89 (m, 1H, Cyclopropyl); 1,49 (q, J=7 Hz, 4H, H-26 u. H-27); 2,88 (dbr, J=10,5 Hz, 1H, H-22); 3,17 (d, J=10 Hz, 1H, H-24); 3,26 (d, J=10 Hz, 1H, H-24'); 3,72 (dbr, J=10,5 Hz, 1H, H-22'); 4,17 (m, 1H, H-3); 4,39 (m, 1H, H-1); 4,97 u. 5,29 (2x s, je 1H, H-19 u. H-19'); 6,00 u. 6,38 (2x d, J=11 Hz, je 1H, H-6 u. H-7)

**20. (5*Z*,7*E*)-(1*S*,3*R*)-26,27-Diethyl-20,21-methylen-23-oxa-9,10-secocholesta-5,7,10(19)-trien-1,3,25-triol 21**

Man bereitet aus 163 mg (1,3 mmol) 1-Brompropan und 32 mg (1,3 mmol) Magnesiumspänen in 5 ml Tetrahydrofuran das Grignard-Reagenz und setzt analog **4.** mit 125 mg (0,13 mmol) 18 um. Das Rohprodukt behandelt man analog **14.** mit 0,72 ml Tetrabutylammoniumfluorid-Lösunng in 5 ml Tetrahydrofuran und erhält nach mehrmaliger Chromatographie 26 mg der Titelverbindung als farblosen Schaum.

$^1$H-NMR ($CD_2Cl_2$): δ= 0,20-0,37 ppm (m, 3H, Cyclopropyl); 0,90 (t, J=7 Hz, 6H, H-30 u. H-31); 1,08 (m, 1H, Cyclopropyl); 2,87 (d, J=10 Hz, 1H, H-22); 3,13 (d, J=9,5 Hz, 1H, H-24); 3,23 (d, J=9,5 Hz, 1H, H-24'); 3,70 (d, J=10 Hz, 1H, H-22'); 4,17 (m, 1H, H-3); 4,37 (m, 1H, H-1); 4,93 u. 5,28 (2x s, je 1H, H-19 u. H-19'); 5,98 u. 6,35 (2x d, J=11 Hz, je 1H, H-6 u. H-7)

**21. 3-[(5*Z*,7*E*)-(1*S*,3*R*)-1,3-Bis[[(1,1-dimethylethyl)diphenylsilyl]oxy]-20,21-methylen-23-oxa-9,10-secochola-5,7,10(19)-trien-24-yl]propansäuremethylester 22**

400 mg (0,48 mmol) 17, 2,2 ml wässrige Natronlauge (25%), 10 mg Tetrabutylammoniumhydrogensulfat und 536 mg (1,44 mmol) 4-Brombuttersäureorthotrimethylester werden in 1 ml Toluol analog **11.** umgesetzt. Aufreinigung liefert 100 mg der Titelverbindung als farblosen Schaum neben 310 mg Ausgangsmaterial.

$^1$H-NMR ($CDCl_3$): δ= 0,18-0,35 ppm (m, 3H, Cyclopropyl); 0,47 (s, 3H, H-18); 0,53 (m, 1H, Cyclopropyl); 0,88 u. 0,97 (2x s, je 9H, Si-t-Butyl); 2,37 (t, J=7 Hz, 2H, H-26); 2,74 (d, J=10,5 Hz, 1H, H-22); 3,35 (m, 2H, H-24); 3,60 (s, 3H, COOMe); 3,62 (d, J=10,5 Hz, 1H, H-22'); 4,17 (m, 1H, H-3); 4,48 (m, 1H, H-1); 4,76 u. 5,02 (2x s, je 1H, H-19 u. H-19'); 5,90 u. 6.02 (2x d, J=11 Hz, je 1H, H-6 u. H-7); 7,20-7,60 (m, 20H, Si-Phenyl)

**22. (5*Z*,7*E*)-(1*S*,3*R*)-1,3-Bis[[(1,1-dimethylethyl)diphenylsilyl]oxy]-24-(3-ethyl-3-hydroxypentyl)-20,21-methylen-23-oxa-9,10-secocholesta-5,7,10(19)-trien <u>23</u>**

Man bereitet das Grignard-Reagenz aus 305 mg (2,8 mmol) Bromethan und 68 mg (2,8 mmol) Magnesiumspänen in 5 ml Tetrahydrofuran und setzt analog **4.** mit 145 mg (0,15 mmol) <u>**22**</u> um. Es werden 103 mg der Titelverbindung als farbloser Schaum erhalten.
$^1$H-NMR ($CDCl_3$): δ= 0,28-0,45 ppm (m, 3H, Cyclopropyl); 0,52 (s, 3H, H-18); 0,61 (m, 1H, Cyclopropyl); 0,88 (t, J=7 Hz, 6H, H-30 u. H-31); 0,90 u. 0,99 (2x s, je 9H, Si-t-Butyl); 2,86 (d, J=10 Hz, 1H, H-22); 3,39 (m, 2H, H-24); 3,70 (d, J=10 Hz, 1H, H-22'); 4,23 (m, 1H, H-3); 4,54 (t, J=6 Hz, 1H, H-1); 4,82 u. 5,09 (2x s, je 1H, H-19 u. H-19'); 5,98 u, 6,09 (2x d, J=11 Hz, je 1H, H-6 u. H-7); 7,22-7,68 (m, 20H, Si-Phenyl)

**23. (5*Z*,7*E*)-(1*S*,3*R*)-24-(3-Ethyl-3-hydroxypentyl)-20,21-methylen-23-oxa-9,10-secochola-5,7,10(19)-trien-1,3-diol <u>24</u>**

Man setzt 100 mg (0,1 mmol) <u>**23**</u> in 5 ml Tetrahydrofuran mit 1 ml Tetrabutylammoniumfluorid analog **11.** um und erhält nach Aufreinigung 21 mg der Titelverbindung als farblosen Schaum.
$^1$H-NMR ($CD_2Cl_2$): δ= 0,10-0,29 ppm (m, 3H, Cyclopropyl); 0,51 (s, 3H, H-18); 0,78 (t, J=7 Hz, H-30 u. H-31); 1,01 (m, 1H, Cyclopropyl); 1,38 (q, J=7 Hz, 4H, H-28 u. H-29); 2,78 (d, J=10 Hz, 1H, H-22); 3,28 (m, 2H, H-24); 3,58 (d, J=10 Hz, 1H, H-22'); 4,09 (m, 1H, H-3); 4,30 (m, 1H, H-1); 4,88 u. 5,22 (2x s, je 1H, H-19); 5,91 u. 6,29 (2x d, J=11 Hz, je 1H, H-6 u. H-7)

**24. [1*S*-(1α,3aβ,4α,7aα)]-4-(Acetyloxy)octahydro-α,α,7a-trimethyl-1H-inden-1-acetaldehyd <u>25</u>**

Man bereitet eine Suspension von 900 mg (30 mmol) Natriumhydrid (80%) in 120 ml Tetrahydrofuran und tropft bei 0°C unter Argon eine Lösung von 6,3 g (25 mmol) [1*R*-[1α(*S**),3aβ,4α,7aα]]-4-(Acetyloxy)-α,7a-dimethyloctahydro-1H-inden-1-acetaldehyd (H.H. Inhoffen et al. Chem. Ber. **91**, 780 (1958), Chem. Ber. **92**, 1772 (1959)) in 60 ml Tetrahydrofuran zu. Nach 30 Min. werden 19,65 g (75 mmol) Methyliodid zugetropft und anschließend wird 6 Std. bei 50°C gerührt. Nach dem Abkühlen gießt man das Reaktionsgemisch auf Natriumchlorid-Lösung, extrahiert mit Essigester, wäscht die organische Phase mit Natriumchlorid-Lösung, trocknet über Natriumsulfat und entfernt das Solvens. Der Rückstand wird an Kieselgel mit Hexan/Essigester chromatographiert, wobei 3,2 g der Titelverbindung als farbloses Öl anfallen.
$^1$H-NMR ($CDCl_3$): δ= 0,82 ppm (s, 3H, H-18); 1,05 u. 1,08 (2x s, je 3H, H-21 u. C-20-Methyl); 1,99 (s, 3H, OAc); 5,09 (m, 1H, H-8); 9,60 (s, 1H, H-22)
IR (Film): ν= 1725, 1710 $cm^{-1}$

**25. [1*S*-(1α,3aβ,4α,7aα)]-4-(Acetyloxy)octahydro-β,β,7a-trimethyl-1H-inden-1-ethanol <u>26</u>**

Man löst 350 mg (1,3 mmol) <u>**25**</u> in 5 ml Tetrahydrofuran und 5 ml Methanol und gibt 193 mg (1,4 mmol) Certrichlorid-Heptahydrat zu. Bei 0°C werden nun portionsweise 46 mg (1,2 mmol) Natriumborhydrid zugegeben und es wird 1 Std. nachgerührt. Anschließend hydrolysiert man mit Natriumchlorid-Lösung, extrahiert mit Essigester, wäscht die organische Phase mit Natriumchlorid-Lösung, trocknet über Natriumsulfat und engt ein. Der Rückstand wird an Kieselgel mit Hexan/Essigester gereinigt, wobei 285 mg der Titelverbindung als farbloses Öl verbleiben.
$^1$H-NMR ($CDCl_3$): δ= 0,90 ppm (s, 3H, H-18); 1,00 u. 1,01 (2x s, je 3H, H-21 u. C-20-Methyl); 2,05 (s, 3H, OAc); 3,29 u. 3,37 (2x d, J=10,5 Hz, je 1H, H-22 u. H-22'); 5,16 (m, 1H, H-8)
IR (Film): ν= 1725 $cm^{-1}$

**26. [1*S*-(1α,3aβ,4α,7aα)]-1,1-Dimethylethyl-[2-[4-(acetyloxy)-7a-methyloctahydro-1H-inden-1-yl]-2-methylpropoxy]acetat <u>27</u>**

Man löst 3,04 g (11,3 mmol) <u>**26**</u> in 40 ml Toluol, gibt 11,9 g (61,3 mmol) Bromessigsäure-tert.-butylester, 33,9 ml wässrige Natriumhydroxid-Lösung (25%) und 172 mg Tetrabutylammoniumhydrogensulfat unter Argon zu. Es wird nun 48 Std. bei Raumtemperatur gerührt und anschließend auf Natriumchlorid-Lösung gegossen. Nach Extraktion mit Essigester, Waschen der organischen Phase mit Natriumchlorid-Lösung, Trocknen über Natriumsulfat und Entfernen des Lösungsmittels chromatographiert man den Rückstand an Kieselgel mit Hexan/Essigester, wobei man 1,1 g der Titelverbindung als farbloses Öl neben 1,93 g des Ausgangsmaterials erhält.
$^1$H-NMR ($CDCl_3$): δ= 0,88 ppm (s, 3H, H-18); 0,93 u. 0,99 (2x s, je 3H, H-21 u. C-20-Methyl); 1,41 (s, 9H, t-Butylester); 1,99 (s, 3H, OAc); 3,03 u. 3,20 (2x d, J=9 Hz, je 1H, H-22 u. H-22'); 3,82 u. 3,90 (2x d, J=16 Hz, je 1H, H-24 u. H-24'); 5,09 (m, 1H, H-8)

**27. [1*S*-(1α,3aβ,4α,7aα)]-1-[1,1-Dimethyl-2-(2-ethyl-2-hydroxybutoxy)ethyl]-7a-methyloctahydro-1H-inden-4-ol 28**

Aus 10,8 g (100 mmol) Bromethan und 928 mg (88 mmol) Magnesiumspänen bereitet man in 20 ml Tetrahydrofuran das Grignard-Reagenz und gibt bei 0°C 1,1 g (2,8 mmol) 27 in 39 ml Tetrahydrofuran hinzu. Man rührt 1 Std. bei Raumtemperatur nach und gießt das Reaktionsgemisch anschließend auf gesättigte Ammoniumchlorid-Lösung. Nach Extraktion mit Essigester, Waschen der organischen Phase mit Natriumchlorid-Lösung, Trocknen über Natriumsulfat und Abdampfen des Lösungsmittels reinigt man das Rohprodukt durch Chromatographie an Kieselgel mit Hexan/Essigester, wobei 995 mg der Titelverbindung als farbloses Öl anfallen.
$^1$H-NMR ($CDCl_3$): δ= 0,88 ppm (s, 3H, H-18); 0,90 (t, J=7 Hz, 6H, H-28 u. H-29); 1,00 u. 1,07 (2x s, je 3H, H-21 u. C-20-Methyl); 1,51 (q, J=7 Hz, 4H, H-26 u. H-27); 3,11 u. 3,16 (2x d, J=9,5 Hz, je 1H, H-22 u. H-22'); 3,21 u. 3,27 (2x d, J=9,5 Hz, je 1H, H-24 u. H-24'); 4,09 (m, 1H, H-8)

**28. [1*S*-(1α,3aβ,4α,7aα)]-1-[1,1-Dimethyl-2-(2-hydroxy-2-methylpropoxy)ethyl]-7a-methyloctahydro-1H-inden-4-ol 29**

Man bereitet das Grignard-Reagenz aus 2,04 g (14,4 mmol) Jodmethan und 350 mg (14,4 mmol) Magnesiumspänen in 20 ml Diethylether und setzt analog **27.** mit 690 mg (1,8 mmol) 27 um. Man erhält 410 mg der Titelverbindung als farbloses Öl.
$^1$H-NMR ($CDCl_3$): δ= 0,87 ppm (s, 1H, H-18); 0,94 u. 1,00 (2x s, je 3H, H-21 u. C-20-Methyl); 1,15 (s, 3H, H-26 u. H-27); 2,31 (sbr, 1H, OH); 3,09 (s, 2H, H-22); 3,11 (d, J=9,5 Hz, 1H, H-24); 3,18 (d, J=9,5 Hz, 1H, H-24'); 4,02 (m, 1H, H-8)

**29. [1*S*-(1α,3aβ,4α,7aα)]-1-[1,1-Dimethyl-2-(2-hydroxy-2-propylpentoxy)ethyl]-7a-methyloctahydro-1H-inden-4-ol 30**

Man bereitet das Grignard-Reagenz aus 1,97 g (14,4 mmol) 1-Brompropan und 350 mg (14,4 mmol) Magnesiumspänen in 20 ml Tetrahydrofuran und setzt analog **27.** mit 690 mg (1,8 mmol) 27 um. Man erhält 630 mg der Titelverbindung als farbloses Öl.
$^1$H-NMR ($CDCl_3$): δ= 0,82 ppm (s, 3H, H-18); 0,84 (t, J=7 Hz, 6H, H-30 u. H-31); 0,93 u. 0,98 (2x s, je 3H, H-21 u. C-20-Methyl); 2,29 (t, J=6 Hz, 1H, OH); 3,07 (s, 2H, H-22); 3,12 (d, J=9,5 Hz, 1H, H-24); 3,18 (d, J=9,5 Hz, 1H, H-24'); 4,01 (m, 1H, H-8)

**30. [1*S*-(1α,3aβ,7aα)]-1-[1,1-Dimethyl-2-(2-ethyl-2-hydroxybutoxy)ethyl]-7a-methyloctahydro-4H-inden-4-on 31**

Man tropft 890 mg (2,8 mmol) 28 in 10 ml Methylenchlorid unter Argon zu einer Suspension von 1,41 g (6,6 mmol) Pyridiniumchlorochromat in 50 ml Methylenchlorid. Nach 2 Std. bei Raumtemperatur wird mit Diethylether verdünnt, mehrmals über Celite filtriert und das Solvens entfernt. Der Rückstand wird an Kieselgel mit Hexan/Essigester chromatographiert, wobei man 696 mg der Titelverbindung als farbloses Öl erhält.
$^1$H-NMR ($CDCl_3$): δ= 0,72 ppm (s, 3H, H-18); 0,90 (t, J=7 Hz, 6H, H-28 u. H-29); 0,92 u. 1,02 (2x s, je 3H, H-21 u. C-20-Methyl); 1,52 (q, J=7 Hz, 4H, H-26 u. H-27); 3,18 (s, 2H, H-22); 3,22 u. 3,28 (2x d, J= 9,5 Hz, je 1H, H-24 u. H-24')

**31. [1*S*-(1α,3aβ,7aα)]-1-[1,1-Dimethyl-2-(2-hydroxy-2-methylpropoxy)ethyl]-7a-methyloctahydro-4H-inden-4-on 32**

Man setzt 410 mg (1,37 mmol) 29 in 20 ml Methylenchlorid mit 379 mg (1,76 mmol) Pyridiniumchlorochromat analog **30.** um und erhält 273 mg der Titelverbindung als farbloses Öl.
$^1$H-NMR ($CDCl_3$): δ= 0,76 ppm (s, 3H, H-18); 0,88 u. 0,97 (2x s, je 1H, H-21 u. C-20-Methyl); 1,15 (s, 6H, H-26 u. H-27); 2,36 (dd, J=10,5, 7,5 Hz, 1H, H-14); 3,12 (s, 2H, H-22); 3,12 (d, J= 9,5 Hz, 1H, H-24); 3,19 (d, J=9,5 Hz, 1H, H-24')

**32. [1*S*-(1α,3aβ,7aα)]-1-[1,1-Dimethyl-2-(2-hydroxy-2-propylpentoxy)ethyl]-7a-methyloctahydro-4H-inden-4-on 33**

Man setzt 640 mg (1,83 mmol) 30 in 20 ml Methylenchlorid mit 503 mg (2,34 mmol) Pyridiniumchlorochromat analog **30.** um und erhält 386 mg der Titelverbindung als farbloses Öl.
$^1$H-NMR ($CDCl_3$): δ= 0,75 ppm (s, 3H, H-18); 0,86 (t, J= 7 Hz, 6H, H-30 u. H-31); 0,86 u. 0,95 (2x s, je 3H, H-21 u. C-20-Methyl); 2,35 (dd, J=10,5, 7,5 Hz, 1H, H-14); 3,08 (s, 2H, H-22); 3,13 (d, J=9,5, 2H, H-22); 3,18 (d, J=9,5 Hz, 1H, H-22')

**33. [1*S*-(1α,3aβ,7aα)]-1-[1,1-Dimethyl-2-[2-ethyl-2-[(trimethylsilyl)oxy]butoxy]ethyl]-7a-methyloctahydro-4H-inden-4-on 34**

Man löst 696 mg (2,1 mmol) 31, 571 mg (8,4 mmol) Imidazol und 456 mg (4,2 mmol) Trimethylchlorsilan in 10 ml Dimethylformamid und rührt über Nacht bei Raumtemperatur unter Argon. Anschließend wird mit Natriumchlorid-Lösung versetzt, mit Essigester extrahiert, die organische Phase mit Natriumchlorid-Lösung gewaschen, über Natriumsulfat getrocknet und das Solvens entfernt. Der Rückstand wird an Kieselgel mit Hexan/Essigester chromatographiert, wobei 766 mg der Titelverbindung als farbloses Öl verbleiben.
$^1$H-NMR ($CD_2Cl_2$): δ= 0,10 ppm (s, 9H, $SiMe_3$); 0,70 (s, 3H, H-18); 0,82 (t, J=7 Hz, 6H, H-28 u. H-29); 0,91 u. 1,03 (2x s, je 3H, H-21 u. C-20-Methyl); 1,53 (q, J=7 Hz, 4H, H-26 u. H-27); 3,05 u. 3,13 (2x d, J= 9 Hz, je 1H, H-22 u. H-22'); 3,20 u. 3,24 (2x d, J=9,5 Hz, je 1H, H-24 u. H-24')

**34. [1*S*-(1α,3aβ,7aα)]-1-[1,1-Dimethyl-2-[2-methyl-2-[(trimethylsilyl)oxy]propoxy]ethyl]-7a-methyloctahydro-4H-inden-4-on 35**

Man setzt 270 mg (0,92 mmol) 32 mit 300 mg (2,76 mmol) Trimethylchlorsilan, 248 mg (3,59 mmol) Imidazol und 0,37 ml Pyridin in 30 ml Diethylether analog **33.** um, wobei man 272 mg der Titelverbindung als farbloses Öl erhält.
$^1$H-NMR ($CDCl_3$): δ=0,11 ppm (s, 9H, $SiMe_3$); 0,72 (s, 3H, H-18); 0,91 u. 1,02 (2x s, je 3H, H-21 u. C-20-Methyl); 1,23 (s, 6H, H-26 u. H-27); 2,41 (dd, J=10,5, 7,5 Hz, 1H, H-14); 3,11 (d, J=9,5 Hz, 1H, H-22); 3,14 (2x d, J=9,5 Hz, je 1H, H-22' u. H-24); 3,19 (d, J=9,5 Hz, 1H, H-24')

**35. [1*S*-(1α,3aβ,7aα)]-1-[1,1-Dimethyl-2-[2-propyl-2-[(trimethylsilyl)oxy]pentoxy]ethyl]-7a-methyloctahydro-4H-inden-4-on 36**

Man setzt 383 mg (1,10 mmol) 33 mit 358 mg (3,30 mmol) Trimethylchlorsilan, 296 mg (4,29 mmol) Imidazol und 0,44 ml Pyridin in 30 ml Diethylether analog **33.** um, wobei man 386 mg der Titelverbindung als farbloses Öl erhält.
$^1$H-NMR ($CDCl_3$): δ=0,11 ppm (s, 9H, $SiMe_3$); 0,73 (s, 3H, H-18); 0,90 (t, J=7 Hz, 6H, H-30 u. H-31); 0,91 u. 1,03 (2x s, je 3H, H-21 u. C-20-Methyl); 2,42 (dd, J=10,5, 7,5 Hz, 1H, H-14); 2,92 u. 3,00 (2x d, J=9,5 Hz, je 1H, H-22 u. H-22'); 3,08 u. 3,12 (2x d, J=9,5 Hz, je 1H, H-24 u. H-24')

**36. (7*E*)-(1*R*,3*R*)-1,3-Bis[[dimethyl(1,1-dimethylethyl)silyl]oxy]-20,26,27-trimethyl-25-[(trimethylsilyl)oxy]-19-nor-23-oxa-9,10-secocholesta-5,7-dien 37**

Man löst 200 mg (0,35 mmol) (3*R*-trans)-[2-[3,5-Bis[[dimethyl(1,1-dimethylethyl)silyl]oxy]cyclohexyliden]ethyl]diphenylphosphinoxid (H.F. DeLuca et al. Tetrahedron Lett. **32**, 7663 (1991)) in 10 ml Tetrahydrofuran und kühlt unter Argon auf -70°C. Nun tropft man 0,21 ml (0,36 mmol) n-Butyllithium-Lösung (1,6 M in Hexan) zu. Nach 5 Min. werden 277 mg (0,7 mmol) 34 in 4 ml Tetrahydrofuran zugetropft und 30 Min. bei dieser Temperatur gerührt. Anschließend hydrolysiert man mit Kalium-Natriumtartrat/Kaliumhydrogencarbonat-Lösung, extrahiert mit Essigester, wäscht die organische Phase mit Natriumchlorid-Lösung, trocknet über Natriumsulfat und entfernt das Solvens. Der Rückstand wird an Kieselgel mit Hexan/Essigester chromatographiert, wobei man 80 mg der Titelverbindung als farblosen Schaum erhält.
$^1$H-NMR ($CDCl_3$): δ= 0,00 ppm (s, 12 H, $SiMe_2$); 0,07 (s, 9H, $SiMe_3$); 0,58 (s, 3H, H-18); 0,80 (t, J=7 Hz, 6H, H-28 u. H-29); 0,82 (s, 18 H, Si-t-Butyl); 0,88 u. 0,98 (2x s, je 3H, H-21 u. C-20-Methyl); 2,98 u. 3,08 (2x d, J=9 Hz, je 1H, H-22 u. H-22'); 3,12 u. 3,18 (2x d, J=9,5 Hz, je 1H, H-24 u. H-24'); 4,02 (m, 2H, H-1 u. H-3); 5,76 u. 6,12 (2x d, J=11 Hz, je 1H, H-6 u. H-7)

**37. (7*E*)-(1*R*,3*R*)-1,3-Bis[[dimethyl(1,1-dimethylethyl)silyl]oxy]-20-methyl-25-[(trimethylsilyl)oxy]-19-nor-23-oxa-9,10-secocholesta-5,7-dien 38**

Man setzt 61 mg (0,16 mmol) 35 analog **36.** um und erhält 75 mg der Titelverbindung als farblosen Schaum.
$^1$H-NMR ($CD_2Cl_2$): δ= 0,04, 0,05, 0,11 ppm (3x s, 21H, SiMe); 0,62 (s, 3H, H-18); 0,87 u. 0,88 (2x s, je 9H, Si-t-Butyl); 0,90 u. 1,01 (2x s, je 3H, H-21 u. C-20-Methyl); 1,22 (s, 6H, H-26 u. H-27); 3,10 (d, J=9,5 Hz, H-22); 3,12 (d, J=9,5 Hz, 1H, H-24); 3,17 (d, J=9,5 Hz, 1H, H-22'); 3,18 (d, J=9,5 Hz, 1H, H-24'); 4,08 (m, 2H, H-1 u. H-3); 5,80 u. 6,18 (2x d, J=11 Hz, je 1H, H-6 u. H-7)

**38. (7*E*)-(1*R*,3*R*)-1,3-Bis[[dimethyl(1,1-dimethylethyl)silyl]oxy]-26,27-diethyl-20-methyl-25-[(trimethylsilyl)oxy]-19-nor-23-oxa-9,10-secocholesta-5,7-dien 39**

Man setzt 126 mg (0,30 mmol) 36 analog **36.** um und erhält 193 mg der Titelverbindung als farblosen Schaum.
$^1$H-NMR ($CD_2Cl_2$): δ= 0,04, 0,05, 0,10 ppm (3x s, 21H, SiMe); 0,62 (s, 3H, H-18); 0,88 (t, J=7 Hz, 6H, H-30 u. H-31); 0,87 (2x s, je 9H, Si-t-butyl); 0,92 u. 1,02 (2x s, je 3H, H-21 u. C-20-Methyl); 3,03 u. 3,12 (2x d, J=9,5 Hz, je 1H, H-22); 3,18 u. 3,21 (2x d, J=9,5 Hz, je 1H, H-24); 4,08 (m, 2H, H-1 u- H-3); 5,81 u. 6,18 (2x d, J=11 Hz, je 1H, H-6 u- H-7)

**39. (7*E*)-(1*R*,3*R*)-20,26,27-Trimethyl-19-nor-23-oxa-9,10-secocholesta-5,7-dien-1,3,25-triol 40**

Man löst 80 mg (0,106 mmol) 37 in 12 ml Tetrahydrofuran, gibt unter Argon 183 mg (0,58 mmol) Tetrabutylammoniumfluorid zu und rührt 2 Std bei 55°C. Danach wird mit Natriumchlorid-Lösung versetzt, mit Essigester extrahiert, die organische Phase mit Natriumchlorid-Lösung gewaschen, über Natriumsulfat getrocknet und das Solvens entfernt. Den Rückstand chromatographiert man an Kieselgel mit Hexan/Essigester, wobei man 24 mg der Titelverbindung als farblose Kristalle erhält.
$^1$H-NMR ($CD_2Cl_2$): δ= 0,63 ppm (s, 3H, H-18); 0,85 (t, J=7 Hz, 6H, H-28 u. H-29); 0,93 u. 1,00 (2x s, je 3H, H-21 u. C-20-Methyl); 1,49 (q, J=7 Hz, 4H, H-26 u. H-27); 3,15 u. 3,17 (2x d, J=9 Hz, je 1H, H-22 u. H-22'); 3,22 u. 3,27 (2x d, J=9,5 Hz, je 1H, H-24 u. H-24'); 3,98 u. 4,07 (2x m, je 1H, H-1 u. H-3); 5,85 u. 6,28 (d, J=11 Hz, je 1H, H-6 u. H-7)
UV (MeOH): $\lambda_{max}$= 251 nm, Fp: 155°C

**40. (7*E*)-(1*R*,3*R*)-20-Methyl-19-nor-23-oxa-9,10-secocholesta-5,7-dien-1,3,25-triol 41**

Man setzt 72 mg (0,10 mmol) 38 in 5 ml Tetrahydrofuran mit 234 mg (0,75 mmol) Tetrabutylammoniumfluorid analog **39.** um und erhält nach Aufreinigung 29 mg der Titelverbindung als farblosen Schaum.
$^1$H-NMR ($CD_2Cl_2$): δ= 0,57 ppm (s, 3H, H-18); 0,87 u. 0,94 (2x s, je 3H, H-21 u. C-20-Methyl); 1,10 (s, 6H, H-26 u. H-27); 3,09 (d, J=9,5 Hz, 1H, H-24); 3,10 (s, 2H, H-22); 3,13 (d, J=9,5 Hz, 1H, H-24'); 3,91 u. 3,99 (2x m, je 1H, H-1 u. H-3); 5,77 u. 6,20 (d, J=11 Hz, je 1H, H-6 u. H-7)

**41. (7*E*)-(1*R*,3*R*)-26,27-Diethyl-20-methyl-19-nor-23-oxa-9,10-secocholesta-5,7-dien-1,3,25- triol 42**

Man setzt 190 mg (0,24 mmol) 39 in 12 ml Tetrahydrofuran mit 571 mg (1,83 mmol) Tetrabutylammoniumfluorid analog **39.** um und erhält nach Aufreinigung 87 mg der Titelverbindung als farblosen Schaum.
$^1$H-NMR ($CD_2Cl_2$): δ= 0,57 ppm (s, 3H, H-18); 0,84 (t, J=7 Hz, 6H, H-30 u. H-31); 0,85 u. 0,92 (2x s, je 3H, H-21 u. C-20-Methyl); 3,08 (s, 2H, H-22); 3,11 (d, J=9,5 Hz, 1H, H-24); 3,17 (d, J=9,5 Hz, 1H, H-24'); 3,91 u. 3,99 (2x m, je 1H, H-1 u. H-3); 5,77 u. 6,20 (2x d, J=11 Hz, je 1H, H-6 u. H-7)

**42. [1*S*-(1α,3aβ,4α,7aα)]-4-[[Dimethyl(1,1-dimethylethyl)silyl]oxy]octahydro-α,α,7a-trimethyl-1H-inden-acetaldehyd 43**

Man setzt 9,2 g (28,34 mmol) [1*R*-[1α(*S**),3aβ,4α,7aα]]-α,7a-Dimethyl-4-[[dimethyl-(1,1-dimeth ylethyl)silyl]oxy]-octahydro-1H-inden-1-acetaldehyd (W.G. Dauben et al. Tetrahedron Lett. 30, 677 (1989) mit 1,02 g (34,05 mmol) Natriumhydrid (80%) und 12,07 g (85,03 mmol) Jodmethan in 130 ml Tetrahydrofuran analog **24.** um und erhält 7,89 g der Titelverbindung als farbloses Öl.
$^1$H-NMR ($CDCl_3$): δ= 0,01 ppm (2x s, je 3H, SiMe); 0,88 (s, 9H, Si-t-Butyl); 0,98 (s, 3H, H-18); 1,09 u. 1,12 (2x s, je 3H, H-21 u. C-20-Methyl); 4,01 (m, 1H, H-8); 9,68 (s, 1H, H-22)

**43. [1*S*-(1α,3aβ,4α,7aα)]-4-[[Dimethyl(1,1-dimethylethyl)silyl]oxy]octahydro-β,β,7a-trimethyl-1H-inden-1-ethanol 44**

Man setzt 3,5 g (10,33 mmol) 43 mit 1,53 g (11,1 mmol) Certrichlorid-Heptahydrat und 365 mg (9,53 mmol) Natriumborhydrid in 27 ml Tetrahydrofuran/27 ml Methanol analog **25.** um. Es werden 2,36 g der Titelverbindung als farbloses Öl erhalten.
$^1$H-NMR ($CDCl_3$): δ= 0,01 ppm (2x s, je 3H, SiMe); 0,89 (s, 9H, Si-t-Butyl); 0,89 (s, 3H, H-18); 0,99 u. 1,05 (2x s, je 3H, H-21 u. H-20-Methyl); 1,60 (t, J=5 Hz, 1H, OH); 3,30 (dd, J=11, 5,5 HZ, 1H, H-22); 3,36 (dd, J=11,5 Hz, 1H, H-22'); 4,00 (m, 1H, H-8)

**44. [1*S*-(1α,3aβ,4α,7aα)]-4-[2-[4-[[Dimethyl(1,1-dimethylethyl)silyl]oxy]-7a-methyl-octahy dro-1H-inden-1-yl]-2-methylpropoxy]butansäuremethylester 45**

Man setzt 2,36 g (6,93 mmol) 44 mit 6,3 g (27,7 mmol) 4-Brombuttersäureorthotrimethylester und 366 mg Tetrabutylammoniumhydrogensulfat in 9,3 ml Natronlauge (25%) und 3 ml Toluol analog **11.** um und erhält 3,14 g der Titelverbindung als farbloses Öl.
$^1$H-NMR ($CDCl_3$): δ= 0,01 ppm (2x s, je 3H, SiMe); 0,89 (s, 9H, Si-t-Butyl); 0,89 (s, 3H, H-18); 0,98 u. 1,03 (2x s, je 3H, H-21 u. C-20-Methyl); 2,41 (t, J=7 Hz, 2H, H-26); 3,03 (d, J=9 Hz, 1H, H-22); 3,10 (d, J=9 Hz, 1H, H-22'); 3,38 (t, J=7 Hz, 2H, H-24); 3,70 (s, 3H, COOMe); 4,00 (m, 1H, H-8)

**45. [1*S*-(1α,3aβ,4α,7aα)]-5-[2-[4-[[Dimethyl(1,1-dimethylethyl)silyl]oxy]-7a-methyloctahydro-1H-inden-1-yl]-2-methylpropoxy]-2-methyl-2-pentanol 46**

Aus 1,21 g (8.5 mmol) Jodmethan und 206 mg (8,5 mmol) Magnesiumspänen in 10 ml Diethylether wird das Grignard-Reagenz bereitet und mit 750 mg (1,7 mmol) 45 analog **27.** umgesetzt. Man erhält 453 mg der Titelverbindung als farbloses Öl.
$^1$H-NMR ($CDCl_3$): δ= 0,02 ppm (2x s, je 3H, SiMe); 0,89 (s, 9H, Si-t-Butyl); 0,92 (s, 3H, H-18); 1,00 u. 1,04 (2x s, je 3H, H-21 u. C-20-Me); 1,22 (s, 6H, H-28 u. H-29); 3,09 (d, J=9,5 Hz, 1H, H-22); 3,18 (d, J=9,5 Hz, 1H, H-22'); 3,42 (t, J=7 Hz, 2H, H-24); 4,00 (m, 1H, H-8)

**46. [1*S*-(1α,3aβ,4α,7aα)]-1-[2-[4-[[Dimethyl(1,1-dimethylethyl)silyl]oxy]-7a-methyloctahydro-1H-inden-1-yl]-2-methylpropoxy]-4-ethyl-4-hexanol 47**

Aus 935 mg (8,5 mmol) Bromethan und 206 mg (8,5 mmol) Magnesiumspänen in 20 ml Tetrahydrofuran wird das Grignard-Reagenz bereitet und analog **27.** mit 750 mg (1,7 mmol) 45 umgesetzt. Man erhält 412 mg der Titelverbindung als farbloses Öl.
$^1$H-NMR ($CDCl_3$): δ= 0,01 ppm (2x s, je 3H, SiMe); 0,88 (t, J=7 Hz, 6H, H-30 u. H-31); 0,89 (s, 9H, Si-t-Butyl); 0,90 (s, 3H, H-18); 1,00 u. 1,04 (2x s, je 3H, H-21 u. C-20-Methyl); 1,48 (q, J=7 Hz, 4H, H-28 u. H-29); 3,07 (d, J=9,5 Hz, 1H, H-22); 3,14 (d, J=9,5 Hz, 1H, H-22'); 3,40 (t, J=7 Hz, 2H, H-24); 3,99 (m, 1H, H-8)

**47. [1*S*-(1α,3aβ,4α,7aα)]-1-[1,1-Dimethyl-[2-(4-hydroxy-4-methylpentoxy)ethyl]-7a-methyloctahydro-1H-inden-4-ol 48**

Man rührt 400 mg (0,91 mmol) 46 in 8,2 ml Tetrahydrofuran/Acetonitril (1:1) mit 4,1 ml Fluorwasserstoffsäure (40%) für 30 min bei Raumtemperatur. Es wird mit verdünnter Natronlauge neutralisiert, mit Essigester extrahiert, die organische Phase mit Natriumchlorid-Lösung gewaschen und über Natriumsulfat getrocknet. Nach Entfernung des Lösungsmittels wird der Rückstand chromatographisch gereinigt, wobei man 266 mg der Titelverbindung als farbloses Öl erhält.
$^1$H-NMR ($CDCl_3$): δ= 0,84 ppm (s, 3H, H-18); 0,92 u. 1,00 (2x s, je 3H, H-21 u. C-20-Methyl); 1,17 (s, 6H, H-28 u. H-29); 3,01 u. 3.09 (2x d, J=9,5 Hz, je 1H, H-22 u. H-22'); 3,36 (t, J=7 Hz, 2H, H-24); 4,00 (m, 1H, H-8)

**48. [1*S*-(1α,3aβ,4α,7aα)]-1-[1,1-Dimethyl-[2-(4-ethyl-4-hydroxyhexoxy)ethyl]-7a-methyloctahydro-1H-inden-4-ol 49**

Man setzt 412 mg (0,87 mmol) 47 mit 3,9 ml Fluorwasserstoffsäure (40%) in 7,8 ml Tetrahydrofuran/Acetonitril (1:1) analog **47.** um und erhält 251 mg der Titelverbindung als farbloses Öl.
$^1$H-NMR ($CDCl_3$): δ= 0,80 ppm (t, J=7 Hz, 6H, H-30 u. H-31); 0,82 (s, 3H, H-18); 0,93 u. 0,99 (2x s, je 3H, H-21 u. C-20-Methyl); 1,42 (q, J=7 Hz, 4H, H-28 u. H-29); 3,00 u. 3,08 (2x d, J=9,5 Hz, je 1H, H-22 u. H-22'); 3,33 (t, J=7 Hz, 2H, H-24); 4,00 (m, 1H, H-8)

**49. [1*S*-(1α,3aβ,7aα)]-1-[1,1-Dimethyl-[2-(4-hydroxy-4-methylpentoxy)ethyl]-7a-methyloctahydro-4H-inden-4-on 50**

Man setzt 260 mg (0,80 mmol) 48 mit 240 mg (1,12 mmol) Pyridiniumchlorochromat in 16 ml Methylenclorid analog **30.** um und erhält 201 mg der Titelverbindung als farbloses Öl.
$^1$H-NMR ($CDCl_3$): δ= 0,69 ppm (s, 3H, H-18); 0,88 u. 1,01 (2x s, je 3H, H-21 u. C-20-Methyl); 1,22 (s, 6H, H-28 u. H-29); 2,42 (dd, J=10,5, 7,5 Hz, 1H, H-14); 3,07 u. 3,13 (2x d, J=9,5 Hz, je 1H, H-22 u. H-22'); 3,39 (t, J=7 Hz, 2H, H-24)

**50. [1*S*-(1α,3aβ,7aα)]-1-[1,1-Dimethyl-[2-(4-ethyl-4-hydroxyhexoxy)ethyl]-7a-methyloctahydro-4H-inden-4-on 51**

Man setzt 251 mg (0,71 mmol) 49 mit 212 mg (0,99 mmol) Pyridiniumchlorochromat in 16 ml Methylenchlorid analog **30.** um und erhält 183 mg der Titelverbindung als farbloses Öl.
$^1$H-NMR ($CDCl_3$): δ= 0,70 ppm (s, 3H, H-18); 0,86 (t, J=7 Hz, 6H, H-30 u. H-31); 0,90 u. 1,01 (2x s, je 3H, H-21 u. H-20-Methyl); 1,48 (q, J=7 Hz, 4H, H-28 u. H-29); 2,42 (dd, J=10,5, 7,5 Hz, 1H, H-14); 3,08 u. 3,15 (2x d, J=9,5 Hz, je 1H, H-22 u. H-22'); 3,40 (t, J=7 Hz, 2H, H-24)

**51. [1*S*-(1α,3aβ,7aα)]-1-[1,1-Dimethyl-[2-[4-methyl-4-[(trimethylsilyl)oxy]pentoxy]ethyl]-7a-methyloctahydro-4H-inden-4-on 52**

Man setzt 201 mg (0,62 mmol) 50 mit 205 mg (1,86 mmol) Trimethylchlorsilan, 167 mg (2,42 mmol) Imidazol und 0,25 ml Pyridin in 15 ml Diethylether analog **33.** um und erhält 194 mg der Titelverbindung als farbloses Öl.
$^1$H-NMR ($CDCl_3$): δ= 0,11 ppm (s, 9H, $SiMe_3$); 0,82 (s, 3H, H-18); 0,90 u. 1,01 (2x s, je 3H, H-21 u. C-20-Methyl); 1,22 (s, 6H, H-29 u. H-30); 2,43 (dd, J=10,5, 7,5 Hz, 1H, H-14); 3,07 u. 3,12 (2x d, J=9,5 Hz, je 1H, H-22); 3,37 (t, J=7 Hz, 2H, H-24)

**52. [1*S*-(1α,3aβ,7aα)]-1-[1,1-Dimethyl-[2-[4-ethyl-4-[(trimethylsilyl)oxy]hexoxy]ethyl]-7a-methyloctahydro-4H-inden-4-on 53**

Man setzt 183 mg (0,52 mmol) 51 mit 171 mg (1,56 mmol) Trimethylchlorsilan, 140 mg (2,03 mmol) Imidazol und 0,21 ml Pyridin in 15 ml Diethylether analog **33.** um und erhält 178 mg der Titelverbindung als farbloses Öl.
$^1$H-NMR ($CDCl_3$): δ= 0,10 ppm (s, 9H, $SiMe_3$); 0,70 (s, 3H, H-18); 0,82 (t, J=7 Hz, 6H, H-30 u. H-31); 0,91 u. 1,00 (2x s, je 3H, H-21 u. C-20-Methyl); 2,42 (dd, J=10,5, 7,5 Hz, 1H, H-14); 3,05 u. 3,11 (2x d, J=9,5 Hz, je 1H, H-22); 3,35 (t, J=7 Hz, 2H, H-24)

**53. (7*E*)-(1*R*,3*R*)-1,3-Bis[[dimethyl(1,1-dimethylethyl)silyl]oxy]-20-methyl-24-[3-methyl -3-[(trimethylsilyl)oxy]butyl]-19-nor-23-oxa-9,10-secochola-5,7-dien 54**

Man setzt 100 mg (0,25 mmol) 52 analog **36.** um und erhält 130 mg der Titelverbindung als farblosen Schaum.
$^1$H-NMR ($CDCl_3$): δ= 0,01 u. 0,08 ppm (2x s, 21H, $SiMe_3$ u. SiMe); 0,58 (s, 3H, H-18); 0,82 (s, 18H, Si-t-Butyl); 0,86 u. 0,97 (2x s, je 3H, H-21 u. C-20-Methyl); 1,18 (s, 6H, H-28 u. H-29); 3,01 u. 3,09 (2x d, J=9,5 Hz, je 1H, H-22 u. H-22'); 3,32 (t, J=7 Hz, 2H, H-24); 4,03 (m, 2H, H-1 u. H-3); 5,76 u. 6,12 (2x d, J=11 Hz, je 1H, H-6 u. H-7)

**54. (7*E*)-(1*R*,3*R*)-1,3-Bis[[dimethyl(1,1-dimethylethyl)silyl]oxy]-20-methyl-24-[3-ethyl-3-[(trimethylsilyl)oxy]pentyl]-19-nor-23-oxa-9,10-secochola-5,7-dien 55**

Man setzt 100 mg (0,24 mmol) 53 analog **36.** um und erhält 111 mg der Titelverbindung als farblosen Schaum.
$^1$H-NMR ($CDCl_3$): δ= 0,02 u. 0,10 ppm (2x s, 21H, $SiMe_3$ u. SiMe); 0,56 (s, 3H, H-18); 0,79 (t, J=7 Hz, 6H, H-30 u. H-31); 0,83 (s, 18H, Si-t-Butyl); 0,85 u. 0,95 (2x s, je 3H, H-21 u. C-20-Methyl); 3,02 u. 3,08 (2x d, J=9,5 Hz, je 1H, H-22 u. H-22'); 3,26 (t, J=7 Hz, 2H, H-24); 3,99 (m, 2H, H-1 u. H-3); 5,70 u. 6,07 (2x d, J=11 Hz, je 1H, H-6 u. H-7)

**55. (7*E*)-(1*R*,3*R*)-24-(3-Hydroxy-3-methylbutyl)-20-methyl-19-nor-23-oxa-9,10-secochola-5,7-dien-1,3-diol 56**

Man setzt 125 mg (0,17 mmol) 54 mit 424 mg (1,36 mmol) Tetrabutylammoniumfluorid in 10 ml Tetrahydrofuran analog **39.** um und erhält 56 mg der Titelverbindung als farblosen Schaum.
$^1$H-NMR ($CD_2Cl_2$): δ= 0,55 ppm (s, 3H, H-18); 0,82 u. 0,90 (2x s, je 3H, H-21 u. C-20-Methyl); 1,10 (s, 6H, H-28 u. H-29); 3,00 u. 3,08 (2x d, J=9,5 Hz, je 1H, H-22 u. H-22'); 3,30 (t, J=7 Hz, 2H, H-24); 3,91 u. 3,98 (2x m, je 1H, H-1 u. H-3); 5,77 u. 6,20 (2x d, J=11 Hz, je 1H, H-6 u. H-7)

**56. (7*E*)-(1*R*,3*R*)-24-(3-Ethyl-3-hydroxypentyl)-20-methyl-19-nor-23-oxa-9,10-secochola-5,7-dien-1,3-diol 57**

Man setzt 106 mg (0,14 mmol) 55 mit 340 mg (1,09 mmol) Tetrabutylammoniumfluorid in 10 ml Tetrahydrofuran analog **39.** um und erhält 57 mg der Titelverbindung als farblosen Schaum.
$^1$H-NMR ($CD_2Cl_2$): δ= 0,54 ppm (s, 3H, H-18); 0,77 (t, J=7 Hz, 6H, H-30 u. H-31); 0,81 u. 0,90 (2x s, je 3H, H-21 u. C-20-Methyl); 1,38 (q, J=7 Hz, 4H, H-28 u. H-29); 3,00 u. 3,08 (2x d, J=9,5 Hz, je 1H, H-22 u. H-22'); 3,30 (t, J=7 Hz, 2H, H-24); 3,91 u. 3,98 (2x m, je 1H, H-1 u. H-3); 5,77 u. 6,20 (2x d, J=11 Hz, je 1H, H-6 u. H-7)

**57. [1*S*-(1α,3aβ,4α,7aα)]-1-[4-(Acetyloxy)-7a-methyloctahydro-1H-inden-1-yl]ethanon 58**

Man löst 19,2 g (76,1 mmol) [1*R*-[1α(*S**),3aβ,4α,7aα]]-4-(Acetyloxy)-α,7a-dimethyloctahydro-1H-inden-1-acetaldehyd (siehe **24.**) in 1000 ml N,N-Dimethylformamid, setzt 7,59 g (66,5 mmol) 1,4-Diazabicyclo[2.2.2]octan, 1,14 g (5,7 mmol) Kupfer(II)acetat-Monohydrat und 909 mg (5,7 mmol) 2,2'-Bipyridyl zu und erhitzt unter Sauerstoffeinleitung auf 70°C. Nach 24 h wird auf Natriumchlorid-Lösung gegossen, mit Essigester extrahiert, mit Natriumchlorid-Lösung gewaschen, über Natriumsulfat getrocknet, das Solvens entfernt und der Rückstand an Kieselgel mit Hexan/Essigester chromatographiert, wobei 12,75 g der Titelverbindung als farbloses Öl anfallen.
$^1$H-NMR ($CDCl_3$): δ= 0,83 ppm (s, 3H, H-18); 2,03 (s, 3H, H-21); 2,12 (s, 3H, OAc); 2,50 (t, J=9,5 Hz, 1H, H-17); 5,19 (m, 1H, H-8)

**58. [1*S*-(1α,3aβ,4α,7aα)]-7a-Methyl-1-(2-methyl-2-oxiranyl)octahydro-1H-inden-4-ol 59**

Man löst 12,75 g (53,5 mmol) 58 in 400 ml N,N-Dimethylformamid und gibt 15,2 g (86 mmol) Trimethylsulfoniumjodid zu. Es wird auf 0°C gekühlt und portionsweise werden 29,8 g (267 mmol) Kalium-tert.-butylat zugegeben. Man rührt 24 Std. bei Raumtemperatur nach und gibt dann Natriumchlorid-Lösung zu. Nach Extraktion mit Essigester, Waschen der organischen Phase mit Natriumchlorid-Lösung, Trocknen über Natriumsulfat und Entfernen des Lösungsmittels reinigt man das Rohprodukt durch Chromatographie an Kieselgel mit Hexan/Essigester als Solvens, wobei 7,91 g der Titelverbindung als farbloses Öl anfallen.
$^1$H-NMR ($CDCl_3$): δ= 1,00 ppm (s, 3H, H-18); 1,30 (s, 3H, H-21); 2,25 (d, J=5 Hz, 1H, H-22); 2,43 (d, J=5 Hz, 1H, H-22'); 4,02 (m, 1H, H-8)

**59. [1*S*-(1α,3aβ,4α,7aα)]-4-[[Dimethyl(1,1-dimethylethyl)silyl]oxy]-7a-methyl-1-(2-methyl-2-oxiranyl)octahydro-1H-inden 60**

Zu einer Lösung von 7,91 g (37,6 mmol) 59 in 200 ml N,N-Dimethylformamid werden 10,19 g (150 mmol) Imidazol und 11,25 g (75 mmol) tert.-Butyldimethylsilylchlorid gegeben und über Nacht bei Raumtemperatur gerührt. Danach gießt man auf Natriumchlorid-Lösung, extrahiert mit Essigester, wäscht die organische Phase mit Natriumchlorid-Lösung, trocknet über Natriumsulfat und entfernt das Solvens. Der Rückstand wird in 50 ml Diethylether gelöst und bei 0°C zu einer Lösung von Lithiumdiethylamid [2,77 g (37,8 mmol) Diethylamin, 24,28 ml n-Butyllithium-Lösung (1,6 M in Hexan)] getropft. Man rührt erneut 24 Std. bei Raumtemperatur, gibt Natriumchlorid-Lösung zu, extrahiert mit Essigester, wäscht die organische Phase mit Natriumchlorid-Lösung, trocknet über Natriumsulfat und entfernt das Solvens. Chromatographische Aufreinigung liefert 8,64 g der Titelverbindung als farbloses Öl.
$^1$H-NMR ($CDCl_3$): δ= 0,01 u. 0,10 ppm (2x s, je 3H, SiMe); 0,88 (s, 9H, Si-t-Butyl); 0,90 (s, 3H, H-18); 1,36 (s, 3H, H-21); 2,31 (d, J=5 Hz, H-22); 2,50 (d, J=5 Hz, H-22'); 4,00 (m, 1H, H-8)

**60. [1*S*-(1α,3aβ,4α,7aα)]-4-[[Dimethyl(1,1-dimethylethyl)silyl]oxy]-7a-methyl-β-methylenoctahydro-1H-inden-1-ethanol 61**

Man löst 8,46 g (26,06 mmol) 60 in 500 ml Toluol, versetzt mit 10,8 g (53,2 mmol) Aluminiumisopropylat und rührt über Nacht bei 120°C. Nach Abkühlen gibt man Wasser/Isopropanol zu, filtriert, engt das Filtrat ein und chromatographiert den Rückstand an Kieselgel mit Hexan/Essigester, wobei 7,56 g der Titelverbindung anfallen.
$^1$H-NMR ($CDCl_3$): δ= 0,01 u. 0,02 ppm (2x s, je 3H, SiMe); 0,80 (s, 3H, H-18); 0,89 (s, 9H, Si-t-Butyl); 2,05 (t, J=9,5 Hz, 1H, H-17); 4,00 (dbr, J=15 Hz, 1H, H-22); 4,02 (m, 1H, H-8); 4,10 (dd, J=15, 5 Hz, 1H, H-22'); 4,92 u. 5,20 (2x s, je 1H, H-21)

**61. [1*S*-[1α,3aβ,4α,7aα]]-1,1-Dimethylethyl-2-[4-[[dimethyl-(1,1-dimethylethyl)silyl]oxy]-7a-methyloctahydro-1H-inden-1-yl]-2-propenoxy]acetat 62**

Man setzt 2 g (6,16 mmol) 61 in 18 ml Toluol mit 27,6 ml Natronlauge (25%), 129 mg Tetrabutylammoniumhydrogensulfat und 5,96 g (30,55 mmol) Bromessigsäure-tert.-butylester analog **3.** um und erhält 2,67 g der Titelverbindung als farbloses Öl.
$^1$H-NMR ($CDCl_3$): δ= 0,01 ppm (2x s, je 3H, SiMe); 0,80 (s, 3H, H-18); 0,90 (s, 9H, Si-t-Butyl); 1,50 (s, 9H, t-Butylester); 3,93 (s, 2H, H-24); 3,98 (s, 2H, H-22); 4,02 (m, 1H, H-8); 4,98 u. 5,22 (2x s, je 1H, H-21)

**62. [1*S*-[1α,3aβ,4α,7aα]]-3-[[[2-[4-[[Dimethyl(1,1-dimethylethyl)silyl]oxy]-7a-methyloctahydro-1H-inden-1-yl]-2-propenyl]oxy]methyl]-3-pentanol 63**

Aus 1,34 g (12,32 mmol) Bromethan und 300 mg (12,3 mmol) Magnesiumspänen in 30 ml Tetrahydrofuran wird das Grignard-Reagenz bereitet und mit 2,67 g (6,24 mmol) 62 analog **4.** umgesetzt. Man erhält 2,58 g der Titelverbindung als farbloses Öl, das ohne Aufreinigung weiter umgesetzt wird.

**63. [1*S*-[1α,3aβ,4α,7aα]]-1-[2-[4-[[Dimethyl(1,1-dimethylethyl)silyl]oxy]-7a-methyloctahydro-1H-inden-1-yl]-2-propenoxy]-2-methyl-2-propanol 64**

Aus 2,52 g (21,3 mmol) Jodmethan und 517 mg (21,3 mmol) Magnesium-Spänen in 100 ml Diethylether wird das Grignard-Reagenz bereitet und mit 3,4 g (7,7 mmol) 62 analog **4.** umgesetzt. Man erhält 2,65 g der Titelverbindung.
$^1$H-NMR ($CDCl_3$): δ=0,01 ppm (2x s, je 3H, SiMe); 0,80 (s, 3H, H-18); 0,88 (s, 9H, Si-t-Butyl); 1,21 (s, 6H, H-26 u. H-27); 3,19 u. 3,25 (2x d, J=9 Hz, je 1H, H-24 u. H-24'); 3,89 u. 3,96 (2x d, J=13 Hz, je 1H, H-22 u. H-22'); 4,01 (m, 1H, H-8); 4,93 u. 5,18 (2x s, je 1H, H-21 u. H-21')

**64. [1*S*-[1α,3aβ,4α,7aα]]-1-[2-(2-Ethyl-2-hydroxybutoxy)-1-methylenethyl]-7a-methyloctahydro-1H-inden-4-ol 65**

Man löst 2,58 g (6,07 mmol) 63 in 60 ml Tetrahydrofuran, gibt 12,4 ml HF/Pyridin (70%) zu und rührt 3 Tage bei Raumtemperatur. Anschließend wird auf Natriumhydrogensulfat-Lösung gegossen, mit Essigester extrahiert die organische Phase mit Natriumhydrogencarbonat-Lösung und Natriumchlorid-Lösung gewaschen und über Natriumsulfat getrocknet. Entfernen des Lösungsmittels und chromatographische Aufreinigung liefern 750 mg der Titelverbindung als farbloses Öl.
$^1$H-NMR ($CDCl_3$): δ= 0,78 ppm (s, 3H, H-18); 0,81 (t, J=7 Hz, 6H, H-28 u. H-29); 1,48 (q, J=7 Hz, 4H, H-26 u. H-27); 3,17 u. 3,23 (2x d, J=9 Hz, je 1H, H-24 u. H-24'); 3,81 u. 3,90 (2x d, J=12,5 Hz, je 1H, H-22 u. H-22'); 4,03 (m, 1H, H-8); 4,89 u. 5,14 (2x s, je 1H, H-21 u. H-21')

**65. [1*S*-[1α,3aβ,4α,7aα]]-1-[2-(2-Hydroxy-2-methylpropoxy)-1-methylenethyl]-7a-methyloctahydro-1H-inden-4-ol 66**

Man setzt 2 g (5 mmol) 64 in 38 ml Acetonitril und 30 ml Tetrahydrofuran mit 30 ml HF (40%) um und rührt 3 Std. bei Raumtemperatur. Anschließend gibt man vorsichtig Natriumhydrogencarbonat-Lösung zu, extrahiert mit Essigester, wäscht mit Natriumchlorid-Lösung und trocknet über Natriumsulfat. Nach Entfernen des Lösungsmittels und chromatographischer Reinigung erhält man 1,25 g der Titelverbindung als farbloses Öl.
$^1$H-NMR ($CDCl_3$): δ= 0,85 ppm (s, 3H, H-18); 1,22 (s, 6H, H-26 u. H-27); 3,21 u. 3,28 (2x d, J=9 Hz, je 1H, H-24 u. H-24'); 3,91 u. 3,98 (2x d, J=13 Hz, je 1H, H-22 u. H-22'); 4,10 (m, 1H, H-8); 4,97 u. 5,22 (2x s, je 1H, H-21 u. H-21')

**66. [1*S*-[1α,3aβ,7aα]]-1-[2-(2-ethyl-2-hydroxybutoxy)-1-methylenethyl]-7a-methyloctahydro-4H-inden-4-on 67**

Man setzt 720 mg (2,40 mmol) 65 mit 755 mg (3,51 mmol) Pyridiniumchlorochromat in 50 ml Methylenchlorid analog **30.** um und erhält 522 mg der Titelverbindung als farbloses Öl.
$^1$H-NMR ($CDCl_3$): δ= 0,52 ppm (s, 3H, H-18); 0,80 (t, J=7 Hz, 6H, H-28 u. H-29); 1,21 (s, 1H, OH); 1,48 (q, J=7 Hz, 4H, H-26 u. H-27); 3,18 u. 3,23 (2x d, J=9,5 Hz, je 1H, H-24 u. H-24'); 3,84 u. 3,95 (2x d, J=13 Hz, je 1H, H-22 u. H-22'); 4,96 u. 5,16 (2x s, je 1H, H-21 u. H-21')

**67. [1*S*-[1α,3aβ,7aα]]-1-[2-(2-Hydroxy-2-methylpropoxy)-1-methylenethyl]-7a-methyloctahydro-4H-inden-4-on 68**

Man setzt 600 mg (2,12 mmol) 66 mit 645 mg (3 mmol) Pyridiniumchlorochromat in 40 ml Methylenchlorid analog **30.** um und erhält 439 mg der Titelverbindung als farbloses Öl.
$^1$H-NMR ($CDCl_3$): δ= 0,58 ppm (s, 3H, H-18); 1,22 (s, 6H, H-26 u. H-27); 2,57 (dd, J=10,5, 7,5 Hz, 1H, H-14); 3,22 u. 3,28 (2x d, J=9 Hz, je 1H, H-24 u. H-24'); 3,95 u. 4,04 (2x d, J=13 Hz, je 1H, H-22 u. H-22'); 5,02 u. 5,23 (2x s, je 1H, H-21 u. H-21')

**68. [1*S*-[1α,3aβ,7aα]]-1-[2-[[2-Ethyl-2-[(trimethylsilyl)oxy]butoxy]-1-methylenethyl]-7a-methyloctahydro-4H-inden-4-on 69**

Man setzt 522 mg (1,75 mmol) 67 mit 554 mg (5,1 mmol) Trimethylchlorsilan, 459 mg (6,63 mmol) Imidazol und 0,7 ml Pyridin in 50 ml Diethylether anlog **33.** um und erhält 343 mg der Titelverbindung als farbloses Öl.

$^1$H-NMR ($CDCl_3$): δ= 0,01 ppm (s, 9H, $SiMe_3$); 0,48 (s, 3H, H-18); 0,77 (t, J=7 Hz, 6H, H-28 u. H-29); 3,11 u. 3,18 (2x d, J=9,5 Hz, je 1H, H-24 u. H-24'); 3,75 u. 3,87 (2x d, J=13 Hz, je 1H, H-22 u. H-22'); 4,91 u. 5,16 (2x s, je 1H, H-21 u. H-21')

**69. 1*S*-[1α,3aβ,7aα]]-7a-Methyl-1-[2-[[2-methyl-2-[(trimethylsilyl)oxy]propoxy]-1-methylenethyl]octahydro-4H-inden-4-on 70**

Man setzt 420 mg (1,5 mmol) 68 mit 325 mg (3 mmol) Trimethylchlorsilan, 408 mg (6 mmol) Imidazol und 0,6 ml Pyridin in 50 ml Diethylether anlog **33.** um und erhält 445 mg der Titelverbindung als farbloses Öl.
$^1$H-NMR ($CDCl_3$): δ= 0,10 ppm (s, 9H, $SiMe_3$); 0,58 (s, 3H, H-18); 1,25 (s, 6H, H-26 u. H-27); 2,57 (dd, J=10,5, 7,5 Hz, 1H, H-14); 3,18 u. 3,22 (2x d, J=9 Hz, je 1H, H-24 u. H-24'); 3,92 u. 4,00 (2x d, J=13 Hz, je 1H, H-22 u. H-22'); 5,01 u. 5,24 (2x s, je 1H, H-21 u. H-21')

**70. (7*E*)-(1*R*,3*R*)-1,3-Bis[[dimethyl(1,1-dimethylethyl)silyl]oxy]-26,27-dimethyl-25-(trimethylsilyl)oxy-19-nor-23-oxa-9,10-secocholesta-5,7,20-trien 71**

Man setzt 300 mg (0,81 mmol) 69 analog **36.** um und erhält 508 mg der Titelverbindung als farblosen Schaum.
$^1$H-NMR ($CDCl_3$): δ= 0,06 u. 0.09 ppm (2x s, 12H u. 9H, SiMe); 0,47 (s, 3H, H-18); 0,83 u. 0,84 (2x s, je 9H, Si-t-Butyl); 0,85 (t, J=7 Hz, 6H, H-28 u. H-29); 1,52 (q, J=7 Hz, 4H, H-26 u. H-27); 3,20 u. 3,27 (2x d, J=9,5 Hz, je 1H, H-24 u. H-24'); 3,84 u. 3,91 (2x d, J=13 Hz, je 1H, H-22 u. H-22'); 4,09 (m, 2H, H-1 u. H-3); 4,97 u. 5,20 (2x s, je 1H, H-21 u. H-21'); 5,83 u. 6,18 (2x d, J=11 Hz, je 1H, H-6 u. H-7)

**71. (7*E*)-(1*R*,3*R*)-1,3-Bis[[dimethyl(1,1-dimethylethyl)silyl]oxy]-25-(trimethylsilyl)oxy-19-nor-23-oxa-9,10-secocholesta-5,7,20-trien 72**

Man setzt 352 mg (1 mmol) 70 analog **36.** um und erhält 50 mg der Titelverbindung als farblosen Schaum, die direkt weiter umgesetzt wurde.

**72. (7*E*)-(1*R*,3*R*)-26,27-Dimethyl-19-nor-23-oxa-9,10-secocolesta-5,7,20-trien-1,3,25-triol 73**

Man setzt 500 mg (0,68 mmol) 71 mit 1,70 g (5,44 mmol) Tetrabutylammoniumfluorid in 45 ml Tetrahydrofuran analog **39.** um und erhält 217 mg der Titelverbindung als farblosen Schaum.
$^1$H-NMR ($CDCl_3$): δ= 0,40 ppm (s, 3H, H-18); 0,82 (t, J=7 Hz, 6H, H-28 u. H-29); 1,48 (q, J=7 Hz, 4H, H-26 u. H-27); 3,18 u. 3,23 (2x d, J=9 Hz, je 1H, H-24 u. H-24'); 3,84 u. 3,92 (2x d, J=13 Hz, je 1H, H-22 u. H-22'); 3,99 u. 4,06 (2x m, je 1H, H-1 u. H-3); 4,92 u. 5,11 (2x s, je 1H, H-21 u. H-21'); 5,81 u. 6,24 (2x d, J=11 Hz, je 1H, H-6 u. H-7)

**73. (7*E*)-(1*R*,3*R*)-19-Nor-23-oxa-9,10-secocolesta-5,7,20-trien-1,3,25-triol 74**

Man setzt 50 mg (0,07 mmol) 72 mit 121 mg (0,385 mmol) Tetrabutylammoniumfluorid in 10 ml Tetrahydrofuran analog **39.** um und erhält 22,5 mg der Titelverbindung als farblosen Schaum.
$^1$H-NMR ($CD_2Cl_2$): δ= 0,40 ppm (s, 3H, H-18); 1,10 (s, 6H, H-26 u. H-27); 3,12 u. 3,18 (2x d, J=9 Hz, je 1H, H-24 u. H-24'); 3,85 u. 3,92 (2x d, J=13 Hz, je 1H, H-22 u. H-22'); 3,93 u. 4,00 (2x m, je 1H, H-1 u. H-3); 4,91 u. 5,10 (2x s, je 1H, H-21 u. H-21'); 5,70 u. 6,20 (2x d, J=11 Hz, je 1H, H-6 u. H-7)

**74. [1*S*-[1α,3aβ,4α,7aα]]-4-[2-[4-[[Dimethyl(1,1-dimethylethyl)silyl]oxy]-7a-methyloctahydro-1H-inden-1-yl]-2-propenoxy]butansäuremethylester 75**

Man setzt 2,30 g (7,09 mmol) 61 mit 6,45 g (28,4 mmol) 4-Brombuttersäureorthotrimethylester, 9,5 ml Natronlauge (50%) und 375 mg Tetrabutylammoniumhydrogensulfat analog **11.** um und erhält 2,31 g der Titelverbindung als farbloses Öl.
$^1$H-NMR ($CDCl_3$): δ= 0,01 ppm (2x s, je 3H, SiMe); 0,80 (s, 3H, H-18); 0,89 (s, 9H, Si-t-Butyl); 2,42 (t, J=6,5 Hz, 2H, H-26); 3,40 (t, J=6,5 Hz, 2H, H-24); 3,68 (s, 3H, COOMe); 3,85 (s, 2H, H-22); 4,01 (m, 1H, H-8); 4,92 u. 5,18 (2x s, je 1H, H-21 u. H-21')

**75. [1*S*-[1α,3aβ,4α,7aα]]-1-[2-[4-[[Dimethyl(1,1-dimethylethyl)silyl]oxy]-7a-methyloctahydro-1H-inden-1-yl]-2-propenoxy]-4-methyl-4-pentanol 76**

Man bereitet das Grignard-Reagenz aus 1,15 g (8,1 mmol) Jodmethan und 196 mg (8,1 mmol) Magnesiumspänen in 15 ml Diethylether und setzt mit 1,15 g (2,7 mmol) 75 analog **4.** um und erhält 934 mg der Titelverbindung als farb-

loses Öl.
$^1$H-NMR ($CDCl_3$): δ= 0,01 ppm (2x s, je 3H, SiMe); 0,80 (s, 3H, H-18); 0,88 (s, 9H, Si-t-Butyl); 1,21 (s, 6H, H-28 u. H-29); 3,42 (m, 2H, H-24); 3,88 (s, 2H, H-22); 4,01 (m, 1H, H-8); 4,92 u. 5,18 (2x s, je 1H, H-21 u. H-21')

**76. [1*S*-[1α,3aβ,4α,7aα]]-1-[2-[4-[[Dimethyl(1,1-dimethylethyl)silyl]oxy]-7a-methyloctahydro-1H-inden-1-yl]-2-propenoxy]-4-ethyl-4 -hexanol 77**

Man bereitet das Grignard-Reagenz aus 882 mg (8,1 mmol) Bromethan und 196 mg (8,1 mmol) Magnesiumspänen in 15 ml Tetrahydrofuran und setzt mit 1,15 g (2,7 mmol) 75 analog **4.** um und erhält 734 mg der Titelverbindung als farbloses Öl.
$^1$H-NMR ($CDCl_3$): δ= 0,01 ppm (2x s, je 3H, SiMe); 0,80 (s, 3H, H-18); 0,88 (t, J=7 Hz, 6H, H-30 u. H-31); 0,89 (s, 9H, Si-t-Butyl); 1,48 (q, J=7 Hz, 4H, H-28 u. H-29); 3,42 (m, 2H, H-24); 3,88 (s, 2H, H-22); 4,02 (m, 1H, H-8); 4,93 u. 5,20 (2x s, je 1H, H-21 u. H-21')

**77. [1*S*-[1α,3aβ,4α,7aα]]-1-[2-(4-Hydroxy-4-methylpentoxy)-1-methylenethyl]-7a-methyloctahydro-1H-inden-4-ol 78**

Man setzt 580 mg (1,37 mmol) 76 mit 4,41 ml HF/Pyridin (70%) in 30 ml Tetrahydrofuran analog **64.** um und erhält 156 mg der Titelverbindung als farbloses Öl.
$^1$H-NMR ($CDCl_3$) : δ= 0,78 ppm (s, 3H, H-18); 1,18 (s, 6H, H-28 u. H-29); 3,48 (m, 2H, H-24); 3,82 (s, 2H, H-22); 4,03 (m, 1H, H-8); 4,89 u. 5,15 (2x s, je 1H, H-21 u. H-21')

**78. [1*S*-[1α,3aβ,4α,7aα]]-1-[2-(4-Ethyl-4-hydroxyhexoxy)-1-methylenethyl]-7a-methyloctahydro-1H-inden-4-ol 79**

Man setzt 734 mg (1,62 mmol) 77 mit 5,22 ml HF/Pyridin (70%) in 35 ml Tetrahydrofuran analog **64.** um und erhält 200 mg der Titelverbindung als farbloses Öl.
$^1$H-NMR ($CDCl_3$): δ= 0,77 ppm (s, 3H, H-18); 0,78 (t, J=7 Hz, 6H, H-30 u. H-31); 1,40 (q, J=7 Hz, 4H, H-28 u. H-29); 3,34 (m, 2H, H-24); 3,70 (s, 2H, H-22); 4,03 (m, 1H, H-8); 4,88 u. 5,14 (2x s, je 1H, H-21 u. H-21')

**79. [1*S*-[1α,3aβ,7aα]]-1-[2-(4-Hydroxy-4-methylpentoxy)-1-methylenethyl]-7a-methyloctahydro-4H-inden-4-on 80**

Man setzt 150 mg (0,48 mmol) 78 mit 144 mg (0,67 mmol) Pyridiniumchlorochromat in 10 ml Methylenchlorid analog **29.** um und erhält 124 mg der Titelverbindung als farbloses Öl.
$^1$H-NMR ($CDCl_3$): δ= 0,58 ppm (s, 3H, H-18); 1,23 (s, 6H, H-28 u. H-29); 2,57 (dd, J=10,5, 7,5 Hz, 1H, H-14); 3,42 u. 3,50 (2x dt, J=9, 7 Hz, je 1H, H-24 u. H-24'); 3,90 u. 3,97 (2x d, J=12,5 Hz, je 1H H-22 u. H-22'); 5,02 u. 5,24 (2x s, je 1H, H-21 u. H-21')

**80. [1*S*-[1α,3aβ,7aα]]-1-[2-(4-Ethyl-4-hydroxyhexoxy)-1-methylenethyl]-7a-methyloctahydro-4H-inden-4-on 81**

Man setzt 200 mg (0,59 mmol) 79 mit 177 mg (0,83 mmol) Pyridiniumchlorochromat in 10 ml Methylenchlorid analog **30.** um und erhält 145 mg der Titelverbindung als farbloses Öl.
$^1$H-NMR ($CDCl_3$): δ= 0,58 ppm (s, 3H, H-18); 0,88 (t, J=7 Hz, 6H, H-30 u. H-31); 1,50 (q, J=7 Hz, 4H, H-28 u. H-29); 2,58 (dd, J=10,5, 7,5 Hz, 1H, H-14); 3,40 u. 3,48 (2x dt, J=9, 7 Hz, je 1H, H-24 u. H-24'); 3,89 u. 3,96 (d, J=12,5 Hz, je 1H, H-22 u. H-22'); 5,01 u. 5,23 (2x s, je 1H, H-21 u. H-21')

**81. [1*S*-[1α,3aβ,7aα]]-1-[2-[4-Methyl-4-[(trimethylsilyl)oxy]pentoxy]-1-methylenethyl]-7a-methyloctahydro-4H-inden-4-on 82**

Man setzt 120 mg (0,39 mmol) 80 mit 127 mg (1,17 mmol) Trimethylchlorsilan, 105 mg (1,52 mmol) Imidazol und 0,16 ml Pyridin in 10 ml Diethylether analog **33.** um und erhält 119 mg der Titelverbindung als farbloses Öl.
$^1$H-NMR ($CDCl_3$) δ= 0,10 ppm (s, 9H, $SiMe_3$); 0,58 (s, 3H, H-18); 1,12 (s, 6H, H-28 u. H-29); 2,57 (dd, J=10,5, 7,5 Hz, 1H, H-14); 3,38 u. 3,43 (2x dt, J=9, 7 Hz, je 1H, H-24 u. H-24'); 3,88 u. 3,96 (2x d, J=12,5 Hz, je 1H, H-22 u. H-22'); 5,01 u. 5,23 (2x s, je 1H, H-21 u. H-21')

**82. [1*S*-[1α,3aβ,7aα]]-1-[2-[4-Ethyl-4-[(trimethylsilyl)oxy]hexoxy]-1-methylenethyl]-7a-methyloctahydro-4H-inden-4-on 83**

Man setzt 140 mg (0,42 mmol) 81 mit 136 mg (1,26 mmol) Trimethylchlorsilan, 113 mg (1,64 mmol) Imidazol und 0,17 ml Pyridin in 10 ml Diethylether analog **33.** um und erhält 136 mg der Titelverbindung als farbloses Öl.
$^1$H-NMR ($CDCl_3$): δ= 0,01 ppm (s, 9H, MeSi); 0,49 (s, 3H, H-18); 0,71 (t, J=7 Hz, 6H, H-30 u. H-31); 1,39 (q, J=7 Hz, 4H, H-28 u. H-29); 2,47 (dd, J=10,5, 7,5 Hz, 1H, H-14); 3,26 u. 3,33 (2x dt, J=9, 7 Hz, je 1H, H-24 u. H-24'); 3,79 u. 3,87 (2x d, J=12,5 Hz, je 1H, H-22 U. H-22'); 4,91 u. 5,14 (2x s, je 1H, H-21 u. H-21')

**83. (7*E*)-(1*R*,3*R*)-1,3-Bis[[dimethyl(1,1-dimethylethyl)silyl]oxy]-24-[3-methyl-3-[(trimethylsilyl)oxy]butyl]-19-nor-23-oxa-9,10-secochola-5,7,20-trien 84**

Man setzt 110 mg (0,29 mmol) 82 analog **36.** um und erhält 125 mg der Titelverbindung als farblosen Schaum.
$^1$H-NMR ($CDCl_3$): δ= 0,01 u. 0,07 ppm (2x s, 12H u. 9H, SiMe); 0,41 (s, 3H, H-18); 0,79 u. 0,80 (2x s, Si-t-Butyl); 1,18 (s, 6H, H-28 u. H-29); 3,37 (m, 2H, H-24); 3,88 (s, 2H, H-22); 4,04 (m, 2H, H-1 u. H-3); 4,93 u. 5,15 (2x s, je 1H, H-21 u. H-21'); 5,79 u. 6,12 (d, J=11 Hz, je 1H, H-6 u. H-7)

**84. (7*E*)-(1*R*,3*R*)-1,3-Bis[[dimethyl(1,1-dimethylethyl)silyl]oxy]-24-[3-ethyl-3-[(trimethylsilyl)oxy]pentyl]-19-nor-23-oxa-9,10-secochola-5,7, 20-trien 85**

Man setzt 126 mg (0,31 mmol) 83 analog **36.** um und erhält 150 mg der Titelverbindung als farblosen Schaum.
$^1$H-NMR ($CDCl_3$): δ= 0,01 u. 0,05 ppm (2 s, 12H u. 9H, SiMe); 0,38 (s, 3H, H-18); 0,79 u. 0,80 (2x s, je 9H, Si-t-Butyl); 0,83 (t, J=7 Hz, 6H, H-30 u. H-31); 1,38 (q, J=7 Hz, 4H, H-28 u. H-29); 3,30 (m, 2H, H-24); 3,81 (s, 2H, H-22); 4,00 (m, 2H, H-1 u. H-3); 4,89 u. 5,10 (2x s, je 1H, H-21 u. H-21'); 5,74 u. 6,08 (2x d, J=11 Hz, je 1H, H-6 u. H-7)

**85. (7*E*)-(1*R*,3*R*)-24-(3-Hydroxy-3-methylbutyl)-19-nor-23-oxa-9,10-secochola-5,7,20-trien-1,3-diol 86**

Man setzt 110 mg (0,15 mmol) 84 mit 374 mg (1,2 mmol) Tetrabutylammoniumfluorid in 10 ml Tetrahydrofuran analog **39.** und erhält 53 mg der Titelverbindung als farblosen Schaum.
$^1$H-NMR ($CD_2Cl_2$): δ= 0,38 ppm (s, 3H, H-18); 1,12 (s, 6H, H-28 u. H-29); 3,33 (m, 2H, H-24); 3,81 (s, 2H, H-22); 3,91 u. 3,98 (2x m, je 1H, H-1 u. H.3); 4,90 u. 5,10 (2x s, je 1H, H-21 u. H-21'); 5,80 u. 6,21 (2x d, J=11 Hz, je 1H, H-6 u. H-7)

**86. (7*E*)-(1*R*,3*R*)-24-(3-Ethyl-3-hydroxypentyl)-19-nor-23-oxa-9,10-secochola-5,7,20-trien-1,3-diol 87**

Man setzt 132 mg (0,17 mmol) 85 mit 424 mg (1,36 mmol) Tetrabutylammoniumfluorid in 10 ml Tetrahydrofuran analog **39.** und erhält 59 mg der Titelverbindung als farblosen Schaum.
$^1$H-NMR ($CD_2Cl_2$): δ= 0,39 ppm (s, 3H, H-18); 0,77 (t, J=7 Hz, 6H, H-30 u. H-31); 1,38 (q, J=7 Hz, 4H, H-28 u. H-29); 3,32 (m, 2H, H-24); 3,80 (s, 2H, H-22); 3,91 u. 3,98 (2x m, je 1H, H-1 u. H-3); 4,89 u. 5,09 (2x s, je 1H, H-21 u. H-21') ; 5,80 u. 6,21 (2x d, J=11 Hz, je 1H, H-6 u. H-7)

**87. (5*Z*,7*E*)-(1*S*,3*R*)-1,3-Bis[[(1,1-dimethylethyl)diphenylsilyl]oxy]-20-formyl-9,10-secopregna-5,7,10(19),20-tetraen 88**

Man löst 2,8 g (3,36 mmol) 11 in 100 ml Methylenchlorid und gibt 11,6 g (133 mmol) Mangandioxid zu. Es wird 1 Std. bei Raumtemperatur nachgerührt und anschließend über Celite abgesaugt. Nach Entfernen des Lösungsmittels erhält man 2,5 g der Titelverbindung als farblosen Schaum.
$^1$H-NMR ($CDCl_3$): δ= 0,35 ppm (s, 3H, H-18); 0,92 u. 0,99 (2x s, je 9H, Si-t-Butyl); 4,23 (m, 1H, H-3); 4,55 (m, 1H, H-1); 4,83 u. 5,10 (2x s, je 1H, H-19 u. H-19'); 6,02 u. 6,09 (2x d, J=11 Hz, je 1H, H-6 u. H-7); 6,11 u. 6,32 (2x s, je 1H, H-21 u. H-21'); 7,23-7,69 (m, 20H, Si-Phenyl); 9,58 (s, 1H, H-22)

**88. (5*Z*,7*E*,22*E*)-(1*S*,3*R*)-1,3-Bis[[(1,1-dimethylethyl)diphenylsilyl]oxy]-9,10-secochola-5,7,10(19),20,22-pentaen-24-säuremethylester 89**

Man legt 182 mg (6 mmol) Natriumhydrid (80%) in 10 ml Tetrahydrofuran vor und tropft bei Raumtemperatur 825 mg (4,8 mmol) Dimethyl(methoxycarbonyl)methylphosphonat in 20 ml Tetrahydrofuran zu. Nach 30 Min. tropft man 1,2 g (1,5 mmol) 88 zu und rührt über Nacht nach. Unter Eiskühlung wird nun gesättigte Natriumchlorid-Lösung zugetropft, mit Essigester extrahiert, die organische Phase mit Natriumchlorid-Lösung gewaschen, über Natriumsulfat getrocknet und der Rückstand chromatographisch an Kieselgel mit Hexan/Essigester als Elutionsmittel gereinigt, wobei 470 mg der Titelverbindung als farbloser Schaum anfallen.

$^1$H-NMR ($CDCl_3$): δ= 0,39 ppm (s, 3H, H-18); 0,93 u. 0,99 (2x s, je 9H, Si-t-Butyl); 3,79 (s, 3H, COOMe); 4,25 (m, 1H, H-3); 4,57 (m, 1H, H-1); 4,83 u. 5,10 (2x s, je 1H, H-19 u. H-19'); 5,35 u. 5,55 (2x s, je 1H, H-21 u. H-21'); 6,05 (d, J=15,5 Hz, 1H, H-23); 6,09 u. 6,20 (2x d, J=11 Hz, je 1H, H-6 u. H-7); 7,24-7,69 (m, 20H, Si-Phenyl)

**89. (5*Z*,7*E*,22*E*)-(1*S*,3*R*)-1,3-Bis[[(1,1-dimethylethyl)diphenylsilyl]oxy]-9,10-secochola-5,7,10(19),20,22-pentaen-24-ol 90**

Man legt 470 mg (0,52 mmol) 89 in 60 ml Tetrahydrofuran vor und troft bei 0°C 2,16 ml DIBAH-Lösung (1.3 M in Toluol) zu. Man rührt 1 Std. bei 0°C, verdünnt dann mit Toluol, setzt 0,1 ml Isopropanol und 1,05 ml Wasser zu und rührt 30 Min. bei Raumtemperatur. Nach Filtration wird das Solvens entfernt und der Rückstand chromatographisch gereinigt, wobei 450 mg der Titelsubstanz als farbloser Schaum anfallen.
$^1$H-NMR ($CDCl_3$): δ= 0,40 ppm (s, 3H, H-18); 0,94 u. 0,99 (2x s, je 9H, Si-t-Butyl); 4,20 (m, 3H, H-3 u. H-24); 4,57 (t, J=5,5 Hz, 1H, H-1); 4,83 (s, 1H, H-19); 5,00 (s, 1H, H-21); 5,10 (s, 1H, H-19'); 5,22 (s, 1H, H-21'); 6,00 (dt, J=16, 5,5 Hz, 1H, H-23); 6,01 u. 6,09 (2x d, J=11 Hz, je 1H, H-6 u. H-7); 6,27 (d, J=16 Hz, 1H, H-22); 7,12-7,70 (m, 20H, Si-Phenyl)

**90. [(5*Z*,7*E*,22*E*)-(1*S*,3*R*)-1,3-Bis[[(1,1-dimethylethyl)diphenylsilyl]oxy]-9,10-secochola-5,7,10(19),20,22-pentaen-24-oxy]essigsäure-1,1-dimethylethylester 91**

Man setzt 450 mg (0,53 mmol) 90 mit 2,7 ml Natronlauge (25%), 11 mg Tetrabutylammoniumhydrogensulfat und 760 mg (3,9 mmol) Bromessigsäure-tert.-butylester analog **3.** um und erhält 340 mg der Titelverbindung als farblosen Schaum.
$^1$H-NMR ($CDCl_3$): δ= 0,40 ppm (s, 3H, H-18); 0,94 u. 1,12 (2x s, je 9H, Si-t-Butyl); 1,28 (s, 9H, t-Butylester); 3,98 (s, 2H, H-26); 4,15 (d, J=5,5 Hz, 2H, H-24); 4,25 (m, 1H, H-3); 4,56 (m, 1H, H-1); 4,84 (s, 1H, H-19); 5,01 (s, 1H, H-21); 5,10 (s, 1H, H-19'); 5,22 (s, 1H, H-21'); 5,90 (dt, J=15,5, 5,5 Hz, 1H, H-23); 6,28 (d, J=15,5 Hz, 1H, H-22); 6,03 u. 6,10 (2x d, J=11 Hz, je 1H, H-6 u. H-7); 7,24-7,70 (m, 20H, Si-Phenyl)

**91.**
**(5*Z*,7*E*,22*E*)-(1*S*,3*R*)-24-(2-Hydroxy-2-methylpropoxy)-9,10-secochola-5,7,10(19),20,22-pentaen-1,3-diol 92**

Man bereitet das Grignard-Reagenz aus 397 mg (2,8 mmol) Jodmethan und 69 mg (2,8 mmol) Magnesiumspänen in 5 ml Diethylether und setzt mit 340 mg (0,35 mmol) 91 analog **4.** um und erhält 210 mg Rohprodukt, das in 5 ml Tetrahydrofuran gelöst und mit 1 ml Tetrabutylammoniumfluorid-Lösung (1M in Tetrahydrofuran) analog **14.** umgesetzt wird. Es werden nach Aufreinigung 60 mg der Titelverbindung als farbloser Schaum isoliert.
$^1$H-NMR ($CD_2Cl_2$): δ= 0,42 ppm (s, 3H, H-18); 1,20 (s, 6H, H-28 u. H-29); 3,25 (s, 2H, H-26); 4,08 (d, J=5,5 Hz, 2H, H-24); 4,13 (m, 1H, H-3); 4,83 (m, 1H, H-1); 4,97 (s, 1H, H-19); 5,00 u. 5,20 (2x s, je 1H, H-21 u. H-21'); 5,30 (s, 1H, H-19'); 5,90 (dt, J=15,5, 5,5 Hz, 1H, H-23); 6,05 u. 6,35 (2x d, J=11 Hz, je 1H, H-6 u. H-7); 6,25 (d, J=15,5 Hz, 1H, H-22)

**92. (5*Z*,7*E*,22*E*)-(1*S*,3*R*)-24-(2-Ethyl-2-hydroxybutoxy)-9,10-secochola-5,7,10(19),20,22-pentaen-1,3-diol 93**

Man bereitet das Grignard-Reagenz aus 136 mg (1,25 mmol) Bromethan und 30 mg (1,25 mmol) Magnesium-Spänen in 5 ml Tetrahydrofuran und setzt mit 120 mg (0,12 mmol) 92 analog **4.** um und erhält 110 mg Rohprodukt, das in 5 ml Tetrahydrofuran gelöst und mit 1 ml Tetrabutylammoniumfluorid-Lösung (1M in Tetrahydrofuran) analog **14.** umgesetzt wird. Es werden nach Aufreinigung 21 mg der Titelverbindung als farbloser Schaum isoliert.
$^1$H-NMR ($CD_2Cl_2$): δ= 0,37 ppm (s, 3H, H-18); 0,78 (t, J=7 Hz, 6H, H-30 u. H-31); 1,40 (q, J=7 Hz, 4H, H-28 u. H-29); 3,20 (s, 2H, H-26); 3,97 (d, J=5,5 Hz, 2H, H-24); 4,10 (m, 1H, H-3); 4,31 (m, 1H, H-1); 4,89 (s, 1H, H-19); 4,91 u. 5,12 (2x s, je 1H, H-21 u. H-21'); 5,23 (s, 1H, H-19'); 5,81 (dt, J=15,5, 5,5 Hz, 1H, H-24); 5,98 u. 6,30 (2x d, J=11 Hz, je 1H, H-6 u. H-7); 6,18 (d, J=15,5 Hz, 1H, H-22)

**Patentansprüche**

1. In der 20-Position modifizierte Vitamin D-Derivate der allgemeinen Formel I

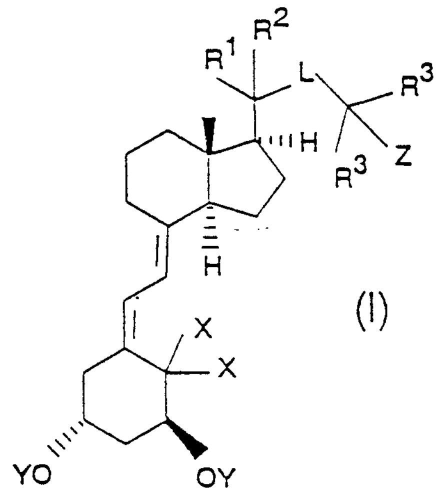

(I)

worin Y je ein Wasserstoffatom oder je eine Alkanoylgruppe mit 1 bis 9 Kohlenstoffatomen, oder eine Aroylgruppe, Z ein Wasserstoffatom, eine Hydroxylgruppe oder eine Alkanoyloxygruppe mit 1 bis 9 Kohlenstoffatomen,
X je ein Wasserstoffatom oder beide X gemeinsam eine exocyclische Methylengruppe,
$R^1$ und $R^2$ unabhängig voneinander je eine Alkylgruppe mit 1 bis 4 Kohlenstoffatomen, gemeinsam eine Methylengruppe oder gemeinsam mit dem quartären Kohlenstoffatom 20 eine Cyclopropyleinheit, wobei wenn beide X eine Methylengruppe bedeuten, $R^1$ und $R^2$ nicht Methyl sind,
$R^3$ je ein Wasserstoffatom oder je eine lineare oder verzweigte Alkylgruppe mit 1 bis 5 Kohlenstoffatomen, je eine Trifluormethylgruppe oder einen gemeinsam mit dem tertiären Kohlenstoffatom 25 gebildeten gesättigten oder ungesättigten, carbocyclischen oder heterocyclischen 3-, 4-, 5- oder 6-gliedrigen Ring,
L entweder die Gruppierung

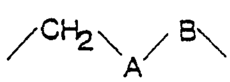

,

wobei
A eine Methylengruppe oder ein Sauerstoff-, Schwefel-, oder ein hydrid- oder $C_1$-$C_4$-alkylsubstituiertes Stickstoffatom, und
B eine Alkylengruppe $-(CH_2)_n-$, worin n = 1, 2, 3, 4, 5 oder 6 ist und eine beliebige Methylengruppe durch ein Sauerstoffatom ersetzt sein kann, darstellen oder
L die Gruppierung

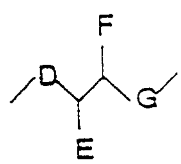

,

wobei
D eine direkte Bindung, eine Methylenbrücke oder eine (E)-1,2-Ethendiylbrücke zwischen den Kohlenstoffatomen 20 und 22,
E und F jeweils ein Wasserstoffatom, oder gemeinsam eine E-Doppelbindung und
G eine direkte Bindung oder eine Alkylengruppe $-(CH_2)_n-$, worin n = 1, 2, 3, 4, 5 oder 6 ist und eine beliebige Methylengruppe durch ein Sauerstoffatom ersetzt sowie jede Methylengruppe durch eine Hydroxylgruppe oder ein Fluor-, Chlor- oder Bromatom substituent sein kann, darstellen, bedeuten.

2. Vitamin D-Derivate nach Anspruch 1, dadurch gekennzeichnet, daß Y Wasserstoffatome oder Alkanoylreste gesättigter Alkancarbonsäuren sind und Z eine Hydroxylgruppe oder ein Alkanoyloxyrest gesättigter Alkancarbonsäuren oder der Benzoyloxyrest ist.

3. Vitamin D-Derivate nach Anspruch 1, dadurch gekennzeichnet, daß für $R^3$ die Methyl-, Ethyl- oder Propylgruppe oder ein gemeinsam mit dem tertiären Kohlenstoffatom 25 gebildeter Cyclopropyl- oder Cyclopentylring stehen.

4. Vitamin D-Derivate nach Anspruch 1, dadurch gekennzeichnet, daß beide Y je für ein Wasserstoffatom, Z für eine Hydroxylgruppe und $R^3$ je für eine Methyl-, Ethyl- oder Propylgruppe stehen.

5. Vitamin D-Derivate nach Anspruch 1, dadurch gekennzeichnet, daß $R^1$ und $R^2$ je eine Alkylgruppe und beide X je ein Wasserstoffatom bedeuten.

6. Vitamin D-Derivate nach Anspruch 1, dadurch gekennzeichnet, daß $R^1$ und $R^2$ gemeinsam eine Methylengruppe und beide X je ein Wasserstoffatom bedeuten.

7. Vitamin D-Derivate nach Anspruch 1, dadurch gekennzeichnet, daß $R^1$ und $R^2$ gemeinsam eine Methylengruppe und beide X gemeinsam eine Methylengruppe bedeuten.

8. Vitamin D-Derivate nach Anspruch 1, dadurch gekennzeichnet, daß $R^1$ und $R^2$ zusammen mit dem Kohlenstoffatom 20 einen Cyclopropylring und beide X gemeinsam eine Methylengruppe bedeuten.

9. Vitamin D-Derivate nach Anspruch 1, dadurch gekennzeichnet, daß $R^1$ und $R^2$ zusammen mit dem Kohlenstoffatom 20 einen Cyclopropylring und beide X je ein Wasserstoffatom bedeuten.

**10.** Vitamin D-Derivate nach Anspruch 1, nämlich

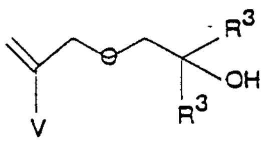

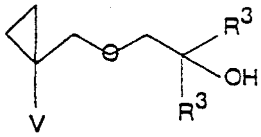

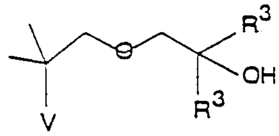

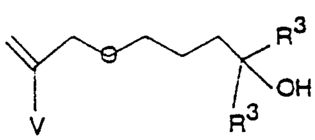

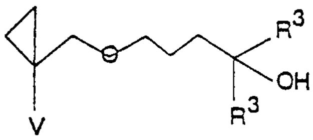

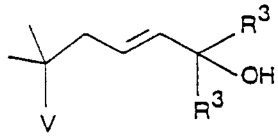

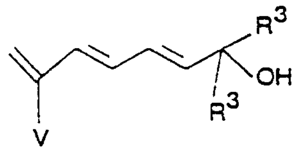

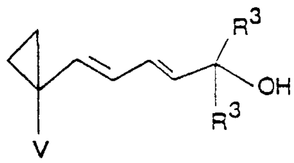

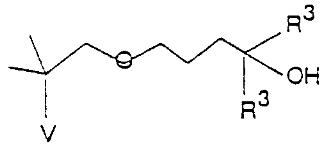

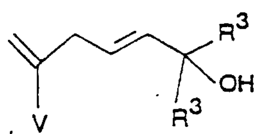

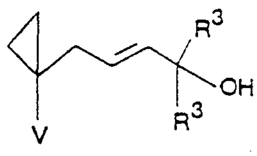

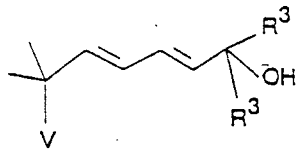

V =

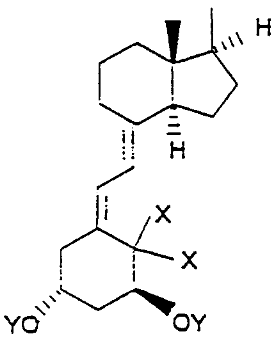

X,X = H,H

X,X = $CH_2$

V =

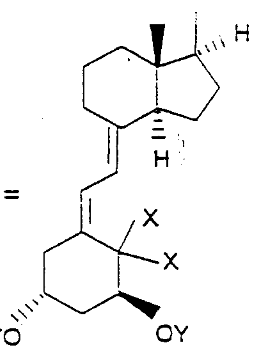

X,X = H,H

**11.** Verbindungen nach Anspruch 1,
(5Z,7E)-(1S,3R)-26,27-Dimethyl-23-oxa-9,10-secocholesta-5,7,10(19),20-tetraen-1,3,25-triol,
(5Z,7E)-(1S,3R)-26,27-Diethyl-23-oxa-9,10-secocholesta-5,7,10(19),20-tetraen-1,3,25-triol,
(7E)-(1R,3R)-20,26,27-Trimethyl-23-oxa-19-nor-9,10-secocholesta-5,7-dien-1,3,25-triol,
(5Z,7E)-(1S,3R)-26,27-Dimethyl-20,21-methylen-23-oxa-9,10-secocholesta-5,7,10(19)-trien-1,3,25-triol,
(5Z,7E)-(1S,3R)-23-Oxa-9,10-secocholesta-5,7,10(19),20-tetraen-1,3,25-triol,
(5Z,7E)-(1S,3R)-24-(3-Hydroxy-3-methylbutyl)-23-oxa-9,10-secochola-5,7,10(19),20-tetraen-1,3-diol,
(5Z,7E)-(1S,3R)-24-(3-Ethyl-3-hydroxypentyl)-23-oxa-9,10-secochola-5,7,10(19),20-tetraen-1,3-diol,
(5Z,7E)-(1S,3R)-26,27-Diethyl-20,21-methylen-23-oxa-9,10-secocholesta-5,7,10(19)-trien-1,3,25-triol,
(7E)-(1R,3R)-23-Oxa-19-nor-9,10-secocholesta-5,7,20-trien-1,3,25-triol,
(5Z,7E)-(1S,3R)-20,21-Methylen-23-oxa-9,10-secocholesta-5,7,10(19)-trien-1,3,25-triol,
(5Z,7E)-(1S,3R)-24-(3-Hydroxy-3-methylbutyl)-20,21-methylen-23-oxa-9,10-secochola-5,7,10(19)-trien-1,3-diol,

(5Z,7E)-(1S,3R)-24-(3-Ethyl-3-hydroxypentyl)-20,21-methylen-23-oxa-9,10-secochola-5,7,10(19)-trien-1,3-diol,
(7E)-(1R,3R)-20-Methyl-19-nor-23-oxa-9,10-secocholesta-5,7-dien-1,3,25-triol,
(7E)-(1R,3R)-26,27-Diethyl-20-methyl-19-nor-23-oxa-9,10-secocholesta-5,7-dien-1,3,25-triol,
(7E)-(1R,3R)-24-(3-Hydroxy-3-methylbutyl)-20-methyl-19-nor-23-oxa-9,10-secochola-5,7-dien-1,3-diol,
(7E)-(1R,3R)-24-(3-Ethyl-3-hydroxypentyl)-20-methyl-19-nor-23-oxa-9,10-secochola-5,7-dien-1,3-diol,
(7E)-(1R,3R)-26,27-Dimethyl-19-nor-23-oxa-9,10-secocholesta-5,7,20-trien-1,3,25-triol,
(7E)-(1R,3R)-26,27-Diethyl-19-nor-23-oxa-9,10-secocholesta-5,7,20-trien-1,3,25-triol,
(7E)-(1R,3R)-24-(3-Hydroxy-3-methylbutyl)-19-nor-23-oxa-9,10-secochola-5,7,20-trien-1,3-diol,
(7E)-(1R,3R)-24-(3-Ethyl-3-hydroxypentyl)-19-nor-23-oxa-9,10-secochola-5,7,20-trien-1,3-diol,
(5Z,7E,22E)-(1S,3R)-24-(2-Hydroxy-2-methylpropoxy)-9,10-secochola-5,7,10(19),20,22-pentaen-1,3-diol,
(5Z,7E,22E)-(1S,3R)-24-(2-Ethyl-2-hydroxybutoxy)-9,10-secochola-5,7,10(19),20,22-pentaen-1,3-diol,
(7E,22E)-(1R,3R)-24-(2-Hydroxy-2-methylpropoxy)19-nor-9,10-secochola-5,7,20,22-tetraen-1,3-diol,
(7E,22E)-(1R,3R)-24-(2-Ethyl-2-hydroxybutoxy)-19-nor-9,10-secochola-5,7,20,22-tetraen-1,3-diol.

12. Verfahren zu Herstellung der Vitamin D-Derivate der allgemeinen Formel I gemäß Anspruch 1, dadurch gekennzeichnet, daß eine Verbindung der allgemeinen Formeln XIV, XVIII, XXVIII, XXXV, XLI oder IL

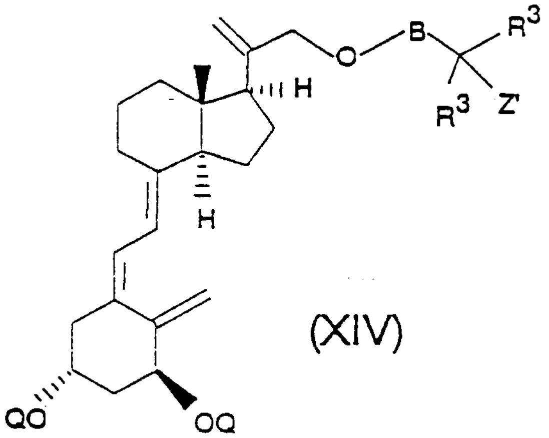

(XIV)

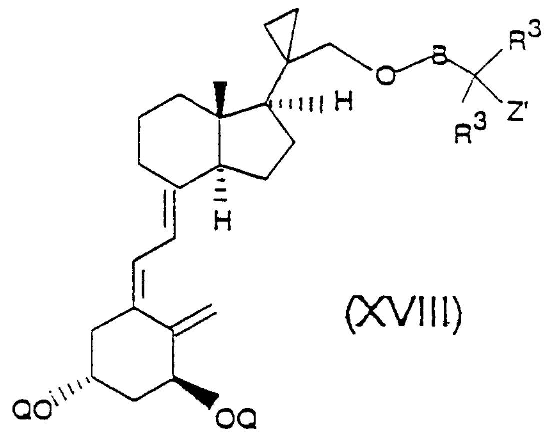

(XVIII)

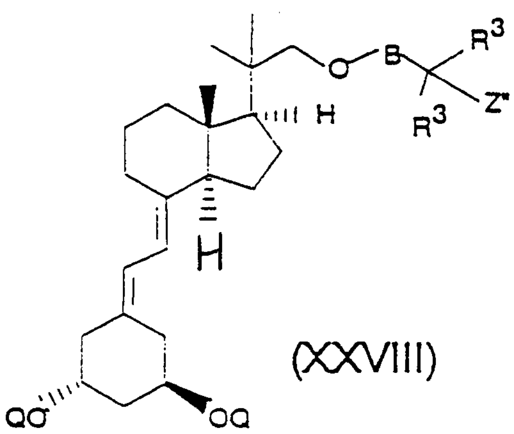

(XXVIII)

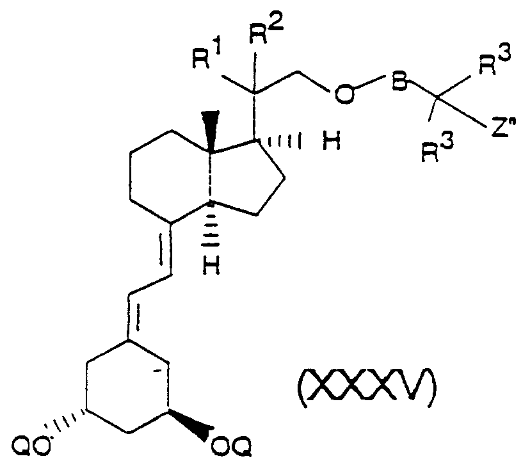

(XXXV)

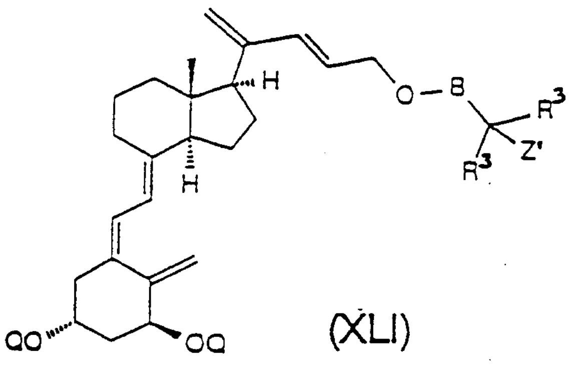

(XLI)

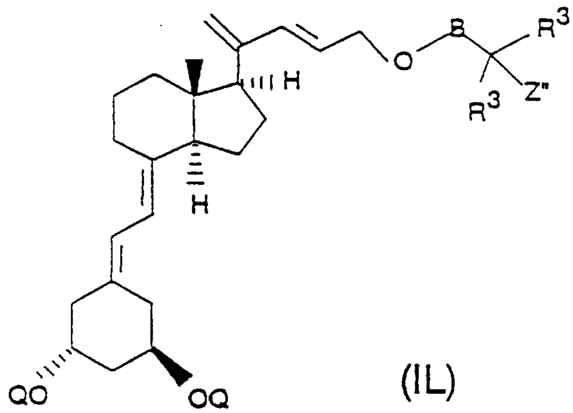

(IL)

worin B, $R^1$, $R^2$ und $R^3$ die in Anspruch 1 angegebene Bedeutung haben Q = alkyl- oder aryl- oder alkyl-/aryl-substituierte Silylgruppe, Z' = Hydroxygruppe; Z" = Siloxygruppe, durch Abspaltung der Schutzgruppen Q und Z" falls anwesend in die entsprechende freie Hydroxyverbindung überführt sowie diese gewünschtenfalls partiell oder vollständig mit einen Alkancarbonsäurechlorid, -bromid oder - anhydrid, welches im Alkanoylrest 1 bis 9 Kohlenstoffatome aufweist, oder Stelle 1 und 3 mit Benzoylchlorid verestert wird.

**13.** Verfahren nach Anspruch 12, dadurch gekennzeichnet, daß die Schutzgruppen mit Tetra-n-butylammoniumfluorid abgespalten werden.

**14.** Zwischenprodukte der allgemeinen Formeln IX und XV

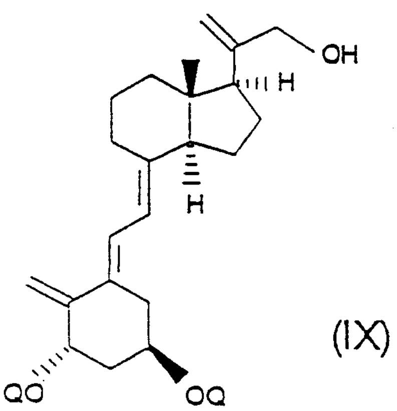

(IX)

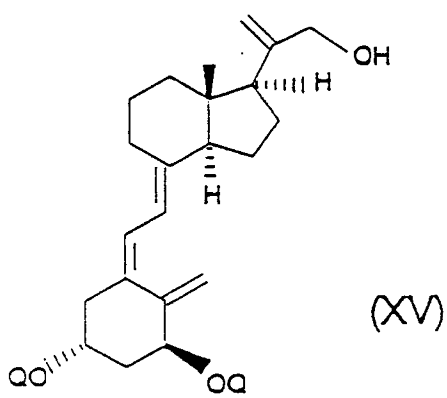

(XV)

worin Q eine alkyl- oder aryl- oder alkyl-/aryl-substituierte Silylgruppe darstellt.

**15.** Verbindungen nach Anspruch 14
(5E,7E)-(1S,3R)-1,3-Bis[[(1,1-dimethylethyl)diphenylsilyl]oxy]-20-methylen-9,10-secopregna-5,7,10(19)-trien-21-ol,
(5Z,7E)-(1S,3R)-1,3-Bis[[(1,1-dimethylethyl)diphenylsilyl]oxy]-20-methylen-9,10-secopregna-5,7,10(19)-trien-21-ol.

**16.** Pharmazeutische Präparate enthaltend mindestens eine Verbindung der allgemeinen Formel I sowie einen pharmazeutisch verträglichen Träger.

**17.** Verwendung der Verbindungen der allgemeinen Formel I zur Herstellung von Arzneimitteln.

## Claims

1. Vitamin D derivatives, modified in the 20-position, of the general formula I

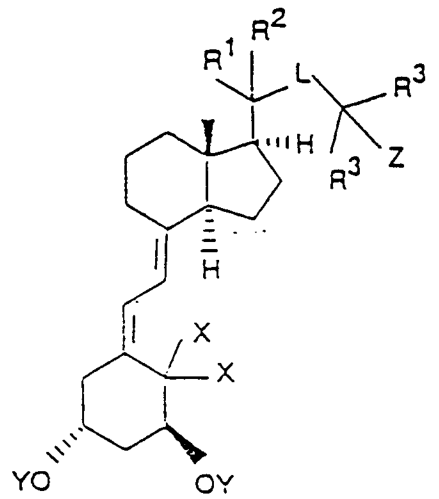

(I)

in which
each of the substituents Y represents a hydrogen atom or an alkanoyl group having from 1 to 9 carbon atoms, or an aroyl group;
Z represents a hydrogen atom, a hydroxy group or an alkanoyloxy group having from 1 to 9 carbon atoms;
each of the substituents X represents a hydrogen atom, or the two substituents X together represent an exocyclic methylene group;
$R^1$ and $R^2$ each independently of the other represents an alkyl group having from 1 to 4 carbon atoms, or $R^1$ and $R^2$ together represent a methylene group or, together with the quaternary carbon atom 20, represent a cyclopropyl unit, $R^1$ and $R^2$ not being methyl when the two substituents X represent a methylene group;
each of the substituents $R^3$ represents a hydrogen atom or a linear or branched alkyl group having from 1 to 5 carbon atoms, a trifluoromethyl group or a saturated or unsaturated, carbocyclic or heterocyclic 3-, 4-, 5- or 6-membered ring formed together with the tertiary carbon atom 25;
either L represents the grouping

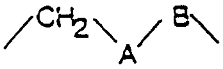
,

wherein
A represents a methylene group or an oxygen atom, a sulphur atom or a hydride- or $C_1$-$C_4$alkyl-substituted nitrogen atom, and
B represents an alkylene group $-(CH_2)_n-$, wherein n is 1, 2, 3, 4, 5 or 6 and any methylene group can be replaced by an oxygen atom; or
L represents the grouping

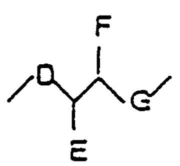
,

wherein
D represents a direct bond, a methylene bridge or an (E)-1,2-ethenediyl bridge between carbon atoms 20 and 22,
E and F each represent a hydrogen atom or together represent an (E) double bond, and
G represents a direct bond or an alkylene group $-(CH_2)_n-$, wherein n is 1, 2, 3, 4, 5 or 6 and any methylene group

can be replaced by an oxygen atom and each methylene group can be substituted by a hydroxy group or by a fluorine, chlorine or bromine atom.

2. Vitamin D derivatives according to claim 1, characterised in that the substituents Y are hydrogen atoms or alkanoyl radicals of saturated alkanecarboxylic acids and Z is a hydroxy group or an alkanoyloxy radical of saturated alkanecarboxylic acids or is the benzoyloxy radical.

3. Vitamin D derivatives according to claim 1, characterised in that the substituents $R^3$ represent the methyl, ethyl or propyl group or a cyclopropyl or cyclopentyl ring formed together with the tertiary carbon atom 25.

4. Vitamin D derivatives according to claim 1, characterised in that the two substituents Y each represent a hydrogen atom, Z represents a hydroxy group and the substituents $R^3$ each represent a methyl, ethyl or propyl group.

5. Vitamin D derivatives according to claim 1, characterised in that $R^1$ and $R^2$ each represent an alkyl group and the two substituents X each represent a hydrogen atom.

6. Vitamin D derivatives according to claim 1, characterised in that $R^1$ and $R^2$ together represent a methylene group and the two substituents X each represent a hydrogen atom.

7. Vitamin D derivatives according to claim 1, characterised in that $R^1$ and $R^2$ together represent a methylene group and the two substituents X together represent a methylene group.

8. Vitamin D derivatives according to claim 1, characterised in that $R^1$ and $R^2$, together with the carbon atom 20, represent a cyclopropyl ring and the two substituents X together represent a methylene group.

9. Vitamin D derivatives according to claim 1, characterised in that $R^1$ and $R^2$, together with the carbon atom 20, represent a cyclopropyl ring and the two substituents X each represent a hydrogen atom.

**10.** Vitamin D derivatives according to claim 1, namely

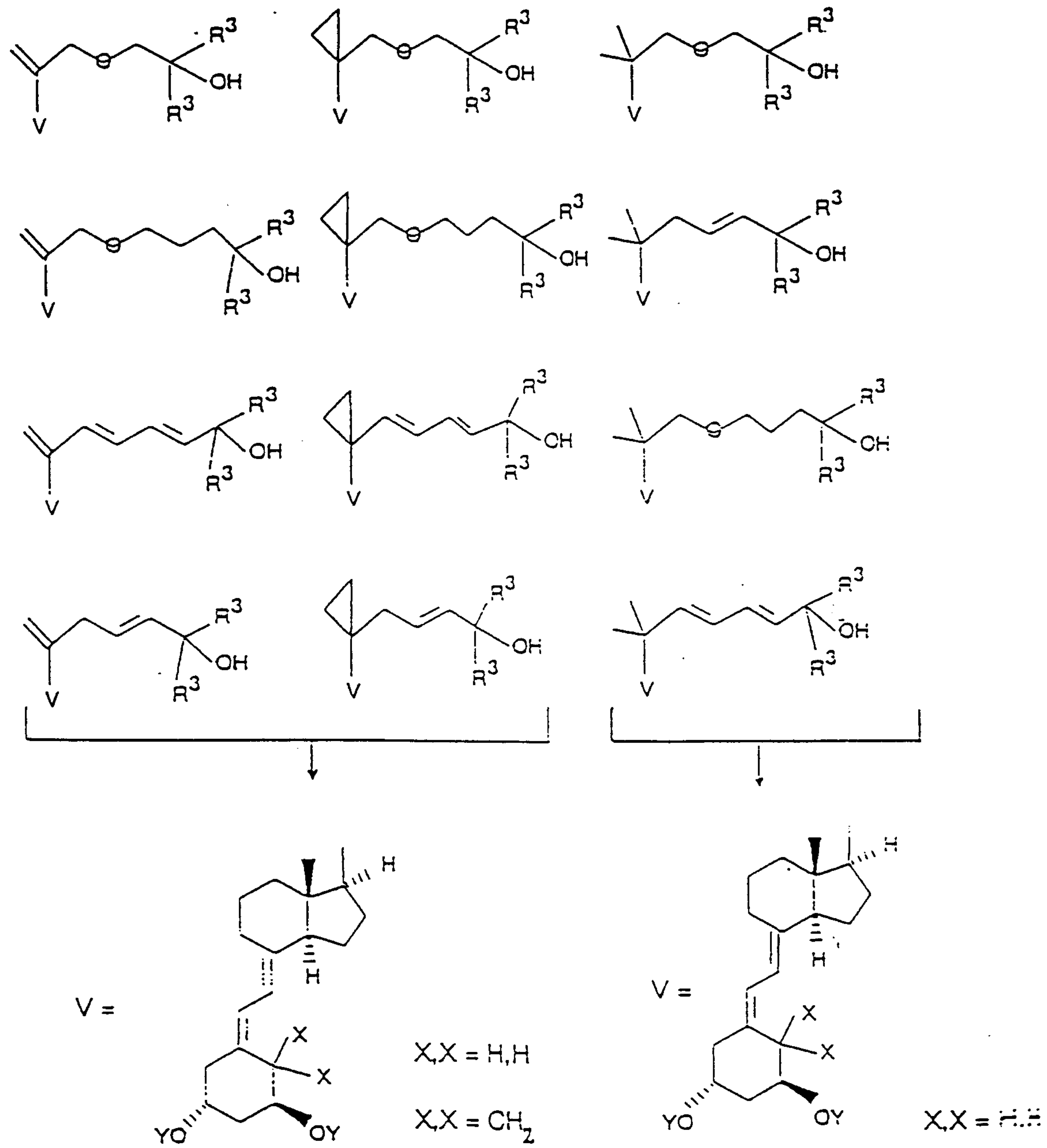

V =

X,X = H,H

X,X = $CH_2$

V =

X,X = H,H

**11.** Compounds according to claim 1,
(5Z,7E)-(1S,3R)-26,27-dimethyl-23-oxa-9,10-secocholesta-5,7,10(19),20-tetraene-1,3,25-triol,
(5Z,7E)-(1S,3R)-26,27-diethyl-23-oxa-9,10-secocholesta-5,7,10(19),20-tetraene-1,3,25-triol,
(7E)-(1R,3R)-20,26,27-trimethyl-23-oxa-19-nor-9,10-secocholesta-5,7-diene-1,3,25-triol,
(5Z,7E)-(1S,3R)-26,27-dimethyl-20,21-methylene-23-oxa-9,10-secocholesta-5,7,10(19)-triene-1,3,25-triol,
(5Z,7E)-(1S,3R)-23-oxa-9,10-secocholesta-5,7,10(19),20-tetraene-1,3,25-triol,
(5Z,7E)-(1S,3R)-24-(3-hydroxy-3-methylbutyl)-23-oxa-9,10-secochola-5,7,10(19),20-tetraene-1,3-diol,
(5Z,7E)-(1S,3R)-24-(3-ethyl-3-hydroxypentyl)-23-oxa-9,10-secochola-5,7,10(19),20-tetraene-1,3-diol,
(5Z,7E)-(1S,3R)-26,27-diethyl-20,21-methylene-23-oxa-9,10-secocholesta-5,7,10(19)-triene-1,3,25-triol,
(7E)-(1R,3R)-23-oxa-19-nor-9,10-secocholesta-5,7,20-triene-1,3,25-triol,
(5Z,7E)-(1S,3R)-20,21-methylene-23-oxa-9,10-secocholesta-5,7,10(19)-triene-1,3,25-triol,
(5Z,7E)-(1S,3R)-24-(3-hydroxy-3-methylbutyl)-20,21-methylene-23-oxa-9,10-secochola-5,7,10(19)-triene-1,3-diol,

(5Z,7E)-(1S,3R)-24-(3-ethyl-3-hydroxypentyl)-20,21-methylene-23-oxa-9,10-secochola-5,7,10(19)-triene-1,3-diol,
(7E)-(1R,3R)-20-methyl-19-nor-23-oxa-9,10-secocholesta-5,7-diene-1,3,25-triol,
(7E)-(1R,3R)-26,27-diethyl-20-methy1-19-nor-23-oxa-9,10-secocholesta-5,7-diene-1,3,25-triol,
(7E)-(1R,3R)-24-(3-hydroxy-3-methylbutyl)-20-methyl-19-nor-23-oxa-9,10-secochola-5,7-diene-1,3-diol,
(7E)-(1R,3R)-24-(3-ethyl-3-hydroxypentyl)-20-methyl-19-nor-23-oxa-9,10-secochola-5,7-diene-1,3-diol,
(7E)-(1R,3R)-26,27-dimethyl-19-nor-23-oxa-9,10-secocholesta-5,7,20-triene-1,3,25-triol,
(7E)-(1R,3R)-26,27-diethyl-19-nor-23-oxa-9,10-secocholesta-5,7,20-triene-1,3,25-triol,
(7E)-(1R,3R)-24-(3-hydroxy-3-methylbutyl)-19-nor-23-oxa-9,10-secochola-5,7,20-triene-1,3-diol,
(7E)-(1R,3R)-24-(3-ethyl-3-hydroxypentyl)-19-nor-23-oxa-9,10-secochola-5,7,20-triene-1,3-diol,
(5Z,7E,22E)-(1S,3R)-24-(2-hydroxy-2-methylpropoxy)-9,10-secochola-5,7,10(19),20,22-pentaene-1,3-diol,
(5Z,7E,22E)-(1S,3R)-24-(2-ethyl-2-hydroxybutoxy)-9,10-secochola-5,7,10(19),20,22-pentaene-1,3-diol,
(7E,22E)-(1R,3R)-24-(2-hydroxy-2-methylpropoxy)-19-nor-9,10-secochola-5,7,20,22-tetraene-1,3-diol,
(7E,22E)-(1R,3R)-24-(2-ethyl-2-hydroxybutoxy)-19-nor-9,10-secochola-5,7,20,22-tetraene-1,3-diol.

**12.** Process for the preparation of vitamin D derivatives of the general formula I according to claim 1, characterised in that a compound of the general formula XIV, XVIII, XXVIII, XXXV, XLI or IL

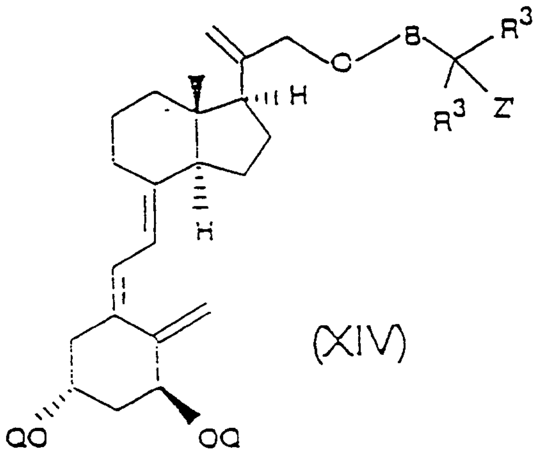

(XIV)

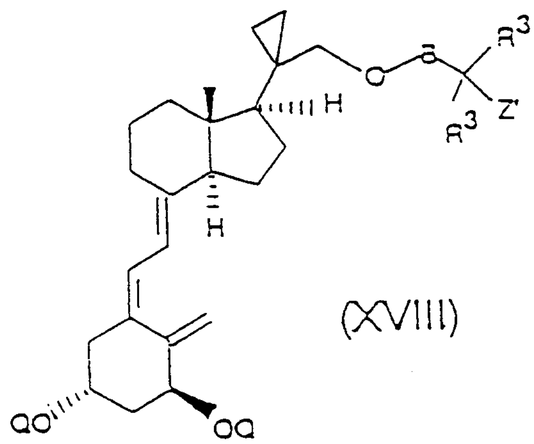

(XVIII)

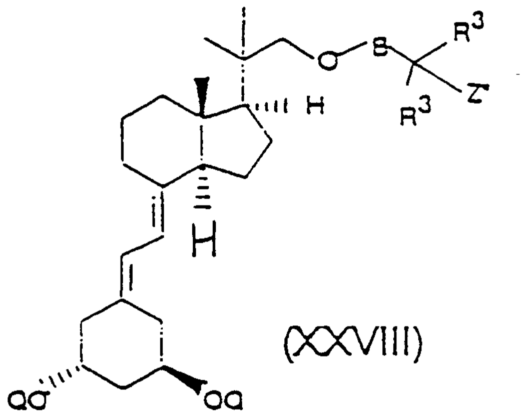

(XXVIII)

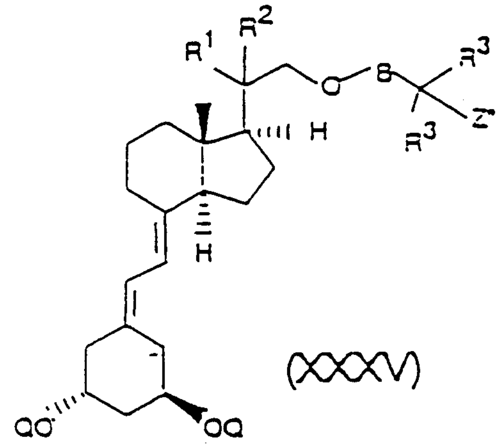

(XXXV)

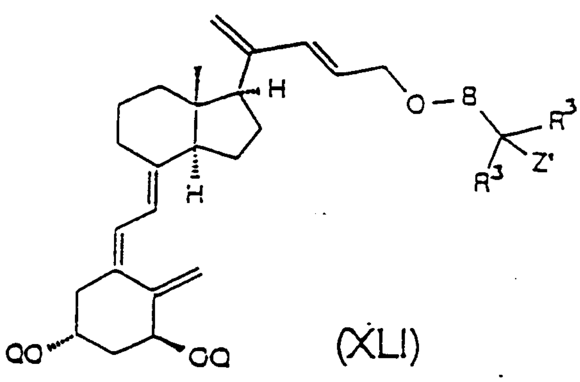

(XLI)

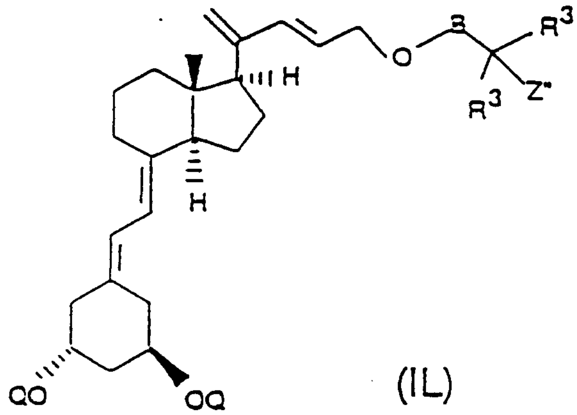

(IL)

in which B, $R^1$, $R^2$ and $R^3$ are as defined in claim 1, Q = alkyl- or aryl- or alkyl-/aryl-substituted silyl group, Z' = hydroxy group, Z" = siloxy group, is converted into the corresponding free hydroxy compound by removal of the protecting groups Q and Z", if present, and the free hydroxy compound is, if desired, esterified partially or completely by an alkanecarboxylic acid chloride, bromide or anhydride containing from 1 to 9 carbon atoms in the alkanoyl moiety, or at positions 1 and 3 by benzoyl chloride.

**13.** Process according to claim 12, characterised in that the protecting groups are removed with tetra-n-butylammonium fluoride.

**14.** Intermediates of the general formulae IX and XV

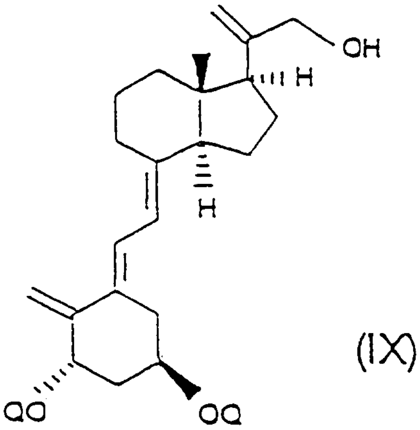

(IX)

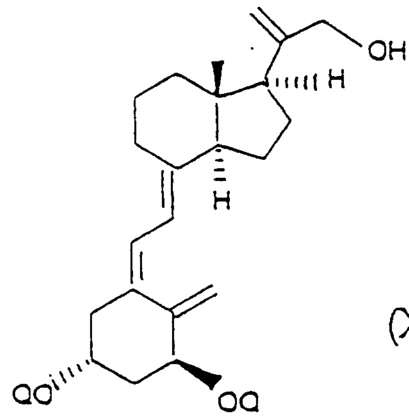

(XV)

in which Q represents an alkyl- or aryl- or alkyl-/aryl-substituted silyl group.

**15.** Compounds according to claim 14,
(5E,7E)-(1S,3R)-1,3-bis[[(1,1-dimethylethyl)diphenylsilyl]oxy]-20-methylene-9,10-secopregna-5,7,10(19)-trien-21-ol,
(5Z,7E)-(1S,3R)-1,3-bis[[(1,1-dimethylethyl)diphenylsilyl]oxy]-20-methylene-9,10-secopregna-5,7,10(19)-trien-21-ol.

**16.** Pharmaceutical preparations comprising at least one compound of the general formula I and a pharmaceutically acceptable carrier.

**17.** Use of compounds of the general formula I in the preparation of medicaments.

**Revendications**

**1.** Dérivés de la vitamine D modifiés en position 20 de formule générale I :

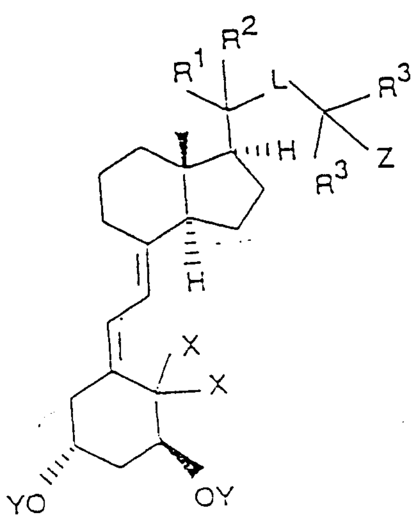

(I)

dans laquelle Y sont chacun un atome d'hydrogène ou un groupe alcanoyle avec 1 à 9 atomes de carbone ou un groupe aroyle,
Z est un atome d'hydrogène, un groupe hydroxyle ou un groupe alcanoyle avec 1 à 9 atomes de carbone,
X est un atome d'hydrogène ou les deux X ensemble forment un groupe méthylène exocyclique,

$R^1$ et $R^2$, indépendamment l'un de l'autre, sont chacun un groupe alkyle avec 1 à 4 atomes de carbone, ensemble avec le groupe méthylène ou ensemble avec l'atome de carbone 20 quaternaire forment un motif cyclopropyle, les deux X représentant un groupe méthylène, $R^1$ et $R^2$ n'étant pas méthyle,
les radicaux $R^3$ sont chacun un atome d'hydrogène ou un groupe alkyle linéaire ou ramifié avec 1 à 5 atomes de carbone, chacun est un groupe trifluorométhyle ou ensemble avec l'atome de carbone 25 tertiaire forment un cycle saturé ou insaturé, carbocyclique ou hétérocyclique à 3, 4, 5 ou 6 chaînons
L est soit le groupement

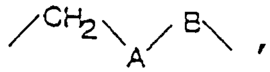

A étant un groupe méthylène ou un atome d'oxygène, de soufre, ou un hydrure soit un atome d'azote substitué par alkyle en $C_1$-$C_4$,
B est un groupe alkylène $-(CH_2)_n-$, dans lequel n = 1, 2, 3, 4, 5 ou 6 et n'importe quel groupe méthylène peut être remplacé par un atome d'oxygène, ou
L représente le groupement

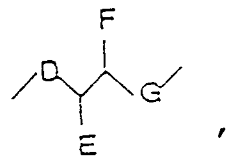

D étant une liaison directe, un pont méthylène ou E un pont 1,2-éthènediyle entre les atomes de carbone 20 et 22, E et F chacun représentent un atome d'hydrogène, ou ensemble une double liaison, et
G est une liaison directe ou un groupe alkylène $-(CH_2)_n-$, dans lequel n = 1, 2, 3, 4, 5 ou 6 et n'importe quel groupe méthylène étant remplacé par un atome d'oxygène ainsi que chaque groupe méthylène peut être substitué par un groupe hydroxyle ou un atome de fluor, de chlore ou de brome.

2. Dérivés de vitamine D selon la revendication 1, caractérisés en ce que Y représente des atomes d'hydrogène ou des radicaux alcanoyle d'acides alcanecarboxyliques saturés et Z un groupe hydroxyle ou un radical alcanoyle d'acides alcanecarboxyliques saturés ou le radical benzoyloxy.

3. Dérivés de vitamine D selon la revendication 1, caractérisés en ce que $R^3$ représente le groupe méthyle, éthyle ou propyle ou représente, ensemble avec l'atome de carbone tertiaire 25, un cycle cyclopropyle ou cyclopentyle.

4. Dérivés de vitamine D selon la revendication 1, caractérisés en ce que les deux Y chacun représentent un atome d'hydrogène, Z un groupe hydroxyle et $R^3$ représente un groupe méthyle, éthyle ou propyle.

5. Dérivés de vitamine D selon la revendication 1 caractérisés en ce que $R^1$ et $R^2$ chacun représentent un groupe alkyle et les deux X chacun représentent un atome d'hydrogène.

6. Dérivés de vitamine D selon la revendication 1, caractérisés en ce que $R^1$ et $R^2$ ensemble forment un groupe méthylène et les deux X chacun représentent un atome d'hydrogène.

7. Dérivés de vitamine D selon la revendication 1, caractérisés en ce que $R^1$ et $R^2$ ensemble forment un groupe méthylène et les deux X ensemble représentent un groupe méthylène.

8. Dérivés de vitamine D selon la revendication 1, caractérisés en ce que $R^1$ et $R^2$ ensemble avec l'atome de carbone 20 forment un cycle cyclopropyle et les deux X ensemble représentent un groupe méthylène.

9. Dérivés de vitamine D selon la revendication 1, caractérisés en ce que $R^1$ et $R^2$ ensemble avec l'atome de carbone 20 forment un cycle cyclopropyle et les deux X chacun représentent un atome d'hydrogène.

**10.** Dérivés de vitamine D selon la revendication 1, à savoir

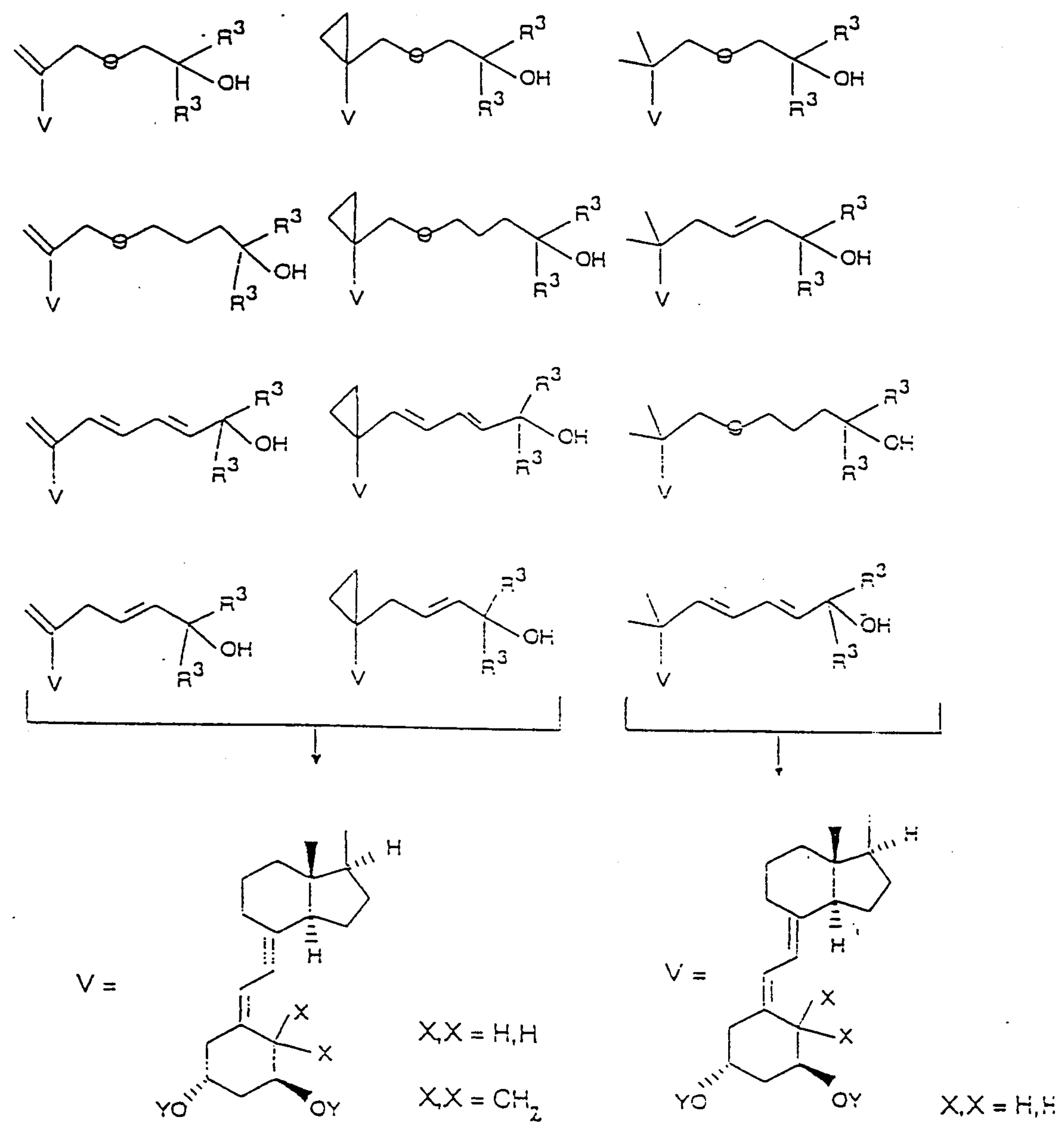


**11.** Composés selon la revendication 1
(5Z,7E)-(1S,3R)-26,27-diméthyl-23-oxa-9,10-sécocholesta-5,7,10(19),20-tétraène-1,3,25-triol,
(5Z,7E)-(1S,3R)-26,27-diéthyl-23-oxa-9,10-sécocholesta-5,7,10(19),20-tétraène-1,3,25-triol,
(7E)-(1R,3R)-20,26,27-triméthyl-23-oxa-19-nor-9,10-sécocholesta-5,7-diène-1,3,25-triol,
(5Z,7E)-(1S,3R)-26,27-diméthyl-20,21-méthylène-23-oxa-9,10-sécocholesta-5,7,10(19),20-triène-1,3,25-triol,
(5Z,7E)-(1S,3R)-23-oxa-9,10-sécocholesta-5,7,10(19),20-tétraène-1,3,25-triol,
(5Z,7E)-(1S,3R)-24-(3-hydroxy-3-méthylbutyl)-23-oxa-9,10-sécochola-5,7,10(19),20-tétraène-1,3-diol,
(5Z,7E)-(1S,3R)-24-(3-éthyl-3-hydroxypentyl)-23-oxa-9,10-sécochola-5,7,10(19),20-tétraène-1,3-diol,
(5Z,7E)-(1S,3R)-26,27-diéthyl-20,21-méthylène-23-oxa-9,10-sécocholesta-5,7,10(19)-triène-1,3,25-triol,
(7E)-(1R,3R)-23-oxa-19-nor-9,10-sécocholesta-5,7,20-triène-1,3,25-triol,
(5Z,7E)-(1S,3R)-20,21-méthylène-23-oxa-9,10-sécocholesta-5,7,10(19)-triène-1,3,25-triol,
(5Z,7E)-(1S,3R)-24-(3-hydroxy-3-méthylbutyl)-20,21-méthylène-23-oxa-9,10-sécochola-5,7,10(19)-triène-1,3,25-diol,
(5Z,7E)-(1S,3R)-24-(3-éthyl-3-hydroxypentyl)-20,21-méthylène-23-oxa-9,10-sécochola-5,7,10(19)-triène-1,3-diol,

(7E)-(1R,3R)-20-méthyl-19-nor-23-oxa-9,10-sécocholesta-5,7-diène-1,3,25-triol,
(7E)-(1R,3R)-26,27-diéthyl-20-méthyl-19-nor-23-oxa-9,10-sécocholesta-5,7-diène-1,3,25-triol,
(7E)-(1R,3R)-24-(3-hydroxy-3-méthylbutyl)-20-méthyl-19-nor-23-oxa-9,10-sécochola-5,7-diène-1,3-diol,
(7E)-(1R,3R)-24-(3-éthyl-3-hydroxypentyl)-20-méthyl-19-nor-23-oxa-9,10-sécochola-5,7-diène-1,3-diol,
(7E)-(1R,3R)-26,27-diméthyl-19-nor-23-oxa-9,10-sécocholesta-5,7,20-triène-1,3,25-triol,
(7E)-(1R,3R)-26,27-diéthyl-19-nor-23-oxa-9,10-sécocholesta-5,7,20-triène-1,3,25-triol,
(7E)-(1R,3R)-24-(3-hydroxy-3-méthylbutyl)-19-nor-23-oxa-9,10-sécochola-5,7,20-triène-1,3-diol,
(7E)-(1R,3R)-24-(3-éthyl-3-hydroxypentyl)-19-nor-23-oxa-9,10-sécochola-5,7,20-triène-1,3-diol,
(5Z,7E,22E)-(1S,3R)-24-(2-hydroxy-2-méthylpropoxy)-9,10-sécochola-5,7,10(19),20,22-pentaène-1,3-diol,
(5Z,7E,22E)-(1S,3R)-24-(2-éthyl-2-hydroxybutoxy)-9,10-sécochola-5,7,10(19),20,22-pentaène-1,3-diol,
(7E,22E)-(1R,3R)-24-(2-hydroxy-2-méthylpropoxy)-19-nor-9,10-sécochola-5,7,20,22-tétraène-1,3-diol,
(7E,22E)-(1R,3R)-24-(2-éthyl-2-hydroxybutoxy)-19-nor-9,10-sécochola-5,7,20,22-tétraène-1,3-diol.

**12.** Procédé pour la préparation de dérivés de vitamine D de formule générale I selon la revendication 1, caractérisé en ce que l'on transforme un composé de formules générales XIV, XVIII, XXVIII, XXXV, XLI ou IL

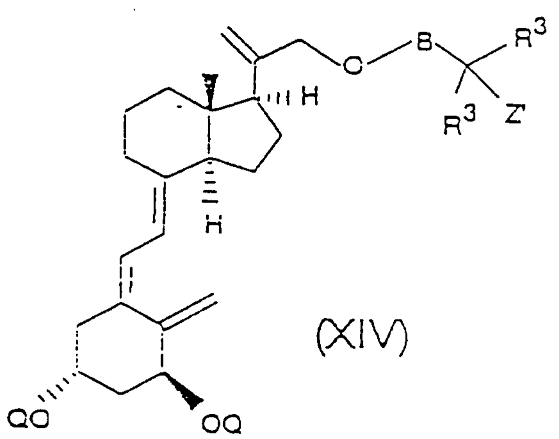

(XIV)

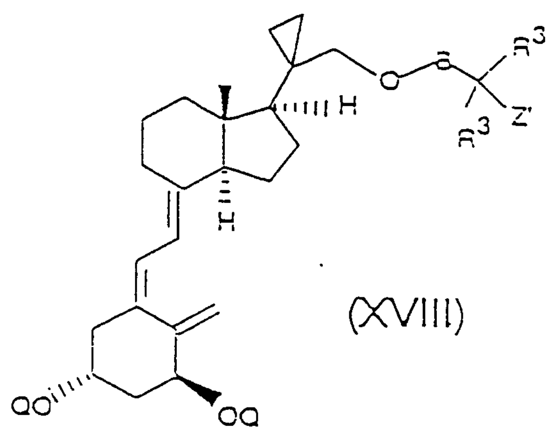

(XVIII)

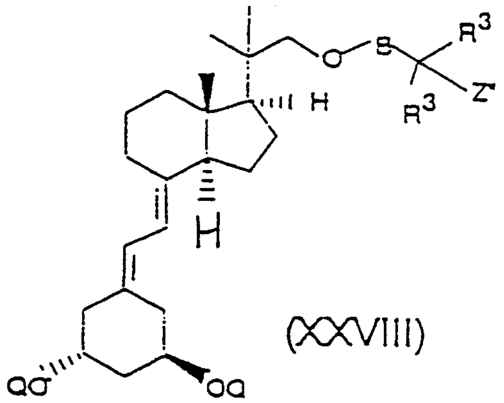

(XXVIII)

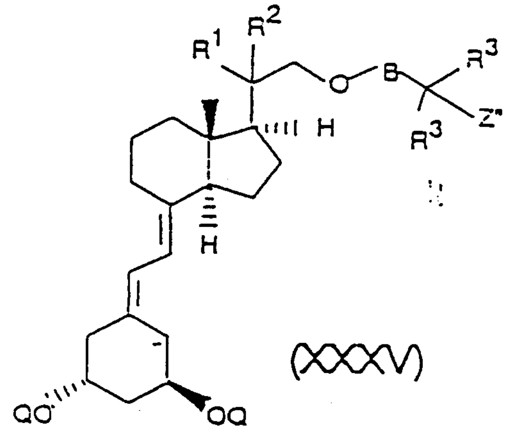

(XXXV)

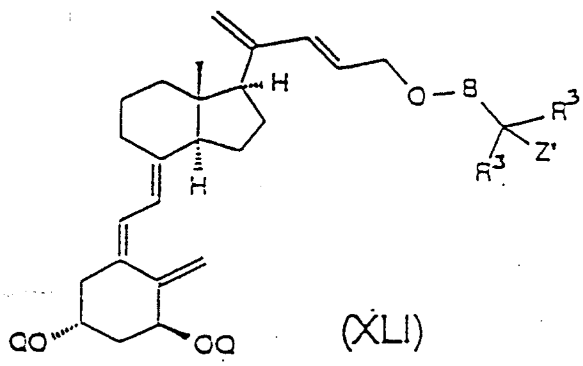

(XLI)

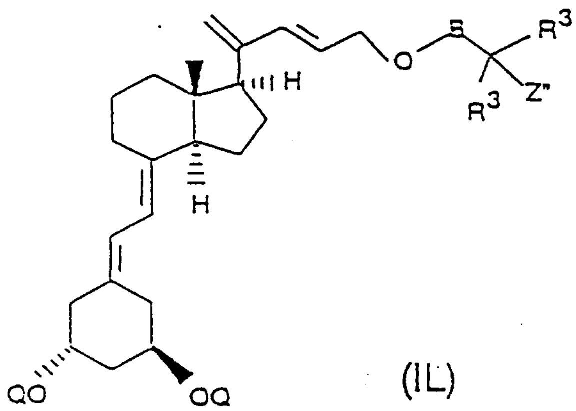

(IL)

où B, $R^1$, $R^2$ et $R^3$ ont les significations données dans la revendication 1 (Q = groupe silyle substitué par alkyle ou aryle ou par alkyle/aryle, Z = groupe hydroxyle ; Z" = groupe silyle) par séparation des groupes protecteurs Q et on transforme Z", si présent, en la liaison hydroxyle libre correspondante ainsi que on les estérifie éventuellement partiellement ou complètement avec un anhydride, bromure ou chlorure d'acide alcanecarboxylique, lequel comporte dans son radical alcanoyle de 1 à 9 atomes de carbone ou on estérifie la position 1 et 3 avec le chlorure de benzoyle.

13. Procédé selon la revendication 12, caractérisé en ce que l'on sépare les groupes protecteurs avec le fluorure de tétra-n-butylammonium.

**14.** Produit intermédiaire de formules générales IX et XV

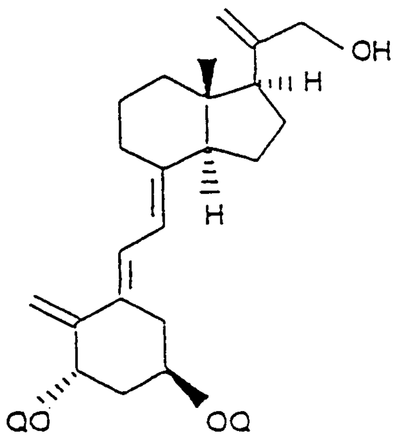

(IX)

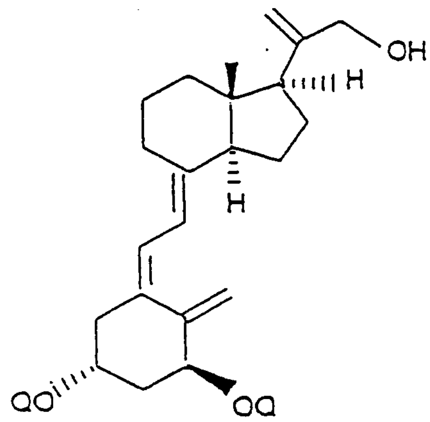

(XV)

dans lesquelles Q représente un groupe silyle substitué par alkyle ou aryle ou par alkyle/aryle.

**15.** Composés selon la revendication 14
(5E,7E)-(1S,3R)-1,3-bis-[[(1,1-diméthyléthyl)-diphénylsilyl]-oxy]-20-méthylène-9,10-sécopregna-5,7,10(19)-triène-21-ol,
(5Z,7E)-(1S,3R)-1,3-bis-[[(1,1-diméthyléthyl)-diphénylsilyl]-oxy]-20-méthylène-9,10-sécopregna-5,7,10(19)-triène-21-ol.

**16.** Préparation pharmaceutique contenant au moins un composé de formule générale I ainsi qu'un véhicule pharmaceutiquement compatible.

**17.** Utilisation des composés de formule générale I pour la préparation de médicaments.